(19) Europäisches Patentamt
European Patent Office
Office européen des brevets

(11) EP 4 015 043 A1

(12) EUROPEAN PATENT APPLICATION

(43) Date of publication:
**22.06.2022 Bulletin 2022/25**

(21) Application number: **22156380.2**

(22) Date of filing: **11.12.2017**

(51) International Patent Classification (IPC):
***A61P 35/00*** (2006.01) ***C07K 16/24*** (2006.01)
***A61K 31/00*** (2006.01) ***A61K 35/17*** (2015.01)
***C07K 14/00*** (2006.01) ***C12N 15/113*** (2010.01)
***A61K 39/395*** (2006.01)

(52) Cooperative Patent Classification (CPC):
(C-Sets available)
**C07K 16/248; A61K 35/17; A61K 39/3955; A61K 45/06; A61P 35/00; C12N 15/1138;**
C07K 2317/70; C07K 2317/76; C12N 2310/14; C12N 2310/20 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2016 US 201662432334 P**
**03.07.2017 EP 17179414**

(62) Document number(s) of the earlier application(s) in accordance with Art. 76 EPC:
**17825416.5 / 3 551 294**

(71) Applicant: **ONK Therapeutics Limited**
**Co. Galway H91 V6KV (IE)**

(72) Inventors:
- **O'DWYER, Michael Eamon Peter**
  **Galway (IE)**
- **HU, Jinsong**
  **Xi'an Shaanxi (CN)**

(74) Representative: **Hughes, Sean David et al**
**Schlich**
**9 St. Catherine's Road**
**Littlehampton, West Sussex BN17 5HS (GB)**

Remarks:
This application was filed on 11.02.2022 as a divisional application to the application mentioned under INID code 62.

# (54) IMPROVED NK-BASED CELL THERAPY

(57) Methods of cancer treatment comprise administration of a natural killer (NK) cell or cell line in combination with an IL-6 antagonist, such as an antibody to IL-6 or its receptor, especially for treatment of cancer expressing IL-6 receptors and in which checkpoint inhibitory receptors, such as PDL-1 and/or PDL-2 are expressed / upregulated during disease.

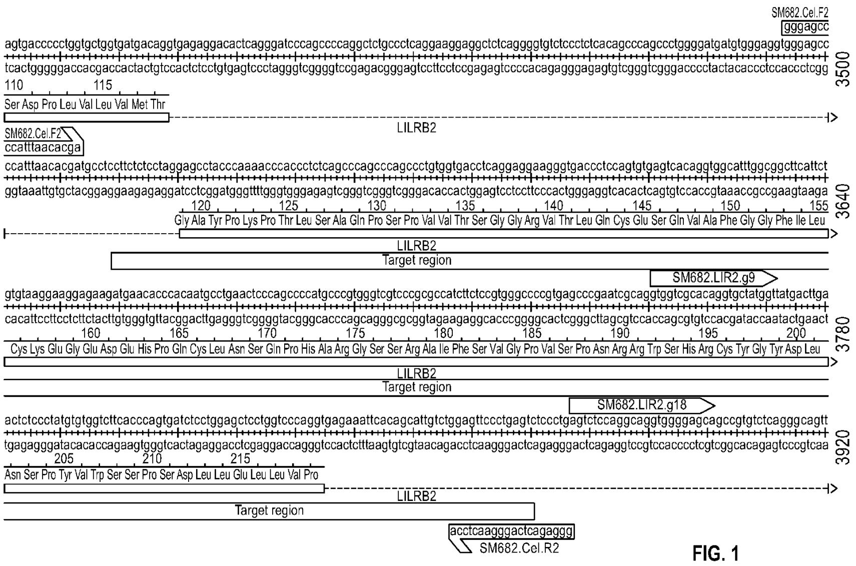


FIG. 1

EP 4 015 043 A1

(52) Cooperative Patent Classification (CPC): (Cont.)

C-Sets
**A61K 35/17, A61K 2300/00;**
**A61K 39/3955, A61K 2300/00**

SEQUENCE LISTING

<110> ONK Therapeutics Limited

<120> IMPROVED NK-BASED CELL THERAPY

<130> P40309EPD1

<160> 12

<170> PatentIn version 3.5

<210> 1
<211> 9540
<212> DNA
<213> Homo sapiens

<400> 1
ctctggcctc tgttctttct tgtgagtccg tctacacttg gggtttccac atgtcttttt 60

ctgctcatga ccttgatact ctgggtattt cagaaatgct acacatacgt ttctccatta 120

cggtcagatg tgacatcttg agtggactca tcaatcacct acagaatgtg gagtccaaca 180

gcaagatcct ctcacgtccc aaagcctcag gtcttaccct ggtctggaaa tcaagcacaa 240

atgagcccct cccaatgtcc caggcaccac tgaccccaca accactgtga cgagtgggat 300

tcatgacaac aatctgcaaa ggaagaaact gaggctcagt gatgggacat tacaaaccaa 360

ggtcacgtag gcagcggatg ataaccagtc atcaaataaa tatcaactcc ctcccccact 420

ccccaaatca aagctcaaac ataagtcatt gttcccaaaa tgttgaccag gaattgaggt 480

gcagagggac ggctaaggac gcaatgggca ccgaggaggc aggaaagact cagaggtttc 540

ttcccggggg ggagggagtg gacgctggag caaaaacatt taaaaagggg aagttaagag 600

gggactattt ggttgaaaga aaacccacaa tccagtgtca agaaagaagt caacttttct 660

tcccctactt ccctgcattt ctcctctgtg ctcactgcca cacacagctc aacctggaca 720

gcacagccag aggcgagatg cttctctgct gatctgagtc tgcctgcagc atggacctgg 780

gtcttccctg aagcatctcc agggctggag ggacgactgc catggtaagg accccacaac 840

gctgtgctga tggatgggct gaaggaggga gggtgaccat gtgggaagct gtgagaagga 900

aggggaagcc actgctaccc tcatcaggaa gggcagacac aagaagcacc agttctattt 960

gctgctacat cccggctctc ggtgagacga ggagaaacca gacagacagt ggctgggggt 1020

caggaaagac cccattacag tctgaaatgt ctgcagaggg cccagtgcct gcccccacct 1080

cagctctaaa agaatgagag tcaggctcct gggagggcag ttccgcttct tgtgtggctg 1140

cagatgacaa caccccatga gaaggaccca gcctctgagt gtccacacag ggtgggaagg 1200

aggggaggct atttctctct gtgtgtctct gtcccgccag caccgagggc tcatccatcc 1260

gcagagcagg gcagtgggag gagacgccat gacccccatc gtcacagtcc tgatctgtct 1320

cggtgagatt tgaagagaga ggggagcttc taacctagga gggacctcac cccacagcca 1380

aactctggtc cctaaggaga ccccaggggc tcacaaagat cccagggagg ggaggacctg 1440

ctcaggcttc aggggggcaaa tccctcacag ggaactctct tccagggctg agtctgggcc 1500

ccaggacccg cgtgcagaca ggtgagtctg tccccagctc tcccaggtcc ctcctcctca 1560

ctggggacaa ggggccacct ccgtgcagct ggggatgggg attagaagtt ctggactgac 1620

tgatgggggc atctggaggg tcctgggctg agagctgaga tctgttgggt gggaaatgac 1680

ttcgaatctg acctttgatt tccttccagg gaccatcccc aagcccaccc tgtgggctga 1740

gccagactct gtgatcaccc aggggagtcc cgtcaccctc agttgtcagg ggagccttga 1800

agcccaggag taccgtctat atagggagaa aaaatcagca tcttggatta cacggatacg 1860

accagagctt gtgaagaacg gccagttcca catcccatcc atcacctggg aacacacagg 1920

gcgatatggc tgtcagtatt acagccgcgc tcggtggtct gagctcagtg acccccctggt 1980

gctggtgatg acaggtgaga ggacactcag ggatcccagc cccaggctct gccctcagga 2040

aggaggctct caggggtgtc tccctctcac agcccagccc tggggatgat gtgggaggtg 2100

ggagccccat ttaacacgat gcctccttct ctcctaggag cctacccaaa acccaccctc 2160

tcagcccagc ccagccctgt ggtgacctca ggaggaaggg tgaccctcca gtgtgagtca 2220

caggtggcat ttggcggctt cattctgtgt aaggaaggag aagatgaaca cccacaatgc 2280

ctgaactccc agccccatgc ccgtgggtcg tcccgcgcca tcttctccgt gggccccgtg 2340

agcccgaatc gcaggtggtc gcacaggtgc tatggttatg acttgaactc tccctatgtg 2400

tggtcttcac ccagtgatct cctggagctc ctggtcccag gtgagaaatt cacagcattg 2460

tctggagttc cctgagtctc cctgagtctc caggcaggtg gggagcagcc gtgtctcagg 2520

gcagttccag gtgggatgat gttggggcga gagggctcag gggtcctggg gccagagaca 2580

caggaagatc agcagtggtg aggcaccggg ggagagggag ggtttgtggg gaagcctgag 2640

ggtcggctcc tggaaaccat gagcaccttt tcccaggtgt ttctaagaag ccatcactct 2700

cagtgcagcc gggtcctgtc atggcccctg gggaaagcct gaccctccag tgtgtctctg 2760

atgtcggcta tgacagattt gttctgtaca aggaggggga acgtgacctt cgccagctcc 2820

ctggccggca gcccccaggct gggctctccc aggccaactt caccctgggc cctgtgagcc 2880

gctcctacgg gggccagtac agatgctacg gtgcacacaa cctctcctct gagtgctcgg 2940

cccccagcga ccccctggac atcctgatca caggtgagga gcccagcggg ttcagtcagg 3000

gacccagact ctgcacaggc cctgccgggg gaatccaatt agtgatggcc aggatgaggc 3060

gggggtggt cccaagggag ggagagacag agagagagac aggggatggg tggggagggg 3120

aagactcaga gaaaacagag acagaggctc ctagagaggc ctggggaggt ctcagctcag 3180

agcaaggtgg ggcagcccct cacccatcct tcttctctcc aggacagatc cgtggcacac 3240

ccttcatctc agtgcagcca ggccccacag tggcctcagg agagaacgtg accctgctgt 3300

gtcagtcatg gcggcagttc cacactttcc ttctgaccaa ggcgggagca gctgatgccc 3360

cactccgtct aagatcaata cacgaatatc ctaagtacca ggctgaattc cccatgagtc 3420

ctgtgacctc agcccacgcg gggacctaca ggtgctacgg ctcactcaac tccgacccct 3480

acctgctgtc tcaccccagt gagcccctgg agctcgtggt ctcaggtggg ggccttgacc 3540

ctgtcctctc tgagctcaaa ggctcagctc aggccctgcc ccccaggaga gctctgggct 3600

gggatggagt gagcgggggt ctgagcgggg ctcagccagt gggagactca ccctcagagg 3660

gaaggaggac aacaggccct cccaggcctg cgcacactca gcggcatcgc cagcatcatg 3720

gacaggagag gcgggtggag ggagggcct ggggaggcca cagggcccat gtagagaaat 3780

ttggtttgag gtggagactt caggaaagcc ccagctcctc accctcctct cattctttca 3840

cccaggaccc tccatgggtt ccagcccccc acccaccggt cccatctcca cacctggtga 3900

gtccctgagg cctctggctc gaagggagcg cagcgacccc cagggcagct ttgagtgtcc 3960

aggaggatcc cattcccttc agggactcaa tcaagggctt ctgtccaggg agctgggcag 4020

agccagagga ggggccacag ggtccccagg gctctgaggc tgggctggtg aggggtgggg 4080

ggtcaaggca gagagaaatg ttggggccca gcctggggga ggagcagccg ggctgatgtg 4140

gggagcaggg cagccccagc cctcacctcc ccgtcctgac ccagcaggcc ctgaggacca 4200

gcccctcacc cccactgggt cggatcccca aagtggtgag tgaggggctc tgagtgggag 4260

gtgggcgggg tcccggggag gcaggggtgg gttctgtcct aggttcaggc tcctctggag 4320

gtggtgatgt agacaggctc ctcccctgcc tgggcctcag tttctccaag tgtaaaggag 4380

agaggcctgc aggtgggaaa gttcctttca gctctcactc ccagctgtga cctcctggga 4440

gaggaggccc ctcagggaag actccaagac tcgattccgc gggggcctgt cccgtcccac 4500

ctgcagcaga gacggtgacc tggggcaggg gaggggagca gagtcgtggt tcaggacggt 4560

aaggctcttt ccctgcagct ccggggctcg gctctggtgc aggaacaagg gctgcaggtc 4620

agactcccag gctcccttcc cagctctgcc gcttcctggc tgggggcccg gggcaggcga 4680

ttcccctctc tgagcgtcag tttttcatct gtagagtggg tggggtggat gtttgtgtgc 4740

tgcacgactg ttgtgggggt tggaggtggt gaacagaagg tccagcagtc acctgcacac 4800

agtaggcgct catttcaatg acatcacccc catccctgac atcatcgtgc tcaaggtctg 4860

ggaaggcacc tgggggttgt gatcggcatc ttggtggccg tcgtcctact gctcctcctc 4920

ctcctcctcc tcttcctcat cctccgacat cgacgtcagg gcaaacactg gacatcgagt 4980

gagtagggaa ggggaaaccc tgtgggccga ccgagggtgg gctcagggca cagccaaaga 5040

gaatccaaac cactgggcaa atgcagcttt gagaaactgt tccagcattt ctcaccaggt 5100

gaatggagaa agcacttaac gtcagtccca tctacaaata taaagtgtcc tccgggctca 5160

gtcccatcta caaatgtaaa gtgtccttcg gactctgtcc atctcatgag gcatttggaa 5220

catggaggca ggagtgtttt taggtttcct tccttacctt cgagctgtgt gtgcagggca 5280

gggggctcca atgttcccag ggctgaggct ctgtccttct tcccccagcc cagagaaagg 5340

ctgatttcca acatcctgca ggggctgtgg ggccagagcc cacagacaga ggcctgcagt 5400

ggaggtaatt ctgcccgaag accccagact cccacctgct cgtggcccat acactgcccc 5460

taaagctccc attcctcccc caggtccagc ccagctgccg acgcccagga agaaaacctc 5520

tgtgagtgag aggaagaggt gaccagccag gagggagata ggggccccga agtttccgta 5580

gcaatgggga aaggggcacc ggctggaaag ggtctggggc tcagggtgag atcatctcac 5640

cccacactgt gggacctcag ggacattgca gcccctccct gcatctcagt agccccatct 5700

gggagcaggg caggggctgg caggactcag aggtcccagg gaaccttccc aagagacgaa 5760

ccccttgctc tgccccagca gatgctgccg tgaaggacac acagcctgaa gatggggtgg 5820

agatggacac tcgggtgaga ccccgcccct gtcccaggca ccaaaggcct cctggtgcca 5880

gatctaatcc agcaggactt ctctgtcctc cttcccccgg ctctcagcat cgtcacggtg 5940

gacccctcct tgtccagcac gctgcctccc gcctgctgtg acctcactct ctcctgctgt 6000

cctgggacct cgtgggcctc ctcccgggtc cccttcctgc tcctcatcct ctgtttggcc 6060

gtctggttgt tagagcgctc cccaggcctc tggaggatga ggaataaatg aaccaccccg 6120

gtcccctggg ctccccttca ttcattcaac cagtgagtgt tcccagggag ctcactgtgg 6180

atcaggctcc ccatgggagc tgcagacaca gcagggagca aagccgcccc cgcctcctga 6240

gctcacctca tggtgggaga caaaatgcaa ataaatgcgc catgtccagg agtgcaacgt 6300

gcttaaagga acatacacca gggaaagggc agagagtgtg gggcagtggg gccagtctga 6360

atggaagggg agggctgtct gctcagctgt catctgagaa gcctggacag agtggggcac 6420

acgatcctct aatggacgag cccctgcagg cagaggaaac agccgtgcaa aggccccgag 6480

gcagcagcga gctcttgcgg gaaggcccat gaggctgcag ccaaatgggc aaggtcaaag 6540

tgaggagcag aggccagaac cacaggaagg gagcggccag accctccacg gccttagggc 6600

gtccctgaga ttccatcggg aaagggatgt aatcggatca ccccgggaac agtgaggaaa 6660

attgactcca ggaggtcagg gggactcaag gacacccccc accactgtct ctctccagca 6720

gagcccacat gatgaagacc cccaggcagt gacatatgcc ccggtgaaac actccagacc 6780

taggagagaa atggcctctc ctccctcccc actgtccggg gaattcctgg acacaaagga 6840

cagacaggca gaagaggaca gacagatgga cactgagaga gtcctttcct ctccaggccc 6900

ccaggcctcc cccaccccca ccacgttcct tacctctcac tctcccccgc tgcaggctgc 6960

tgcatctgaa gccccccagg atgtgaccta cgcccagctg cacagcttga ccctcagacg 7020

gaaggcaact gagcctcctc catcccagga aagggaacct ccagctgagc ccagcatcta 7080
cgccaccctg gccatccact agcccggagg gtacgcagac tccacactca gtagaaggag 7140
actcaggact gctgaaggca cgggagctgc ccccagtgga caccaatgaa ccccagtcag 7200
cctggacccc taacaaagac catgaggaga tgctgggaac tttgggactc acttgattct 7260
gcagtcgaaa taactaatat ccctacattt tttaattaaa gcaacagact tctcaataat 7320
caatgagtta accgagaaaa ctaaaatcag aagtaagaat gtgctttaaa ctgaatcaca 7380
atataaatat tacacatcac acaatgaaat tgaaaaagta caaaccacaa atgaaaaaag 7440
tagaaacgaa aaaaaaaaac taggaaatga atgacgttgg ctttcgtata aggaatttag 7500
aaaaagaata accaattatt ccaaatgaag gtgtaagaaa gggaataaga agaagaagag 7560
ttgctcatga ggaaaaacca aaacttgaaa attcaacaaa gccaatgaag ctcattcttg 7620
aaaatattaa ttacagtcat aaatcctaac tacattgagc aagagaaaga aagagcaggc 7680
acgcatttcc atatgggagt gagccagcag acagcccagc agatcctaca cacattttca 7740
caaactaacc ccagaacagg ctgcaaacct ataccaatat actagaaaat gcagattaaa 7800
tggatgaaat attcaaaact ggagtttaca taatgaacgt aagagtaatc agagaatctg 7860
actcatttta aatgtgtgtg tatgtgtgtg tatatatatg tgtgtgtgtg tgtgtgtgtg 7920
tgtgtgtgaa aaacattgac tgtaataaaa atgttcccat cgtatcaact ccagttcagg 7980
aagtttcact ggtgatttct tacaaatatt gacgcactaa tgaaacacac aaacacaccc 8040
agagcatcac aaatgtttct tgagaataga aaaagaggca atgtgcccgg gtgcggtggc 8100
tcacgcctgt aatctcaaca cctagggagg cagaggccac agattacttg aggccgggag 8160
ttcaagacca gcatggccaa caaggcaaaa ccccatctct actaaaaata caaaaattag 8220
ctggacatgg tggcgcacgc tgcaatccca gctacttggg aggcagaggc aggaggatca 8280
cttgaatgaa cccgggaggt ggaggttgaa gtgagcaaaa acaaaccccc tacaattcag 8340
cctaggatat gtttattaaa tttacatttg tctttttgct taagattgct ttggtattca 8400
tcctcttttt ggttccatat gaattttagg atttttttct aattctgtga aaaaaatgat 8460
gttgatattt tgatgggaat tgcattgaac ctaaatattg ctttgggaag tgtgatcatt 8520
ttcacaatat tgattctgcc aatccatgag catgggatat atttctattt tgctgtgtca 8580
tctacgattt ctttctgcag cattttgttg ttcttcttgt agagatcttt cacctcctca 8640
gttaggtata ttcttagata tttttaattt tttgcaactg atgtacaagg gattgagttt 8700
tgcagcaacc tggatgagct ggaggccatt attcatgaca ccacatccag ctaatttttg 8760
tatttcttgt agagatgagg ttttgccatg ttgcccaggc tggtcttgaa ctcctgggcc 8820
caagtgaccc gcccgccttg acctcccaaa gtgctgggac tgcaggcatg agccacggtg 8880
cctggcccat catagcactt ttgatcatta ggataattcc ttctccttgt catttttgga 8940

```
cacatgcttc ccacatgcct catcttccag agagggtttc caccagggct gtgctgggag      9000

ttaaggctgg aaaaggggag atggttccac ctgccagtgc cacatgagtc tactcagggc      9060

tgtaaccagc agggagggtc cagtgtgagc ctcagactcg catgtgggac agacgcccat      9120

gtgtgacaac gctgcagtga atctgtttca cacacatgga ggaggcggct cagggctgac      9180

catggacctg agtcaatgag cagagatatc ccagtgccat ccacaaacac aggggagaag      9240

gagccacaac ttcccacttt catccaaaac cccgacccct ccctgtctgt gagggccctg      9300

gggttctcct ctgtctcata cagaggcaga aacctccccc ttagtgaccc ccagctttgc      9360

aagtcaccag cagcccctcg gcgctggcat cttctgcttc ttaaggtttc ctgcctatga      9420

caggaagtct catttctcat tttcttcatt ggaccatggc tacatatttc agacacatta      9480

taagtaggtt ttcccagtgt taggagcaga tgtgggctgt tgagcacata agtcactcac      9540


<210>  2
<211>  101
<212>  PRT
<213>  Homo sapiens

<400>  2

Gly Ala Tyr Pro Lys Pro Thr Leu Ser Ala Gln Pro Ser Pro Val Val
1               5                   10                  15


Thr Ser Gly Gly Arg Val Thr Leu Gln Cys Glu Ser Gln Val Ala Phe
            20                  25                  30


Gly Gly Phe Ile Leu Cys Lys Glu Gly Glu Asp Glu His Pro Gln Cys
        35                  40                  45


Leu Asn Ser Gln Pro His Ala Arg Gly Ser Ser Arg Ala Ile Phe Ser
    50                  55                  60


Val Gly Pro Val Ser Pro Asn Arg Arg Trp Ser His Arg Cys Tyr Gly
65                  70                  75                  80


Tyr Asp Leu Asn Ser Pro Tyr Val Trp Ser Ser Pro Ser Asp Leu Leu
                85                  90                  95


Glu Leu Leu Val Pro
            100


<210>  3
<211>  23
<212>  DNA
<213>  Homo sapiens

<400>  3
```

gagtcacagg tggcatttgg cgg 23

<210> 4
<211> 23
<212> DNA
<213> Homo sapiens

<400> 4
cgaatcgcag gtggtcgcac agg 23

<210> 5
<211> 20
<212> DNA
<213> Homo sapiens

<400> 5
gggagcccca tttaacacga 20

<210> 6
<211> 20
<212> DNA
<213> Homo sapiens

<400> 6
gggagactca gggaactcca 20

<210> 7
<211> 7375
<212> DNA
<213> Homo sapiens

<400> 7
ctttggacct tcttcaactc tgttttgtct ctgttgagtt aaggctttta agaacacctg 60

aattctttcc ttctgcaaaa ccagaggcag cttcttttcc gcctattttc agtttatttc 120

ttgtgatttt agttttttttc tcttaaccaa atgctaaatg gatttaggag aaataaactt 180

atttgtaaag ctgtcaaggg accattagaa ggatggtgct tcacagatag aatacagttt 240

ttattaatga tgcctagaca aatcctgcca ttagcccaag ggctcagaaa gttagcagcc 300

tagtagtttt ggagttgtca atgaaatgaa ttggactgga tggttaagga tgcccagaag 360

attgaataaa attgggattt aggaggaccc ttgtactcca ggaaattctc caagtctcca 420

cttagttatc cagatcctca aagtgaacat gaagcttcag tttcaaattg aatacatttt 480

ccatccatgg attggcttgt tttgttcagt tgagtgcttg aggttgtctt ttcgacgtaa 540

cagctaaacc cacggcttcc tttctcgtaa aaccaaaaca aaaaggcttt ctattcaagt 600

gccttctgtg tgtgcacatg tgtaatacat atctgggatc aaagctatct atataaagtc 660

cttgattctg tgtgggttca aacacatttc aaagcttcag gatcctgaaa ggttttgctc 720

tacttcctga agacctgaac accgctccca taaagccatg gcttgccttg gatttcagcg 780

gcacaaggct cagctgaacc tggctaccag gacctggccc tgcactctcc tgtttttttct 840

tctcttcatc cctgtcttct gcaaaggtga gtgagacttt tggagcatga agatggagga 900
ggtgtttctc ctacctgggt ttcatttgtt tcagcagtca aaggcagtga tttatagcaa 960
agccagaagt taaaggtaaa actccaatct ggcttggctg gctctgtatt ccagggccag 1020
cagggagcag ttgggcggca gcaaataagg caaagagata gctcagaaca gagcgccagg 1080
tatttagtag gggcttcatg aatgcatgtg agttggttta gtagagagac acaggcaatt 1140
tcagacccttt ctatgagact ggaagtgatt taagagggaa aggatagcca tagtcctgaa 1200
tacatttgag ctgggtttca ggatgagctc acaagttcct ttaaaaaaaa ttgacttaag 1260
caaatcctgg gaagagtttt tttgctatac aattcaaggt tttaaggtcc tcggattcat 1320
atactttata aatgaattag ccagcttgtt taaaatgtag ggaaattgtg ggaagaatgc 1380
cttctttact taattcaagg ttttaaggtt ctcttaatca attctactag ctaattagcc 1440
aattatttaa aaataaaagt ttgaaattgc caaaaaaaaa agacaaggaa aaggaaagaa 1500
agaaagccac cagtctgttt ggcatacaat acttaattgt tgcctgacct acgtgtgggt 1560
ttcagatgca gatcctcagt tttcagctct tcagagactg acaccaggtt tgttacacgg 1620
cttaaaatga tgagtatatc cattgaatct caaccttatc tctctctaga ccttcttggt 1680
taagaaacca tgtagtttgt atgaagtagg tactcaaaag atatttgatg atttaatttt 1740
tactggagaa gaaatattca tatatgtttt cttattttta catgttttaa atatgtaaag 1800
attaaataaa cactcttaga agtatttaaa tttcctaaag taaatttatc tcaaccagta 1860
acaggaccct cccaatactg gaaagttgag tgtgaccgca tttagtggtg atgagtgtga 1920
gcttgcttgg ggagagggca ggacatttag gatttcttaa gcttagagtc aatacaataa 1980
agattattga gtgctcactt gggtgggcta taatcactgc tcacaggagt tcatgaacca 2040
caagtaaaag agtgaggaga tatgattagc tcacaaataa ctttaataca gagcagaaag 2100
taatgaacta ctgcaatgga gttatcacag tgctaaggat gctcagaggg catctctgat 2160
aggcagaggt gagggttagg gaaggaagct gtagtctagc tagctagagc tgctggaata 2220
gacatgacaa tggctgctgc caaactgttt tctcttctga ggacagatgt cccgtgcaag 2280
tggcttggtg gaagggacta gtgtctctaa tatagggtga tttataagca ggaaagtgtg 2340
tcctagaaat tcagaccaga gtgatagatt ggaattggat catgggggac tcattgaatg 2400
ttatttattg tatttgtttt tgcgatcagt gttagtaaag tgtcaaaggg attgagcaga 2460
tgagtgacat catgcaacac aagttttgag tttcacttgt cagactgact ggagaggggc 2520
ctggttagtt acaggaaggt aatttggcat gcagccacta tttttgagtt gatgcaagcc 2580
tctctgtatg gagagctggt ctcctttatc ctgtgggaaa agagaacaaa ggagcatggg 2640
agtgttcaag ggaaggagaa ataaagggca gagaggcagc ggtggtgtca ggggaagccc 2700

acaggagtta acagcagggt tgcctcaacc tagagaggaa gcgacctggt gccctcggct 2760

ctgtggcttc cttcatctaa caacatcttc cactctacaa caatgccagg gaaggcggag 2820

gctggtacag tgcatcaaga cacagctact cctgggtgac agaggttcag ggccagctca 2880

ctaagtaggc agaagttttt gacatatact ttgagagata aagcaagatt ctgtacctca 2940

accttcagaa tttcccctac cactcattat agttccggag ctatatagct cctatcattc 3000

tatcataacc ttagaatacc agagaacata tcatctcatc taattatctc ttactatatg 3060

tgaaaaaaat gaaggacatg gggaagtgt gacttgcccc aaatcacata tttcatggta 3120

gagccaggtc ttctgtttgt catatcagtg ttcttcctgc cacaaccatc ttgaagaatc 3180

tatttctcag taagaaaata tctttatgga gagtagctgg aaaacagttg agagatggag 3240

gggaggctgg gggtgtggag aggggaaggg gtaagtgata gattcgttga agggggggaga 3300

aaaggccgtg gggatgaagc tagaaggcag aagggcttgc ctgggcttgg ccatgaagga 3360

gcatgagttc actgagttcc ctttggcttt tccatgctag caatgcacgt ggcccagcct 3420

gctgtggtac tggccagcag ccgaggcatc gccagctttg tgtgtgagta tgcatctcca 3480

ggcaaagcca ctgaggtccg ggtgacagtg cttcggcagg ctgacagcca ggtgactgaa 3540

gtctgtgcgg caacctacat gatggggaat gagttgacct tcctagatga ttccatctgc 3600

acgggcacct ccagtggaaa tcaagtgaac ctcactatcc aaggactgag ggccatggac 3660

acgggactct acatctgcaa ggtggagctc atgtacccac cgccatacta cctgggcata 3720

ggcaacggaa cccagattta tgtaattggt gagcaaagcc atttcactga gttgacacct 3780

gttgcattgc agtcttctat gcacaaaaac agttttgttc cttaatttca ggaggtttac 3840

ttttaggact gtggacattc tctttaagag ttctgtacca catggtagcc ttgcttattg 3900

tgggtggcaa ccttaatagc attctgactg taaaataaaa tgatttgggg aagttggggc 3960

tctcgctctg gagtgctaac catcatgacg tttgatctgt acttttgata tgatatgatg 4020

ctcctgggga agtagtccca aatagccaaa cctattggtg ggctacccat gcaatttagg 4080

ggtggacctc aaggcctgga agctctaatg tcctttttttc accaatgttg gggagtagag 4140

ccctagagtt taaaactgtc tcagggaggc tctgctttgt tttctgttgc agatccagaa 4200

ccgtgcccag attctgactt cctcctctgg atccttgcag cagttagttc ggggttgttt 4260

ttttatagct ttctcctcac agctgtttct ttgagcaaaa tggtgagtgt ggtgctgatg 4320

gtgcaccatg tctgatgggg ataccctttag tggtatcaac tggccaaaag atgatgttga 4380

gtttagtgtt cttgagatga gatgaggcaa taaatgaaga ggaaggacag tggtaaagaa 4440

cgcactagaa ccgtaggcat tggcatttga ggtttcagaa tgactaatat tttagatgaa 4500

tttgtttgac attgaatgtt catgtgcttc tgagcagggt ttcaatttga gtaaccgttg 4560

caataacatg gggcagctgt tttgctcttt gtcttcatga caactgtact taagctaaca 4620

gccctgaaac atgagattag gctgggcaga atgctgctag agaggaccac ttggatggtc 4680
tttattctcc ttctccatgt ccctctccat cacctggaag tcacctctgg gtgccactct 4740
ggtgccttcc ttgtcgaagc tgtagctgct cacatgacac ctatccctgt tatccagttt 4800
gcttgactgg gacgttttgc cttccccttc agccaggaag tgaaagtccc agtttttatt 4860
tatcacaggt gttggtattg gtggtagaag aggtagaatt atggaatcag gcctcctgtc 4920
aggatttctt tttgacagtc cctctcagac acctctgcct aaggccagct ttgccattac 4980
aaactctccc ttctccctct ctcccttctt ctcttcctct tccttcttct cgctctttct 5040
ctctctctct ttctccctct ctgtctctta tacacataca caaagatata ctctattcca 5100
acatcctcta cccaacctga cagagatgtc ctttgctgta ggttcagcag tggggatgag 5160
aaatacagct ctcaaacagg ataactaaag cttattatct tatcaagctt gttcccttgc 5220
agacaagatt gatcaattat cataggcttt ctgggtgttc tttctgaagc tttctcaaag 5280
tctctttctc ctatcttcca ttcaaggcaa atgattgcca tttaacatca aaatcacagt 5340
tatttatcta aaataaattt taatagctga atcaagaaaa tctcctgagg tttataattc 5400
tgtatgctgt gaacattcat ttttaaccag ctagggaccc aatatgtgtt gagttctatt 5460
atggttagaa gtggcttccg tattcctcag tagtaattac tgtttctttt tgtgtttgac 5520
agctaaagaa aagaagccct cttacaacag gggtctatgt gaaaatgccc ccaacagagc 5580
cagaatgtga aaagcaattt cagccttatt ttattcccat caattgagaa accattatga 5640
agaagagagt ccatatttca atttccaaga gctgaggcaa ttctaacttt tttgctatcc 5700
agctattttt atttgtttgt gcatttgggg ggaattcatc tctctttaat ataaagttgg 5760
atgcggaacc caaattacgt gtactacaat ttaaagcaaa ggagtagaaa gacagagctg 5820
ggatgtttct gtcacatcag ctccactttc agtgaaagca tcacttggga ttaatatggg 5880
gatgcagcat tatgatgtgg gtcaaggaat taagttaggg aatggcacag cccaaagaag 5940
gaaaaggcag ggagcgaggg agaagactat attgtacaca ccttatattt acgtatgaga 6000
cgtttatagc cgaaatgatc ttttcaagtt aaattttatg ccttttattt cttaaacaaa 6060
tgtatgatta catcaaggct tcaaaaatac tcacatggct atgttttagc cagtgatgct 6120
aaaggttgta ttgcatatat acatatatat atatatatat atatatatat atatatatat 6180
atatatatat atatatattt taatttgata gtattgtgca tagagccacg tatgtttttg 6240
tgtatttgtt aatggtttga atataaacac tatatggcag tgtctttcca ccttgggtcc 6300
cagggaagtt ttgtggagga gctcaggaca ctaatacacc aggtagaaca caaggtcatt 6360
tgctaactag cttggaaact ggatgaggtc atagcagtgc ttgattgcgt ggaattgtgc 6420
tgagttggtg ttgacatgtg ctttggggct tttacaccag ttcctttcaa tggtttgcaa 6480

ggaagccaca gctggtggta tctgagttga cttgacagaa cactgtcttg aagacaatgg 6540

cttactccag gagacccaca ggtatgacct tctaggaagc tccagttcga tgggcccaat 6600

tcttacaaac atgtggttaa tgccatggac agaagaaggc agcaggtggc agaatggggt 6660

gcatgaaggt ttctgaaaat taacactgct tgtgttttta actcaatatt ttccatgaaa 6720

atgcaacaac atgtataata tttttaatta aataaaaatc tgtggtggtc gttttccgga 6780

gttgtcttta tcatccttgc atttgaatat tgtgttcaaa tttttgattg attcattcag 6840

tatctggtgg agtctccaat attagaaata ctggaaacaa actgaaaaac cacaaaagga 6900

caaataatgc ttcatgagtc agctttgcac cagccattac ctgcaagtca ttcttggaag 6960

gtatccatcc tctttccttt tgatttcttc accactattt gggatataac gtgggttaac 7020

acagacatag cagtccttta taaatcaatt ggcatgctgt ttaacacagg ttcttcacct 7080

ccccttctt accgcctgct ttctcagctc aactatcaca ggcattacag ttgtcatggc 7140

aaccccaatg ttggcaacca cgtcccttgc agccattttg atctgccttc ctgaaatata 7200

gagcttttcc ctgtggcttc caaatgaact attttgcaaa tgtggggaaa acacacacct 7260

gtggtcctat gttgctatca gctggcacac ctaggcctgg cacactaagc cctctgtgat 7320

tcttgcttaa ccaatgtata gtctcagcac atttggtttc cacttaaggt ttcct 7375

<210> 8
<211> 36
<212> PRT
<213> Homo sapiens

<400> 8

Met Ala Cys Leu Gly Phe Gln Arg His Lys Ala Gln Leu Asn Leu Ala
1 5 10 15

Thr Arg Thr Trp Pro Cys Thr Leu Leu Phe Phe Leu Leu Phe Ile Pro
20 25 30

Val Phe Cys Lys
35

<210> 9
<211> 23
<212> DNA
<213> Homo sapiens

<400> 9
cactcacctt tgcagaagac agg 23

<210> 10
<211> 23
<212> DNA
<213> Homo sapiens

<400> 10
cettgtgccg ctgaaatcca agg 23

<210> 11
<211> 29
<212> DNA
<213> Homo sapiens

<400> 11
aggacccttg tactccagga aattctcca 29

<210> 12
<211> 26
<212> DNA
<213> Homo sapiens

<400> 12
agcccctact aaatacctgg cgctct 26

**Description**

## Introduction

The present invention relates to therapies using natural killer (NK) cells or cell lines as effectors, for treatment of cancer. It relates to combination therapies, methods of treatment and compositions, especially for treatment of blood cancers.

## Background

Despite significant investment in a variety of physical, pharmaceutical and other therapies, human cancer remains a significant cause of mortality across all age groups.

As one example, acute myeloid leukemia (AML) is a hematopoietic malignancy involving precursor cells committed to myeloid development, and accounts for a significant proportion of acute leukemias in both adults (90%) and children (15-20%) (Hurwitz, Mounce et al. 1995; Lowenberg, Downing et al. 1999). Despite 80% of patients achieving remission with standard chemotherapy (Hurwitz, Mounce et al. 1995; Ribeiro, Razzouk et al. 2005), survival remains unsatisfactory because of high relapse rates from minimal residual disease (MRD). The five-year survival is age-dependent; 60% in children (Rubnitz 2012), 40% in adults under 65 (Lowenberg, Downing et al. 1999) and 10% in adults over 65 (Ferrara and Schiffer 2013). These outcomes can be improved if patients have a suitable hematopoietic cell donor, but many do not, highlighting the need for an alternative approach to treatment.

Natural killer (NK) cells are cytotoxic lymphocytes, with distinct phenotypes and effector functions that differ from natural killer T (NK-T) cells. For example, while NK-T cells express both CD3 and T cell antigen receptors (TCRs), NK cells do not. NK cells are generally found to express the markers CD16 and CD56, wherein CD16 functions as an Fc receptor and mediates antibody dependent cell-mediated cytotoxicity (ADCC) which is discussed below. KHYG-1 is a notable exception in this regard.

Despite NK cells being naturally cytotoxic, NK cell lines with increased cytotoxicity have been developed. NK-92 and KHYG-1 represent two NK cell lines that have been researched extensively and show promise in cancer therapeutics (Swift et al. 2011; Swift et al. 2012).

Adoptive cellular immunotherapy for use in cancer treatment commonly involves administration of natural and modified T cells to a patient. T cells can be modified in various ways, e.g. genetically, so as to express receptors and/or ligands that bind specifically to certain target cancer cells. Transfection of T cells with high-affinity T cell receptors (TCRs) and chimeric antigen receptors (CARs), specific for cancer cell antigens, can give rise to highly reactive cancer-specific T cell responses. A major limitation of this immunotherapeutic approach is that T cells must either be obtained from the patient for autologous *ex vivo* expansion or MHC-matched T cells must be used to avoid immunological eradication immediately following transfer of the cells to the patient or, in some cases, the onset of graft-vs-host disease (GVHD). Additionally, successfully transferred T cells often survive for prolonged periods of time in the circulation, making it difficult to control persistent side-effects resulting from treatment.

In haplotype transplantation, the graft-versus-leukemia effect is believed to be mediated by NK cells when there is a KIR inhibitory receptor-ligand mismatch, which can lead to improved survival in the treatment of AML (Ruggeri, Capanni et al. 2002; Ruggeri, Mancusi et al. 2005). Furthermore, rapid NK recovery is associated with better outcome and a stronger graft-vs-leukemia (GVL) effect in patients undergoing haplotype T-depleted hematopoietic cell transplantation (HCT) in AML (Savani, Mielke et al. 2007). Other trials have used haploidentical NK cells expanded *ex vivo* to treat AML in adults (Miller, Soignier et al. 2005) and children (Rubnitz, Inaba et al. 2010).

Several permanent NK cell lines have been established, and the most notable is NK-92 (mentioned above), derived from a patient with non-Hodgkin's lymphoma expressing typical NK cell markers, with the exception of CD16 (Fc gamma receptor III). NK-92 has undergone extensive preclinical testing and exhibits superior lysis against a broad range of tumours compared with activated NK cells and lymphokine-activated killer (LAK) cells (Gong, Maki et al. 1994). Cytotoxicity of NK-92 cells against primary AML has been established (Yan, Steinherz et al. 1998).

Another NK cell line, KHYG-1, has been identified as a potential contender for clinical use (Suck et al. 2005) but has reduced cytotoxicity so has received less attention than NK-92. KHYG-1 cells are known to be pre-activated. Unlike endogenous NK cells, KHYG-1 cells are polarized at all times, increasing their cytotoxicity and making them quicker to respond to external stimuli. NK-92 cells have a higher baseline cytotoxicity than KHYG-1 cells.

Cifaldi et al. (Arthritis Rheumatol. 2015 Nov;67(11):3037-46) reported that IL-6 decreased NK cell cytotoxicity in mice and human arthritis patients. Kang et al (Hum Reprod. 2014 Oct 10;29(10):2176-89) showed that NK cytotoxicity in peritoneal fluid of patients with endometriosis can be reversed using IL-6 neutralizing antibodies. Targeting the IL-6/STAT3 pathway in cancer therapy has been speculated by Wang et al. (PLoS One. 2013 Oct 7;8(10):e75788) with no supporting data provided. Additionally, IL-6 has also previously been shown to have a role in upregulating PD-L1 expression on certain myeloma cell lines (Tamura et al. Leukemia. 2013 Feb;27(2):464-72). Separately, others report that IL-6 antagonists led to reduced tumour control (Idorn et al. Cancer Immunol Immunother. 2017 May;66(5):667-671).

There exists a need for alternative and preferably improved cancer therapy using such NK cells, and using NK cells in general.

An object of the invention is to provide combination therapies using NK cells and NK cell lines that are more effective, e.g. more cytotoxic, than therapies relying only on the NK cells. More particular embodiments aim to provide treatments for identified cancers, e.g. blood cancers, such as leukemia.

## Summary

There are provided herein methods of treatment of cancer using antagonists to IL-6 in combination with NK cells. The cells may be the patient's, in which case an intervention may comprise administering the antagonist, hence relying on already present NK cells of the patient. The cells may be administered as part of the therapy, in which case they may be autologous, allogeneic, primary cells or cell lines, etc. Together these therapies are referred to as a combination in that both NK cells and the antagonists are required.

The invention provides methods of treatment comprising administering the antagonists, comprising administering the antagonists and the cells and comprising administering the cells (where the antibodies are separately present, for example as part of a related therapy). The invention provides the combination for use in treatment of cancer. The invention further provides compositions comprising both the cells and the antagonists.

Additionally, the invention provides NK cells modified so as to have reduced or absent expression of IL-6 receptors.

Diseases particularly treatable according to the invention using the NK cells described herein include cancers, e.g. blood cancers, e.g. leukemia, and specifically acute myeloid leukemia and myeloma. Tumours and cancers in humans in particular can be treated. References to tumours herein include references to neoplasms.

## Detailed description

Accordingly, the present invention uses a natural killer (NK) cell or NK cell line in a combination therapy. As described in detail below, NK cells and NK cell lines can also be genetically modified so as to increase their cytotoxic activity against cancer in such therapies. Together, primary NK cells and NK cell lines will be referred to as the NK cells (unless the context requires otherwise). The NK cells described herein further use antagonists of IL-6 signaling, alone or in combination with NK cells.

The invention hence provides, inter alia, a natural killer (NK) cell or cell line in combination with an IL-6 antagonist for use in treating cancer. The cancer suitably expresses IL-6 receptors and/or expresses one or more checkpoint inhibitory receptor ligands, e.g. PDL-1 and/or PDL-2.

Similarly, the invention provides methods of treating cancer comprising administering to a patient an effective amount of a combination of an NK cell and an IL-6 antagonist. Again, the cancer suitably expresses IL-6 receptors and/or expresses one or more checkpoint inhibitory receptor ligands, e.g. PDL-1 and/or PDL-2.

The NK cell or cell line is optionally provided with pre-bound IL-6 antagonist. Antagonist and cells can also be provided not pre-bound, in a single formulation, or in separate formulations.

This combination therapy may also be employed as an adjunct to other, separate anti-cancer therapy, such as those utilising endogenous NK cells as immune effector cells. Cancer treatment comprising antibody dependent cell-mediated cytotoxicity (ADCC) may thus be supplemented by employing the methods and compositions described herein.

NK cells or cell lines for use according to the invention may be genetically modified to have reduced expression of an IL-6 receptor. Separately, the NK cell line may be selected from known lines, e.g. NK-92, or KHYG-1 as used in examples below. The cell or cell line can exhibit a high level of expression of cell surface E-selectin ligand. E-selectin ligand expression is determined using the HECA-452 antibody. In a certain embodiment the NK cell or cell line exhibits a high level of cell-surface expression of E-selectin ligand. Preferably, a high level of cell surface expression of E-selectin ligand is exhibited by at least a 2, 4, 6, 8, or 10-fold increase in HECA-452 antibody binding compared to an isotype control antibody binding. This expression can be measured, for example by flow cytometry. The KHYG-1 cell line is one such cell line that expresses a high level of HECA-452 antigen compared to other NK cell lines, such as NK-92 cells. Other ways of modifying a cell to express a high level of E-selectin ligand include, for example: 1) chemical treatment with GDP-fucose substrate and the alpha 1,3 fucosyltransferase-VI enzyme; and 2) and expression or over expression of *FUT6* or *FUT7.* Additionally, the cell line can exhibit a low level of cell-surface expression of a TRAIL receptor, for example, DR4 or DR5. KHYG-1 cells, for example, express a low level of DR4 or DR5 compared to NK-92 cells. Cell surface TRAIL receptor expression can be quantified for example using flow cytometry as detailed in the examples.

Further provided herein are therapies in which NK cells are present already in the patient; hence the invention provides an IL-6 antagonist for use in treating cancer, wherein cells of the cancer express IL-6 receptors, and methods of treating cancer comprising administering an effective amount of an IL-6 antagonist.

As described in more detail in examples, the combination has been found effective in cancer models *in vitro,* and in particular wherein the cancer expresses IL-6 receptors. It is further noted that cancers that express one or more checkpoint inhibitory receptor ligands, e.g. in response to, or in the course of treatment, are treatable by the invention. In a specific example PDL-1 and/or PDL-2 were expressed by cancers treated using the combination of the invention.

The invention also provides compositions comprising an NK cell or cell line and an IL-6 antagonist. In the compositions, the cells are optionally modified according to one or more or all modifications described elsewhere herein.

In operating the invention, antagonists of IL-6 work by blocking IL-6 signal transduction and hence inhibit IL-6 activity.

Examples of IL-6 antagonists useful in the invention include antibodies, suitably as defined below. These include e.g. IL-6 antibodies, IL-6R antibodies and gp130 antibodies. These antibodies bind to IL-6, IL-6R or gp130 to inhibit binding between IL-6 and IL-6R, or IL-6R and gp130.

The antibodies thus block IL-6 signal transduction, inhibiting IL-6 activity, and hence reduce IL-6 signaling in NK cells and/or target (cancer) cells.

Useful antibodies hence reduce or stop the IL-6 signal as well as downstream effects on the NK cells / target. A feature of certain embodiments of the invention is that as well as a first effect in blocking direct IL-6 action on NK cells (IL-6 would otherwise reduce NK activity) there is a second effect in blocking the effect of IL-6 action on target (cancer) cells; IL-6 would otherwise promote or facilitate a response in the target that dampens the cytotoxic activity of NK cells. Specifically, blocking IL-6 action on target cells has been shown to prevent expression of checkpoint inhibitory receptor ligands on the target - hence, the target is more vulnerable to NK cell cytotoxicity.

Examples of IL-6 antibodies suitable for use in the combination therapies of the invention include siltuximab (an FDA-approved antibody), olokizumab (CDP6038), elsilimomab, BMS-945429 (Clazakizumab / ALD518) MH-166 and sirukumab (CNTO 136). Examples of IL-6R antibodies include tocilizumab (an FDA-approved antibody), sarilumab, PM-1 and AUK12-20. An example of a gp130 antibody is AM64.

Further IL-6 antagonists suitable for use in the combination therapies of the invention include mAb 1339 (OP-R003), PF-04236921, MEDI 5117, C326 (AMG-220), 6a, sgp130Fc (FE301), ALX-0061, NRI, SANT-7, ERBF, ERBA, MDL-A, SC144, Raloxifene (Keoxifene / LY156758 / Evista), Bazedoxifene (viviant) and LMT-28.

Monoclonal antibodies are prepared via conventional techniques, using one of e.g. IL-6, IL-6R or gp130 as a sensitizing antigen for immunization.

Antibodies specific for IL-6R may bind one or both of the two types of IL-6R that exist, i.e. membrane-bound IL-6R and soluble IL-6R (sIL-6R) which is separated from the cell membrane. sIL-6R consists mainly of the extracellular domain of IL-6R which is attached to the cell membrane, and it differs from the membrane-bound IL-6R in that it lacks the transmembrane domain and/or the intracellular domain.

The antibodies specific for IL-6, IL-6R or gp130 can be administered separately or simultaneously with NK cells or NK cell lines of the invention. Since the optimal pharmacokinetics of the NK cells and NK cell lines may differ from the optimal pharmacokinetics of the antibodies, the NK cells and NK cell lines can be administered on a different schedule than the IL-6, IL-6R or gp130 antibodies. For example, an antibody of the invention can be administered weekly while NK cells and cell lines can be administered twice -weekly. Another example is that an antibody of the invention can be administered every two weeks while NK cells and cell lines can be administered weekly.

As used herein, unless otherwise indicated, the term "antibody" includes antigen binding fragments of antibodies, i.e. antibody fragments that retain the ability to bind specifically to the antigen bound by the full-length antibody, e.g. fragments that retain one or more CDR regions. Examples of antibody fragments include, but are not limited to, Fab, Fab', F(ab')2, and Fv fragments; diabodies; linear antibodies; single-chain antibody molecules, e.g. single-chain variable region fragments (scFv), nanobodies and multispecific antibodies formed from antibody fragments with separate specificities, such as a bispecific antibody. Preferably the antibodies are humanized in such a way as to reduce an individual's immune response to the antibody. For example the antibodies may be chimeric, e.g. non-human variable region with human constant region, or CDR grafted, e.g. non-human CDR regions with human constant region and variable region framework sequences.

As noted above, a present observation is that, in addition to activity on NK cells, IL-6 signaling in cancer cells indirectly suppresses NK cell cytotoxicity by upregulating checkpoint inhibitory receptor (cIR) ligands on the cancer cell membrane, and this other effect of IL-6 signaling can also be prevented or reduced. These cIR ligands bind cIRs on NK cells and dampen NK cell cytotoxicity.

Checkpoint inhibitory receptor ligands expressed by IL-6R expressing cancers include ligands for the cIRs CD96 (TACTILE), CD152 (CTLA4), CD223 (LAG-3), CD279 (PD-1), CD328 (SIGLEC7), SIGLEC9, TIGIT and TIM-3.

IL-6 antagonists, according to the invention, may alone be administered to a patient in an effective dose. IL-6 antagonists are able to prevent IL-6 signal transduction in cells of the cancer and endogenous NK cells. The suppressive effects of IL-6 on NK cell cytotoxicity, both directly, via signaling in NK cells, and indirectly, via signaling in cancer cells, are prevented by IL-6 antagonists.

Thus, the invention provides IL-6 antagonists for use in treating cancers, such as those expressing IL-6R. The cancer to be treated may be a solid tissue tumor, e.g. a liver tumor, including hepatocellular carcinoma; a lung tumor; non-small cell lung cancer; a pancreatic tumor, including pancreatic adenocarcinoma or acinar cell carcinoma of the pancreas; a colon cancer, stomach cancer, kidney cancer, including renal cell carcinoma (RCC) and transitional cell carcinoma (TCC, also known as urothelial cell carcinoma); ovarian cancer; prostate cancer; breast cancer; or cervical cancer. The cancers to be treated are preferably hematological cancers, also referred to as blood cancers, including leukemias, myelomas and lymphomas. More preferably, the cancer to be treated is selected from acute lymphocytic leukemia (ALL), acute myeloid leukemia (AML), chronic lymphocytic leukemia (CLL), chronic myeloid leukemia (CML), hairy cell leukemia, T-cell prolymphocytic leukemia, large granular lymphocytic leukemia, Hodgkin's lymphoma, non-Hodgkin's lymphoma, including T-cell lymphomas and B-cell lymphomas, asymptomatic myeloma, smoldering multiple myeloma (SMM), multiple myeloma (MM) or light chain myeloma.

The combination therapies of the invention can be undertaken with NK cells in general, including but not limited to autologous cells or allogeneic cells or specific lines such as NK92 or KHYG-1 or others. Specific examples below utilize selected NK cells for illustrative purposes only. The therapies can utilize modified NK cells as now described.

In a first example of such modified cells, NK cells are provided / used having reduced or absent checkpoint inhibitory receptor (cIR) function. Thus in examples below, NK cells are produced that have one or more cIR genes knocked out. Preferably, these receptors are specific cIRs. Preferably still, these checkpoint inhibitory receptors are one or more or all of CD96 (TACTILE), CD152 (CTLA4), CD223 (LAG-3), CD279 (PD-1), CD328 (SIGLEC7), SIGLEC9, TIGIT and/or TIM-3.

NK cells may also be provided / used in which one or more inhibitory receptor signaling pathways are knocked out or exhibit reduced function - the result again being reduced or absent inhibitory receptor function. For example, signaling pathways mediated by SHP-1, SHP-2 and/or SHIP are knocked out by genetic modification of the cells.

The resulting NK cells exhibit improved cytotoxicity and are of greater use therefore in cancer therapy, especially blood cancer therapy, in particular treatment of leukemias and multiple myeloma.

In an embodiment, the genetic modification occurs before the cell has differentiated into an NK cell. For example, pluripotent stem cells (e.g. iPSCs) can be genetically modified to lose the capacity to express one or more checkpoint inhibitory receptors. The modified iPSCs are then differentiated to produce genetically modified NK cells with increased cytotoxicity.

It is preferred to reduce function of checkpoint inhibitory receptors over other inhibitory receptors, due to the expression of the former following NK cell activation. The normal or 'classical' inhibitory receptors, such as the majority of the KIR family, NKG2A and LIR-2, bind MHC class I and are therefore primarily involved in reducing the problem of self-targeting. Preferably, therefore, checkpoint inhibitory receptors are knocked out. Reduced or absent function of these receptors according to the invention prevents cancer cells from suppressing immune effector function (which might otherwise occur if the receptors were fully functional). Thus a key advantage of these embodiments of the invention lies in NK cells that are less susceptible to suppression of their cytotoxic activities by cancer cells; as a result they are useful in cancer treatment.

As used herein, references to inhibitory receptors generally refer to a receptor expressed on the plasma membrane of an immune effector cell, e.g. a NK cell, whereupon binding its complementary ligand resulting intracellular signals are responsible for reducing the cytotoxicity of said immune effector cell. These inhibitory receptors are expressed during both 'resting' and 'activated' states of the immune effector cell and are often associated with providing the immune system with a 'self-tolerance' mechanism that inhibits cytotoxic responses against cells and tissues of the body. An example is the inhibitory receptor family 'KIR' which are expressed on NK cells and recognize MHC class I expressed on healthy cells of the body.

Also as used herein, checkpoint inhibitory receptors are usually regarded as a subset of the inhibitory receptors above. Unlike other inhibitory receptors, however, checkpoint inhibitory receptors are expressed at higher levels during prolonged activation and cytotoxicity of an immune effector cell, e.g. a NK cell. This phenomenon is useful for dampening chronic cytotoxicity at, for example, sites of inflammation. Examples include the checkpoint inhibitory receptors PD-1, CTLA-4 and CD96, all of which are expressed on NK cells.

The invention hence also may use a NK cell lacking a gene encoding a checkpoint inhibitory receptor selected from CD96 (TACTILE), CD152 (CTLA4), CD223 (LAG-3), CD279 (PD-1), CD328 (SIGLEC7), SIGLEC9, TIGIT and TIM-3. In a certain embodiment, the NK cell or cell line lacks two or more of CD96 (TACTILE), CD152 (CTLA4), CD223 (LAG-3), CD279 (PD-1), CD328 (SIGLEC7), SIGLEC9, TIGIT and TIM-3. In a certain embodiment, the NK cell or cell line lacks two or more of CD96 (TACTILE), CD152 (CTLA4), CD279 (PD-1), or CD328 (SIGLEC7). In a certain embodiment, the NK cell or cell line lacks three or more of CD96 (TACTILE), CD152 (CTLA4), CD279 (PD-1), or CD328 (SIGLEC7). In a certain embodiment, the NK cell or cell line lack CD96 (TACTILE) and CD328 (SIGLEC7).

Alternatively the NK cell may exhibit reduced expression of a checkpoint inhibitory receptor selected from CD96 (TACTILE), CD152 (CTLA4), CD223 (LAG-3), CD279 (PD-1), CD328 (SIGLEC7), SIGLEC9, TIGIT and TIM-3. In a certain embodiment, the NK cell or cell line exhibits reduced expression of two or more of CD96 (TACTILE), CD152 (CTLA4), CD223 (LAG-3), CD279 (PD-1), CD328 (SIGLEC7), SIGLEC9, TIGIT and TIM-3. In a certain embodiment,

the NK cell or cell line exhibits reduced expression of two or more of CD96 (TACTILE), CD152 (CTLA4), CD279 (PD-1), or CD328 (SIGLEC7). In a certain embodiment, the NK cell or cell line exhibits reduced expression of three or more of CD96 (TACTILE), CD152 (CTLA4), CD279 (PD-1), or CD328 (SIGLEC7). In a certain embodiment, the NK cell or cell line exhibits reduced expression of CD96 (TACTILE) and CD328 (SIGLEC7). NK cells can be modified to reduce expression of a checkpoint inhibitory receptor by using, for example, siRNA, shRNA constructs (plasmid or viral vector), or antisense technology.

A NK cell lacking a gene can refer to either a full or partial deletion, mutation or otherwise that results in no functional gene product being expressed. In embodiments, the NK cell lacks genes encoding two or more of the inhibitory receptors.

More specific embodiments comprise a NK cell lacking a gene encoding a checkpoint inhibitory receptor selected from CD96 (TACTILE), CD152 (CTLA4) and CD279 (PD-1). Specific embodiments described below in more detail comprise a NK cell being a derivative of KHYG-1.

In examples described below, the inventors have reliably shown the cytotoxic effects of using siRNA to knock down expression of the checkpoint inhibitory receptor CD96 in KHYG-1 cells. CD96 knockdown (KD) KHYG-1 cells demonstrated enhanced cytotoxicity against leukemia cells at a variety of effector:target (E:T) ratios.

In other embodiments, modified NK cells are provided / used that express a TRAIL ligand or, preferably, a mutant (variant) TRAIL ligand. As further described in examples below, cytotoxicity-enhancing modifications of NK cells hence also include increased expression of both TRAIL ligand and/or mutated TRAIL ligand variants.

The resulting NK cells exhibit increased binding to TRAIL receptors and, as a result, increased cytotoxicity against cancers, especially blood cancers, in particular leukemias.

The mutants / variants preferably have lower affinity (or in effect no affinity) for 'decoy' receptors, compared with the binding of wild type TRAIL to decoy receptors. Such decoy receptors represent a class of TRAIL receptors that bind TRAIL ligand but do not have the capacity to initiate cell death and, in some cases, act to antagonize the death signaling pathway. Mutant / variant TRAIL ligands may be prepared according to WO 2009/077857.

The mutants / variants may separately have increased affinity for TRAIL receptors, e.g. DR4 and DR5. Wildtype TRAIL is typically known to have a $K_D$ of >2 nM for DR4, >5 nM for DR5 and >20 nM for the decoy receptor DcR1 (WO 2009/077857; measured by surface plasmon resonance), or around 50 to 100 nM for DR4, 1 to 10 nM for DR5 and 175 to 225 nM for DcR1 (Truneh, A. et al. 2000; measured by isothermal titration calorimetry and ELISA). Therefore, an increased affinity for DR4 is suitably defined as a $K_D$ of <2 nM or <50 nM, respectively, whereas an increased affinity for DR5 is suitably defined as a $K_D$ of <5 nM or <1 nM, respectively. A reduced affinity for decoy receptor DcR1 is suitably defined as a $K_D$ of >50 nM or >225 nM, respectively. In any case, an increase or decrease in affinity exhibited by the TRAIL variant/mutant is relative to a baseline affinity exhibited by wildtype TRAIL. The affinity is preferably increased at least 10%, more preferably at least 25%, at least 50%, or 100% compared with that exhibited by wildtype TRAIL.

The TRAIL variant preferably has an increased affinity for DR5 as compared with its affinity for DR4, DcR1 and DcR2. Preferably, the affinity is at least 1.5-fold, 2-fold, 5-fold, 10-fold, 100-fold, or even 1,000-fold or greater for DR5 than for one or more of DR4, DcR1 and DcR2. More preferably, the affinity is at least 1.5-fold, 2-fold, 5-fold, 10-fold, 100-fold, or even 1,000-fold or greater for DR5 than for at least two, and preferably all, of DR4, DcR1 and DcR2.

A key advantage of these embodiments of the invention lies in NK cells that have greater potency in killing cancer cells.

The combination therapies offer the potential for still further advances in effect against cancers.

Further specific embodiments comprise / use a NK cell expressing a mutant TRAIL ligand that has reduced or no affinity for TRAIL decoy receptors. Preferably, this NK cell is a derivative of KHYG-1. Further specific embodiments comprise a NK cell expressing a mutant TRAIL ligand that has reduced or no affinity for TRAIL decoy receptors and increased affinity for DR4 and/or DR5. In a certain embodiment, the TRAIL receptor variant comprises two amino acid mutations of human TRAIL, D269H and E195R. In another embodiment, the TRAIL receptor variant comprises three amino acid mutations of human TRAIL, G131R, N199R, and K201H.

In examples of the invention, described in more detail below, NK cells were genetically modified to express a mutant TRAIL. Modified KHYG-1 cells expressed mutant TRAIL, and NK-92 expressed a mutant TRAIL. The modified KHYG-1 cells exhibited improved cytotoxicity against cancer cell lines *in vitro*. KHYG-1 cells express TRAIL receptors (e.g. DR4 and DR5), but at low levels. Other preferred embodiments of the modified NK cells express no or substantially no TRAIL receptors, or do so only at a low level - sufficiently low that viability of the modified NK cells is not adversely affected by expression of the mutant TRAIL.

In an optional embodiment, treatment of a cancer using modified NK cells expressing TRAIL or a TRAIL variant is enhanced by administering to a patient an agent capable of upregulating expression of TRAIL death receptors on cancer cells. This agent may be administered prior to, in combination with or subsequently to administration of the modified NK cells. It is preferable, however, that the agent is administered prior to administering the modified NK cells.

In a preferred embodiment the agent upregulates expression of DR5 on cancer cells. The agent may optionally be a chemotherapeutic medication, e.g. Bortezomib, and administered in a low dose capable of upregulating DR5 expression on the cancer.

The invention is not limited to any particular agents capable of upregulating DR5 expression, but examples of DR5-inducing agents include Bortezomib, Gefitinib, Piperlongumine, Doxorubicin, Alpha-tocopheryl succinate and HDAC inhibitors.

According to a preferred embodiment of the invention, the mutant / variant TRAIL ligand is linked to one or more NK cell costimulatory domains, e.g. 41BB / CD137, CD3zeta / CD247, DAP12 or DAP10. Binding of the variant to its receptor on a target cell thus promotes apoptotic signals within the target cell, as well as stimulating cytotoxic signals in the NK cell.

According to further preferred embodiments of the invention, NK cells are provided and/or used that both have reduced checkpoint inhibitory receptor function and also express a mutant TRAIL ligand, as described in more detail above in relation to these respective NK cell modifications. In even more preferred embodiments, a NK cell expressing a mutant TRAIL ligand that has reduced or no affinity for TRAIL decoy receptors and may be a derivative of KHYG-1, further lacks a gene encoding a checkpoint inhibitory receptor selected from CD96 (TACTILE), CD152 (CTLA4), CD223 (LAG-3), CD279 (PD-1), CD328 (SIGLEC7), SIGLEC9, TIGIT and TIM-3.

The present invention also provides and/or uses NK cells and NK cell lines, preferably KHYG-1 cells and derivatives thereof, modified to express one or more CARs. In general, the CARs that bind to a cancer associated antigen, such as, CD38, CD319/SLAMF-7, TNFRSF17/BCMA, SYND1/CD138, CD229, CD47, Her2/Neu, epidermal growth factor receptor (EGFR), CD123/IL3-RA, CD19, CD20, CD22, Mesothelin, EpCAM, MUC1, MUC16, Tn antigen, NEU5GC, NeuGcGM3, GD2, CLL-1, HERV-K. Also contemplated are CARs that bind specifically to blood cancer antigens such as CD38, CD319/SLAMF-7, TNFRSF17/BCMA, SYND1/CD138, CD229, CD47, CD123/IL3-RA, CD19, CD20, CD22, GD2, CLL-1, HERV-K.

Suitably for cancer therapy uses, the CARs specifically bind to one or more ligands on cancer cells, e.g. CS1 (SLAMF7) on myeloma cells. For use in treating specific cancers, e.g. multiple myeloma, the CAR may bind CD38. For example, the CAR may include the binding properties of e.g. variable regions derived from, similar to, or identical with those from the known monoclonal antibody daratumumab. Such NK cells may be used in cancer therapy in combination with an agent that inhibits angiogenesis, e.g. lenalidomide. For use in therapy of cancers, especially leukemias and AML in particular, the CAR may bind to CLL-1.

The CAR-NKs may be bispecific, wherein their affinity is for two distinct ligands / antigens. Bispecific CAR-NKs can be used either for increasing the number of potential binding sites on cancer cells or, alternatively, for localizing cancer cells to other immune effector cells which express ligands specific to the NK-CAR. For use in cancer therapy, a bispecific CAR may bind to a target tumour cell and to an effector cell, e.g. a T cell, NK cell or macrophage. Thus, for example, in the case of multiple myeloma, a bispecific CAR may bind a T cell antigen (e.g. CD3, etc.) and a tumour cell marker (e.g. CD38, etc.). A bispecific CAR may alternatively bind to two separate tumour cell markers, increasing the overall binding affinity of the NK cell for the target tumour cell. This may reduce the risk of cancer cells developing resistance by downregulating one of the target antigens. An example in this case, in multiple myeloma, would be a CAR binding to both CD38 and CS-1/SLAMF7. Another tumour cell marker suitably targeted by the CAR is a "don't eat me" type marker on tumours, exemplified by CD47.

Optional features of the invention include providing further modifications to the NK cells and NK cell lines described above, wherein, for example, a Fc receptor (which can be CD16, CD32 or CD64, including subtypes and derivatives) is expressed on the surface of the cell. In use, these cells can show increased recognition of antibody-coated cancer cells and improve activation of the cytotoxic response.

Further optional features of the invention include adapting the modified NK cells and NK cell lines to better home to specific target regions of the body. NK cells of the invention may be targeted to specific cancer cell locations. In preferred embodiments for treatment of blood cancers, NK effectors of the invention are adapted to home to bone marrow. Specific NK cells are modified by fucosylation and/or sialylation to home to bone marrow. This may be achieved by genetically modifying the NK cells to express the appropriate fucosyltransferase and/or sialyltransferase, respectively. Increased homing of NK effector cells to tumour sites may also be made possible by disruption of the tumour vasculature, e.g. by metronomic chemotherapy, or by using drugs targeting angiogenesis (Melero et al, 2014) to normalize NK cell infiltration via cancer blood vessels.

Yet another optional feature of the invention is to provide / use modified NK cells and NK cell lines with an increased intrinsic capacity for rapid growth and proliferation in culture. This can be achieved, for example, by transfecting the cells to overexpress growth-inducing cytokines IL-2 and IL-15. Moreover, this optional alteration provides a cost-effective alternative to replenishing the growth medium with cytokines on a continuous basis.

The invention further provides / uses a method of making a modified NK cell or NK cell line, comprising genetically modifying the cell or cell line as described herein so as to increase its cytotoxicity. This genetic modification can be a stable knockout of a gene, e.g. by CRISPR, or a transient knockdown of a gene, e.g. by siRNA.

In a preferred embodiment, a stable genetic modification technique is used, e.g. CRISPR, in order to provide a new NK cell line with increased cytotoxicity, e.g. a derivative of KHYG-1 cells.

In embodiments, the method is for making a NK cell or NK cell line that has been modified so as to reduce inhibitory receptor function. Preferably, these inhibitory receptors are checkpoint inhibitory receptors.

More specific embodiments comprise a method for making a NK cell or NK cell line with reduced inhibitory receptor function, wherein the checkpoint inhibitory receptors are selected from CD96 (TACTILE), CD152 (CTLA4), CD223 (LAG-3), CD279 (PD-1), CD328 (SIGLEC7), SIGLEC9, TIGIT and TIM-3.

In preferred embodiments, the method comprises modifying the NK cells to reduce function of two or more of the inhibitory receptors.

The invention still further provides / uses a method of making a modified NK cell or NK cell line comprising genetically modifying the cell or cell line to express TRAIL ligand or mutant TRAIL (variant) ligand.

In embodiments, the method comprises modifying a NK cell or NK cell line to express mutant TRAIL ligand that has an increased affinity for TRAIL receptors. Preferably, the TRAIL receptors are DR4 and/or DR5. Preferred embodiments provide a method of modifying the NK cells or NK cell lines to express a mutant TRAIL ligand that has a reduced affinity for decoy TRAIL receptors.

In further preferred embodiments, the method comprises modifying a NK cell or NK cell line to remove function of a checkpoint inhibitory receptor and also to express a mutant TRAIL ligand with reduced or no binding affinity for decoy TRAIL receptors.

Further typical embodiments provide a method for making a NK cell or NK cell line, in which function of one or more checkpoint inhibitory receptors has been removed and/or a mutant TRAIL ligand is expressed, which has reduced or no binding affinity for decoy TRAIL receptors, and the cell is further modified to express a CAR or bispecific CAR. The properties of the CAR are optionally as described above.

In embodiments, the method comprises making a NK cell or NK cell line, in which function of one or more checkpoint inhibitory receptors has been removed and/or a mutant TRAIL ligand is expressed, which has reduced or no binding affinity for decoy TRAIL receptors, and the cell is optionally modified to express a CAR or bispecific CAR, and the cell is further modified to express one or more Fc receptors. Suitable Fc receptors are selected from CD16 (FcRIII), CD32 (FcRII) and CD64 (FcRI).

Preferred embodiments of all the above comprise a method of making NK cells and NK cell lines being a derivative of KHYG-1.

As per the objects of the invention, the modified NK cell, NK cell line or composition thereof with increased cytotoxicity are for use in treating cancer in a patient, especially blood cancer.

In even more preferred embodiments, invention is a NK cell line obtained as a derivative of KYHG-1 by reducing checkpoint inhibitory receptor function in a KHYG-1 cell or expressing a mutant TRAIL ligand in a KHYG-1 cell, or both, for use in treating blood cancer.

Modified NK cells, NK cell lines and compositions thereof described herein, above and below, are suitable for treatment of cancer, in particular cancer in humans, e.g. for treatment of cancers of blood cells or solid cancers. The NK cells and derivatives are preferably human NK cells. For human therapy, human NK cells are preferably used.

The invention further provides perse NK cells having reduced or absent IL-6R function, e.g. genetically modified NK cells lacking IL-6 receptor function. The NK cells of the invention may also be modified as described herein to have reduced or absent function of one or more cIRs, to express mutant TRAIL, or all three of these modifications.

Various routes of administration will be known to the skilled person to deliver active agents and combinations thereof to a patient in need. Embodiments of the invention are for blood cancer treatment. Administration of the modified NK cells and/or NK cell lines can be systemic or localized, such as for example via the intraperitoneal route.

In other embodiments, active agent is administered more directly. Thus administration can be directly intratumoural, suitable especially for solid tumours.

NK cells in general are believed suitable for the methods, uses and compositions of the invention. As per cells used in certain examples herein, the NK cell can be a NK cell obtained from a cancer cell line. Advantageously, a NK cell, preferably treated to reduce its tumourigenicity, for example by rendering it mortal and/or incapable of dividing, can be obtained from a blood cancer cell line and used in methods of the invention to treat blood cancer.

To render a cancer-derived cell more acceptable for therapeutic use, it is generally treated or pre-treated in some way to reduce or remove its propensity to form tumours in the patient. Specific modified NK cell lines used in examples are safe because they have been rendered incapable of division; they are irradiated and retain their killing ability but die within about 3-4 days. Specific cells and cell lines are hence incapable of proliferation, e.g. as a result of irradiation. Treatments of potential NK cells for use in the methods herein include irradiation to prevent them from dividing and forming a tumour *in vivo* and genetic modification to reduce tumourigenicity, e.g. to insert a sequence encoding a suicide gene that can be activated to prevent the cells from dividing and forming a tumour *in vivo*. Suicide genes can be turned on by exogenous, e.g. circulating, agents that then cause cell death in those cells expressing the gene. A further alternative is the use of monoclonal antibodies targeting specific NK cells of the therapy. CD52, for example, is expressed on KHYG-1 cells and binding of monoclonal antibodies to this marker can result in antibody-dependent cell-mediated cytotoxicity (ADCC) and KHYG-1 cell death.

As discussed in an article published by Suck et al, 2006, cancer-derived NK cells and cell lines are easily irradiated using irradiators such as the Gammacell 3000 Elan. A source of Cesium-137 is used to control the dosing of radiation and a dose-response curve between, for example, 1 Gy and 50 Gy can be used to determine the optimal dose for eliminating the proliferative capacity of the cells, whilst maintaining the benefits of increased cytotoxicity. This is achieved by assaying the cells for cytotoxicity after each dose of radiation has been administered.

There are significant benefits of using an irradiated NK cell line for adoptive cellular immunotherapy over the well-established autologous or MHC-matched T cell approach. Firstly, the use of a NK cell line with a highly proliferative nature means expansion of modified NK cell lines can be achieved more easily and on a commercial level. Irradiation of the modified NK cell line can then be carried out prior to administration of the cells to the patient. These irradiated cells, which retain their useful cytotoxicity, have a limited life span and, unlike modified T cells, will not circulate for long periods of time causing persistent side-effects.

Additionally, the use of allogeneic modified NK cells and NK cell lines means that MHC class I expressing cells in the patient are unable to inhibit NK cytotoxic responses in the same way as they can to autologous NK cytotoxic responses. The use of allogeneic NK cells and cell lines for cancer cell killing benefits from the previously mentioned GVL effect and, unlike for T cells, allogeneic NK cells and cell lines do not stimulate the onset of GVHD, making them a much preferred option for the treatment of cancer via adoptive cellular immunotherapy.

The modified NK cells can be administered in an amount greater than about $1\times10^6$ cells/kg, about $1\times10^7$ cells/kg, about $1\times10^8$ cells/kg, about $1\times10^9$ cells/kg, and about $1\times10^{10}$ cells/kg. In certain embodiments, the modified NK cells are administered in an amount between about $1\times10^6$ and about $1\times10^{11}$ cells/kg. In certain embodiments, the modified NK cells are administered in an amount between about $1\times10^7$ and about $1\times10^{10}$cells/kg. In certain embodiments, the modified NK cells are administered in an amount between about $1\times10^8$ and about $1\times10^{10}$ cells/kg. In certain embodiments, the modified NK cells are administered in an amount between about $1\times10^9$ and about $1\times10^{10}$ cells/kg. In certain embodiments, the modified NK cells are administered in an amount between about $1\times10^7$ and about $1\times10^9$ cells/kg. In certain embodiments, the modified NK cells are administered in an amount between about $1\times10^7$ and about $1\times10^8$cells/kg. In certain embodiments, the modified NK cells are administered in an amount between about $1\times10^8$and about $1\times10^9$ cells/kg. For a hematological cancer, cells can be administered intravenously. For a solid tissue cancer, cells can be administered intratumorally or intraperitoneally.

IL-6 antagonist antibodies, as well as combinations thereof, can be administered to a subject at a concentration of between about 0.1 and 30 mg/kg, such as about 0.4 mg/kg, about 0.8 mg/kg, about 1.6 mg/kg, or about 4 mg/kg of bodyweight. In a preferred embodiment, the IL-6 antagonist antibodies described herein, as well as combinations thereof, are administered to a recipient subject at a frequency of once every twenty-six weeks or less, such as once every sixteen weeks or less, once every eight weeks or less, or once every four weeks or less. In another preferred embodiment, the IL-6 antagonist antibodies are administered to a recipient subject at a frequency of about once per period of approximately one week, once per period of approximately two weeks, once per period of approximately three weeks or once per period of approximately four weeks.

It is understood that the effective dosage may depend on recipient subject attributes, such as, for example, age, gender, pregnancy status, body mass index, lean body mass, condition or conditions for which the composition is given, other health conditions of the recipient subject that may affect metabolism or tolerance of the composition, levels of IL-6 in the recipient subject, and resistance to the composition (for example, arising from the patient developing antibodies against the composition). The modified NK cells that are administered with an IL-6 antagonist can also be administered with certain adjuvants interleukin-2 (IL-2), interleukin 8 (IL-8), interleukin-12 (IL-12), interleukin-15 (IL-15), or proteasome inhibitor, such as bortezomib, carfilzomib, ixazomib, or a combination thereof. In certain embodiments, any of IL-2, IL-8, IL-12, IL-15, or a proteasome inhibitor can be administered to a patient before administration of a modified NK cell. In certain embodiments, any of IL-2, IL-8, IL-12, IL-15, or a proteasome inhibitor can be administered to a patient during administration of a modified NK cell. In certain embodiments, any of IL-2, IL-8, IL-12, IL-15, or a proteasome inhibitor can be administered to a patient after administration of a modified NK cell. In certain embodiments, the activity of IL-2, IL-8, IL-12, IL-15 can be supplied by a non-interleukin agonist for the IL-2, IL8, IL-12, and IL-15 receptors. For example, an interleukin-12 agonist can be ALT-803 or ALT-801; an interleukin-15 agonist can be NIZ985.

Also envisioned herein are certain treatment adjuvants primarily the use of metronomic cyclophosphamide or a tetracycline antibiotic. Either of these adjuvants can be administered before or during treatment with an modified NK cell. They can also be administered simultaneously during a treatment course with a modified NK cell and an IL-6 antagonist such as an IL-6 antibody.

A tetracycline antibody, such as doxycycline, can be administered at a concentration of between about 50 mg and about 300 mg per day, or at a concertation of between about 100 mg and 200 mg per day, either orally or intravenously. Other equivalent tetracycline antibiotics can be used as well, such as tetracycline, doxycycline, minocycline, tigecycline, demeclocycline, methacycline, chlortetracycline, oxytetracycline, lymecycline, meclocycline, or rolitetracycline.

Cyclophosphamide can be administered either orally or intravenously. In certain embodiments, the cyclophosphamide is administered in a metronomic fashion, for example, sustained low doses of cyclophosphamide. In certain embodiments, cyclophosphamide is administered orally at a dose of between about 100 mg to about 25 mg a day or every other day for one, two, three, four, or more weeks. In certain embodiments, cyclophosphamide is administered orally at a dose of about 50 mg a day for one, two, three, four, or more weeks. In certain embodiments, cyclophosphamide is administered intravenously at a dose of between about 1000 mg to about 250 mg a week for one, two, three, four, or more weeks. In certain embodiments, cyclophosphamide is administered intravenously at a dose of about 750 mg, 500 mg, 250 mg or less a week for one, two, three, four, or more weeks.

According to the invention, there is further provided a method of treating cancer comprising administering to a patient an effective amount of a combination of an NK cell and an IL-6 antagonist. The methods comprise preferred and optional features of other aspects of the invention described herein.

According to the invention, described herein, is a method of treating cancer comprising administering to a patient an effective amount of (a) an NK cell, and (b) an IL-6 antagonist. The NK cell and the IL-6 antagonist are optionally administered separately. Further optionally, the patient is pretreated with an IL-6 antagonist before administration of an NK cell.

According to the invention, there is further provided a pharmaceutical composition comprising an NK cell or cell line and an IL-6 antagonist. The pharmaceutical compositions comprise preferred and optional features of other aspects of the invention described herein.

As used herein singular articles such as "a" or "an" includes the plural unless the context clearly dictates otherwise.

As used herein the term "about" refers to an amount that is near the stated amount by about 10%, 5%, or 1%.

As used herein, unless otherwise indicated, the term "antibody" includes antigen binding fragments of antibodies, i.e. antibody fragments that retain the ability to bind specifically to the antigen bound by the full-length antibody, e.g. fragments that retain one or more CDR regions. Examples of antibody fragments include, but are not limited to, Fab, Fab', F(ab')2, and Fv fragments; diabodies; linear antibodies; single-chain antibody molecules, e.g. single-chain variable region fragments (scFv), nanobodies and multispecific antibodies formed from antibody fragments with separate specificities, such as a bispecific antibody. Preferably, the antibodies are humanized in such a way as to reduce an individual's immune response to the antibody. For example the antibodies may be chimeric, e.g. non-human variable region with human constant region, or CDR grafted, e.g. non-human CDR regions with human constant region and variable region framework sequences.

As set out in the claims and elsewhere herein, the invention provides the following embodiments:

1. A natural killer (NK) cell or cell line in combination with an IL-6 antagonist for use in treating cancer.
2. An NK cell or cell line for use according to embodiment 1, wherein the cancer expresses IL-6 receptors.
3. An NK cell or cell line for use according to embodiment 1 or 2, wherein the cancer expresses PDL-1 and/or PDL-2.
4. An NK cell or cell line for use according to any preceding embodiment, wherein the IL-6 antagonist is an antibody that binds one of IL-6, IL-6R or gp130.
5. An NK cell or cell line for use according to embodiment 4, wherein the IL-6 antibody is selected from siltuximab, olokizumab (CDP6038), elsilimomab, BMS-945429 (ALD518), MH-166 and sirukumab (CNTO 136).
6. An NK cell or cell line for use according to embodiment 5 or 6, wherein the IL-6R antibody is selected from tocilizumab, sarilumab, PM-1 and AUK12-20.
7. An NK cell or cell line for use according to embodiment 4, wherein the gp130 antibody is AM64.
8. An NK cell or cell line for use according to any preceding embodiment in combination with a separate anti-cancer therapy.
9. An NK cell or cell line for use according to embodiment 8, wherein the separate anti-cancer therapy utilises endogenous NK cells as immune effector cells.
10. An NK cell or cell line for use according to either embodiment 8 or 9, wherein the separate anti-cancer therapy is antibody dependent cell-mediated cytotoxicity (ADCC).
11. An NK cell or cell line for use according to any preceding embodiment, wherein the cancer is a blood cancer.
12. An NK cell or cell line for use according to embodiment 11, wherein the blood cancer is acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), multiple myeloma (MM) or light chain myeloma.
13. An NK cell or cell line for use according to any preceding embodiment, wherein the NK cell or cell line has been genetically modified to have reduced expression of one or more checkpoint inhibitory receptors.
14. An NK cell or cell line for use according to embodiment 13, wherein the checkpoint inhibitory receptors are selected from CD96 (TACTILE), CD152 (CTLA4), CD223 (LAG-3), CD279 (PD-1), CD328 (SIGLEC7), SIGLEC9, TIGIT and TIM-3.
15. An NK cell or cell line for use according to any preceding embodiment, wherein the NK cell or cell line has been genetically modified to express a mutant TRAIL ligand.

16. An NK cell or cell line for use according to embodiment 15, wherein the mutant TRAIL ligand has an increased affinity for TRAIL receptors, e.g. DR4 and/or DR5.

17. An NK cell or cell line for use according to embodiment 15 or 16, wherein the mutant TRAIL ligand has reduced affinity for decoy TRAIL receptors.

18. An NK cell or cell line for use according to any preceding embodiment, expressing a chimeric antigen receptor (CAR).

19. An NK cell or cell line for use according to embodiment 18, wherein the CAR is a bispecific CAR.

20. An NK cell or cell line for use according to embodiment 19, wherein the bispecific CAR binds two ligands on one cell type.

21. An NK cell or cell line for use according to embodiment 19, wherein the bispecific CAR binds one ligand on each of two distinct cell types.

22. An NK cell or cell line for use according to embodiments 11 and 22, wherein the ligand(s) for the CAR or bispecific CAR is/are expressed on a cancer cell.

23. An NK cell or cell line for use according to embodiment 22, wherein the ligands for the bispecific CAR are both expressed on a cancer cell.

24. An NK cell or cell line for use according to embodiment 22, wherein the ligands for the bispecific CAR are expressed on a cancer cell and an immune effector cell.

25. An NK cell or cell line for use according to any preceding embodiment, wherein the NK cell or cell line has been genetically modified to have reduced expression of an IL-6 receptor.

26. An NK cell or cell line for use according to any preceding embodiment, wherein the NK cell line is KHYG-1.

27. An IL-6 antagonist for use in treating cancer, wherein cells of the cancer express IL-6 receptors.

28. An IL-6 antagonist for use according to embodiment 27, wherein the cancer expresses PDL-1 and/or PDL-2.

29. An IL-6 antagonist for use according to embodiments 27-28, wherein the IL-6 antagonist is an antibody that binds one of IL-6, IL-6R or gp130.

30. An IL-6 antagonist for use according to embodiment 29, wherein the IL-6 antibody is selected from siltuximab, olokizumab (CDP6038), elsilimomab, BMS-945429 (ALD518), MH-166 and sirukumab (CNTO 136).

31. An IL-6 antagonist for use according to embodiment 29, wherein the IL-6R antibody is selected from tocilizumab, sarilumab, PM-1 and AUK12-20.

32. An IL-6 antagonist for use according to embodiment 29, wherein the gp130 antibody is AM64.

33. An IL-6 antagonist for use according to any of embodiments 27-32, wherein the IL-6 antagonist is used in combination with a separate anti-cancer therapy.

34. An IL-6 antagonist for use according to embodiment 33, wherein the separate anti-cancer therapy utilises endogenous NK cells as immune effector cells.

35. An IL-6 antagonist for use according either embodiment 33 or 34, wherein the separate anti-cancer therapy is antibody dependent cell-mediated cytotoxicity (ADCC).

36. An IL-6 antagonist for use according to any of embodiments 27-36, wherein the cancer is a blood cancer.

37. An IL-6 antagonist for use according to embodiment 36, wherein the blood cancer is acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), multiple myeloma (MM) or light chain myeloma.

38. A method of treating cancer comprising administering to a patient an effective amount of a combination of an NK cell and an IL-6 antagonist.

39. A method according to embodiment 38, wherein the cancer expresses IL-6 receptors.

40. A method according to any of embodiments 38-39, wherein the cancer expresses PDL-1 and/or PDL-2.

41. A method according to any of embodiments 38-40, wherein the NK cell or cell line is provided with pre-bound IL-6 antagonist.

42. A method according to embodiments 38-41, wherein the IL-6 antagonist is an antibody that binds one of IL-6, IL-6R or gp130.

43. A method according to embodiment 42, wherein the IL-6 antibody is selected from siltuximab, olokizumab (CDP6038), elsilimomab, BMS-945429 (ALD518), MH-166 and sirukumab (CNTO 136).

44. A method according to embodiment 42, wherein the IL-6R antibody is selected from tocilizumab, sarilumab, PM-1 and AUK12-20.

45. A method according to embodiment 42, wherein the gp130 antibody is AM64.

46. A method according to any of embodiments 38-45, used in combination with a separate anti-cancer therapy.

47. A method according to embodiment 46, wherein the separate anti-cancer therapy utilises endogenous NK cells as immune effector cells.

48. A method according either embodiment 46 or 47, wherein the separate anti-cancer therapy is antibody dependent cell-mediated cytotoxicity (ADCC).

49. A method according to any of embodiments 38-48, wherein the cancer is a blood cancer.
50. A method according to embodiment 49, wherein the blood cancer is acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), multiple myeloma (MM) or light chain myeloma.
51. A method according to any of embodiments 38-50, wherein the NK cell or cell line has been genetically modified to have reduced expression of one or more checkpoint inhibitory receptors.
52. A method according to embodiment 51, wherein the checkpoint inhibitory receptors are selected from CD96 (TACTILE), CD152 (CTLA4), CD223 (LAG-3), CD279 (PD-1), CD328 (SIGLEC7), SIGLEC9, TIGIT and TIM-3.
53. A method according to any of embodiments 38-52, wherein the NK cell or cell line has been genetically modified to express a mutant TRAIL ligand.
54. A method according to embodiment 53, wherein the mutant TRAIL ligand has an increased affinity for TRAIL receptors, e.g. DR4 and/or DR5.
55. A method according to either of embodiments 53 or 54, wherein the mutant TRAIL ligand has reduced affinity for decoy TRAIL receptors.
56. A method according to any of embodiments 38-55, wherein the NK cell or cell line expresses a chimeric antigen receptor (CAR).
57. A method according to embodiment 56, wherein the CAR is a bispecific CAR.
58. A method according to embodiment 57, wherein the bispecific CAR binds two ligands on one cell type.
59. A method according to embodiment 58, wherein the bispecific CAR binds one ligand on each of two distinct cell types.
60. A method according to either of embodiments 58 or 59, wherein the ligand(s) for the CAR or bispecific CAR is/are expressed on a cancer cell.
61. A method according to embodiment 60, wherein the ligands for the bispecific CAR are both expressed on a cancer cell.
62. A method according to embodiment 60, wherein the ligands for the bispecific CAR are expressed on a cancer cell and an immune effector cell.
63. A method according to any of embodiments 38-62, wherein the NK cell or cell line has been genetically modified to have reduced expression of the IL-6 receptor.
64. A method according to any of embodiments 38-63, wherein the NK cell line is KHYG-1.
65. A composition comprising an NK cell or cell line and an IL-6 antagonist, the NK cell being optionally modified as described herein.
66. An NK cell or cell line, modified to have reduced or absent function of IL-6 receptors.
67. An NK cell or cell line according to embodiment 66, genetically modified to have reduced or absent expression of IL-6R.

## Examples

The present invention is now described in more and specific details in relation to the production of NK cell line KHYG-1 derivatives, modified to exhibit more cytotoxic activity and hence ability to cause leukemia cell death in humans and in relation to use of IL-6 antagonists to increase NK cell cytotoxicity and reduce target (cancer)-induced anti-NK cell cytotoxicity responses.
The invention is now illustrated in specific embodiments with reference to the accompanying drawings in which:

Fig. 1 shows the DNA sequence of the LIR2 gene target region and marks the gRNA flanking regions;
Fig. 2 shows the DNA sequence of the CTLA4 gene target region and marks the gRNA flanking regions;
Fig. 3 shows the gRNA construct (expression vector) used for transfection;
Fig. 4 shows gel electrophoresis bands for parental and mutated LIR2 DNA, before and after transfection;
Fig. 5 shows gel electrophoresis bands for parental and mutated CTLA4 DNA, before and after transfection;
Fig. 6A is a FACS plot showing successful CD96 knockdown using electroporation;
Fig. 6B is a FACS plot showing successful CD96 knockdown using electroporation;
Fig. 7 is a bar chart showing increased cytotoxicity of CD96 knockdown KHYG-1 cells against K562 cells at various E:T ratios;
Fig. 8 shows knockdown of CD328 (Siglec-7) in NK-92 cells;
Fig. 9 shows enhanced cytotoxicity of NK Cells with the CD328 (Siglec-7) knockdown;
Fig. 10 shows a FACS plot of the baseline expression of TRAIL on KHYG-1 cells;
Fig. 11 shows a FACS plot of the expression of TRAIL and TRAIL variant after transfection of KHYG-1 cells;
Fig. 12 shows a FACS plot of the expression of CD107a after transfection of KHYG-1 cells;

Fig. 13 shows the effects of transfecting KHYG-1 cells with TRAIL and TRAIL variant on cell viability;
Fig. 14 shows a FACS plot of the baseline expression of DR4, DR5, DcR1 and DcR2 on both KHYG-1 cells and NK-92 cells;
Fig.s 15, 16 and 17 show the effects of expressing TRAIL or TRAIL variant in KHYG-1 cells on apoptosis of three target cell populations: K562, RPMI8226 and MM1.S, respectively;
Fig. 18 shows two FACS plots of DR5 expression on RPMI8226 cells and MM1.S cells, respectively, wherein the effects of Bortezomib treatment on DR5 expression are shown;
Fig. 19 shows FACS plots of apoptosis in Bortezomib-pretreated/untreated MM1.S cells co-cultured with KHYG-1 cells with/without the TRAIL variant;
Fig. 20 shows a FACS plot of perforin expression levels in KHYG-1 cells treated with 100 nM CMA for 2 hours;
Fig. 21 shows FACS plots of KHYG-1 cell viability after treatment with 100 nM CMA or vehicle;
Fig. 22 shows FACS plots of apoptosis in MM1.S cells co-cultured with KHYG-1 cells with/without the TRAIL variant and pretreated with/without CMA;
Fig. 23 shows FACS plots of apoptosis in K562 cells co-cultured with KHYG-1 cells with CD96-siRNA and/or TRAIL variant expression;
Fig. 24 shows FACS plots of apoptosis in MM1.S cells co-cultured with KHYG-1 cells with CD96-siRNA and/or TRAIL variant expression;
Fig. 25 shows FACS plots of apoptosis in RPMI8226 cells co-cultured with KHYG-1 cells or KHYG-1 cells previously exposed to IL-6 for 12 hours;
Fig. 26 shows FACS plots of apoptosis in RPMI8226 cells, with or without prior exposure to IL-6 for 12 hours, co-cultured with KHYG-1 cells;
Fig. 27 shows FACS plots of apoptosis in MM1.S cells co-cultured with KHYG-1 cells or KHYG-1 cells previously exposed to IL-6 for 12 hours;
Fig. 28 shows FACS plots of apoptosis in MM1.S cells, with or without prior exposure to IL-6 for 12 hours, co-cultured with KHYG-1 cells;
Fig. 29 shows FACS plots of apoptosis in K562 cells co-cultured with KHYG-1 cells or KHYG-1 cells previously exposed to IL-6 for 12 hours;
Fig. 30 shows FACS plots of apoptosis in K562 cells, with or without prior exposure to IL-6 for 12 hours, co-cultured with KHYG-1 cells;
Fig.s 31 and 32 show FACS plots of IL-6R (CD126) expression on KHYG-1 cells;
Fig.s 33 and 34 show FACS plots of gp130 (CD130) expression on KHYG-1 cells;
Fig. 35 shows a FACS plot of IL-6R (CD126) expression on NK-2 cells;
Fig. 36 shows a FACS plot of gp130 (CD130) expression on NK-92 cells;
Fig. 37 shows a FACS plot of IL-6R (CD126) and gp130 (CD130) expression on U266 cells;
Fig. 38 shows a FACS plot of IL-6R (CD126) and gp130 (CD130) expression on RPM18226 cells;
Fig. 39 shows FACS plots of IL-6R (CD126) and gp130 (CD130) expression on NCI-H929 cells;
Fig. 40 shows a FACS plot of IL-6R (CD126) and gp130 (CD130) expression on KMS11 cells;
Fig. 41 shows a FACS plot of IL-6R (CD126) and gp130 (CD130) expression on MM1.S cells;
Fig.s 42 and 43 show FACS plots of IL-6R (CD126) expression on K562 cells;
Fig. 44 shows a FACS plot of gp130 (CD130) expression on K562 cells;
Fig. 45 shows FACS plots of PD-L1 expression on RPMI8226 cells in the presence or absence of IL-6 for 48 hours;
Fig. 46 shows FACS plots of PD-L2 expression on RPMI8226 cells in the presence or absence of IL-6 for 48 hours;
Fig. 47 shows FACS plots of PD-L1 expression on NCI-H929 cells in the presence or absence of IL-6 for 48 hours;
Fig. 48 shows FACS plots of PD-L2 expression on NCI-H929 cells in the presence or absence of IL-6 for 48 hours;
Fig.s 49 and 50 show FACS plots of PD-L1 expression on MM1.S cells in the presence or absence of IL-6 for 48 hours;
Fig.s 51 and 52 show FACS plots of PD-L2 expression on MM1.S cells in the presence or absence of IL-6 for 48 hours;
Fig.s 53 and 54 show FACS plots of PD-L1 expression on U266 cells in the presence or absence of IL-6 for 48 hours;
Fig.s 55 and 56 show FACS plots of PD-L2 expression on U266 cells in the presence or absence of IL-6 for 48 hours;
Fig.s 57 - 60 show FACS plots of PD-L1 expression on U266 cells in the presence or absence of IL-6 blocking antibody for 48 hours;
Fig.s 61 - 64 show FACS plots of PD-L2 expression on U266 cells in the presence or absence of IL-6 blocking antibody for 48 hours;
Fig. 65 shows gel electrophoresis of STAT3-S727, STAT3-Tyr705, total STAT3, SHP-1, SHP-2 and P44/42 following KHYG-1 cell exposure to IL-2;
Fig. 66 shows gel electrophoresis of STAT3-S727, STAT3-Tyr705, total STAT3, SHP-1, SHP-2, P44/42 and total p44/42 following KHYG-1 cell exposure to IL-6;
Fig. 67 shows gel electrophoresis of P44/42, total p44/42 and actin following KHYG-1 cell exposure to IL-2 and IL-6;
Fig. 68 shows FACS plots of PD-1 expression on KHYG-1 cells cultured alone and after co-culture with K562, U937, HL60, Raji, RPMI8226, U266 or MM1.S cells for 24 hours;
Fig. 69 shows neutralizing IL-6 improves KHYG-1 cell cytotoxicity against U266 cells; and
Fig. 70 shows that IL-6 directly inhibits KHYG-1 cell cytotoxicity by decreasing NKG2D expression (70A) and increasing NKG2A expression (70B).

DNA, RNA and amino acid sequences are referred to below, in which:

SEQ ID NO: 1 is the full LIR2 DNA sequence;
SEQ ID NO: 2 is the LIR2 amino acid sequence;
SEQ ID NO: 3 is the LIR2 g9 gRNA sequence;
SEQ ID NO: 4 is the LIR2 g18 gRNA sequence;
SEQ ID NO: 5 is the LIR2 forward primer sequence;
SEQ ID NO: 6 is the LIR2 reverse primer sequence;
SEQ ID NO: 7 is the full CTLA4 DNA sequence;
SEQ ID NO: 8 is the CTLA4 amino acid sequence;
SEQ ID NO: 9 is the CTLA4 g7 gRNA sequence;
SEQ ID NO: 10 is the CTLA4 g15 gRNA sequence;
SEQ ID NO: 11 is the CTLA4 forward primer sequence; and
SEQ ID NO: 12 is the CTLA4 reverse primer sequence.

**Example 1 - Knockout of Inhibitory Receptor Function**

CRISPR/Cas9

Cells were prepared as follows, having inhibitory receptor function removed. gRNA constructs were designed and prepared to target genes encoding the 'classical' inhibitory receptor LIR2 and the 'checkpoint' inhibitory receptor CTLA4 in the human genome of NK cells. CRISPR/Cas9 genome editing was then used to knock out the LIR2 and CTLA4 target genes.

Two gRNA candidates were selected for each target gene and their cleavage efficacies in K562 cells determined. The sequences of the gRNA candidates are shown in Table 1 and the Protospacer Adjacent Motif (PAM) relates to the last 3 bases of the sequence. The flanking regions of the gRNA sequences on the LIR2 gene (SEQ ID NO: 1) and the CTLA4 gene (SEQ ID NO: 7) are shown in Figures 1 and 2, respectively.

Table 1. gRNA candidates and sequences

| Gene | Plasmid Name | Sequence |
|---|---|---|
| hLIR2 | SM682.LIR2.g9 | GAGTCACAGGTGGCATTTGGCGG (SEQ ID NO: 3) |
| | SM682.LIR2.g18 | CGAATCGCAGGTGGTCGCACAGG (SEQ ID NO: 4) |
| hCTLA4 | SM683.CTLA4.g7 | CACTCACCTTTGCAGAAGACAGG (SEQ ID NO: 9) |
| | SM683.CTLA4.g15 | CCTTGTGCCGCTGAAATCCAAGG (SEQ ID NO: 10) |

K562 cells were transfected with the prepared gRNA constructs (Figure 3) and subsequently harvested for PCR amplification. The presence of GFP expression was used to report successful incorporation of the gRNA construct into the K562 cells. This confirmed expression of the Cas9 gene and therefore the ability to knock out expression of the LIR2 and CTLA4 genes.

The cleavage activity of the gRNA constructs was determined using an *in vitro* mismatch detection assay. T7E1 endonuclease I recognises and cleaves non-perfectly matched DNA, allowing the parental LIR2 and CTLA4 genes to be compared to the mutated genes following CRISPR/Cas9 transfection and non-homologous end joining (NHEJ).

Figure 4 shows the resulting bands following agarose gel electrophoresis after knockout of the LIR2 gene with the g9 and g18 gRNA sequences. The three bands corresponding to each mutation relate to the parental gene and the two resulting strands following detection of a mismatch in the DNA sequence after transfection. The g9 gRNA sequence resulted in an 11% success rate of transfection, whereas the g18 gRNA resulted in 10%.

Figure 5 shows the resulting bands following agarose gel electrophoresis after knockout of the CTLA4 gene with the g7 and g15 gRNA sequences. The g7 gRNA sequence resulted in a 32% success rate of transfection, whereas the g15 gRNA resulted in 26%.

Following the successful knockout of LIR2 and CTLA4 in K562 cells, KHYG-1 cells were transfected with gRNA constructs.

KHYG-1 derivative clones having homozygous deletions were selected. A Cas9 / puromycin acetyltransferase (PAC) expression vector was used for this purpose. Successfully transfected cells were selected, based on their resistance to the antibiotic puromycin.

Cas9 RNP

Another protocol used for knockout of checkpoint inhibitory receptors in NK cells was that of Cas9 RNP transfection. An advantage of using this protocol was that similar transfection efficiencies were achievable but with significantly lower toxicity compared to using the DNA plasmids of the CRISPR/Cas9 protocol.

$1\times10^6$ KHYG1 cells were harvested for each transfection experiment. The cells were washed with PBS and spun down in a centrifuge. The supernatant was then discarded. The CRISPR RNP (RNA binding protein) materials were then prepared as follows:

(1) a 20μM solution of the required synthesized crRNA and tRNA (purchased from Dharmacon) was prepared.
(2) 4μl of crRNA (20μM) and 4μl of tRNA (20μM) were mixed together.
(3) The mixture was then added to 2μl Cas9 protein (5μg/μl).
(4) All of the components were mixed and incubated at room temperature for 10 minutes.

Following the Neon® Transfection System, the cells were mixed with Cas9 RNP and electroporation was performed using the following parameters:

Voltage: 1450v
Pulse width: 30ms
Pulse number: 1

The cells were then transferred to one well of a 12-well plate containing growth medium (inc. IL-2 and IL-15).

The cells were harvested after 48-72 hours to confirm gene editing efficiency by T7 endonuclease assay and/or Sanger sequencing. The presence of indels were confirmed, indicating successful knockout of CTLA4, PD1 and CD96 in KHYG1 cells.

Site-specific nucleases

Another protocol used for knockout of checkpoint inhibitory receptors in NK cells was that of XTN TALEN transfection. An advantage of using this protocol was that a particularly high level of specificity was achievable compared to wildtype CRISPR.

*Step 1: Preparation of Reagents*

KHYG-1 cells were assayed for certain attributes including transfection efficiency, single cell cloning efficiency and karyotype/copy number. The cells were then cultured in accordance with the supplier's recommendations.

Depending on the checkpoint inhibitory receptor being knockout out, nucleases were prepared by custom-design of at least 2 pairs of XTN TALENs. The step of custom-design includes evaluation of gene locus, copy number and functional assessment (i.e. homologs, off-target evaluation).

*Step 2: Cell Line Engineering*

The cells were transfected with the nucleases of Step 1; this step was repeated up to 3 times in order to obtain high levels of cutting and cultures were split and intermediate cultures maintained prior to each transfection.

Initial screening occurred several days after each transfection; the pools of cells were tested for cutting efficiency via the Cel-1 assay. Following the level of cutting reaching acceptable levels or plateaus after repeated transfections, the cells were deemed ready for single cell cloning.

The pooled cells were sorted to one cell per well in a 96-well plate; the number of plates for each pool was dependent on the single cell cloning efficiency determined in Step 1. Plates were left to incubate for 3-4 weeks.

*Step 3 - Screening and Expansion*

Once the cells were confluent in the 96-well plates, cultures were consolidated and split into triplicate 96-well plates; one plate was frozen as a backup, one plate was re-plated to continue the expansion of the clones and the final plate was used for genotype confirmation.

Each clone in the genotype plate was analyzed for loss of qPCR signal, indicating all alleles had been modified. Negative clones were PCR amplified and cloned to determine the nature of the indels and lack of any wildtype or in-frame indels.

Clones with the confirmed knockout were consolidated into no more than one 24-well plate and further expanded; typically 5-10 frozen cryovials containing $1\times10^6$ cells per vial for up to 5 individual clones were produced per knockout.

*Step 4 - Validation*

Cells were banked under aseptic conditions.

Basic release criteria for all banked cells included viable cell number (pre-freeze and post-thaw), confirmation of identity via STR, basic sterility assurance and mycoplasma testing; other release criteria were applied when necessary (karyotype, surface marker expression, high level sterility, knockout evaluation of transcript or protein, etc).

**Example 2 - Knockdown of Checkpoint Inhibitory Receptor CD96 Function via RNAi**

siRNA knockdown of CD96 in KHYG-1 cells was performed by electroporation. The Nucleofection Kit T was used, in conjunction with the Amaxa Nucleofector II, from Lonza, as it is appropriate for use with cell lines and can successfully transfect both dividing and non-dividing cells and achieves transfection efficiencies of up to 90%.

Control siRNA (catalog number: sc-37007) and CD96 siRNA (catalog number: sc-45460) were obtained from Santa Cruz Biotechnology. Antibiotic-free RPMI-1640 containing 10% FBS, 2mM L-glutamine was used for post-Nucleofection culture. Mouse anti-human CD96-APC (catalog number: 338409) was obtained from Biolegend for staining.

A 20μM of siRNA stock solution was prepared. The lyophilized siRNA duplex was resuspended in 33μl of the RNAse-free water (siRNA dilution buffer: sc-29527) to FITC-control/control-siRNA, in 165μl of the RNAse-free water for the target gene siRNA (siRNA CD96). The tube was heated to 90°C for 1 minute and then incubated at 37°C for 60 minutes. The siRNA stock was then stored at -20°C until needed.

The KHYG-1 cells were passaged one to two days before Nucleofection, as the cells must be in logarithmic growth phase.

The Nucleofector solution was warmed to room temperature (100ul per sample). An aliquot of culture medium containing serum and supplements was also pre-warmed at 37°C in a 50ml tube. 6-well plates were prepared by adding 1.5ml of culture medium containing serum and supplements. The plates were pre-incubated in a humidified 37°C / 5% $CO_2$ incubator.

$2\times10^6$ cells in 100μl Nucleofection solution was mixed gently with 4μl 20μM siRNA solution (1.5μg siRNA). Air bubbles were avoided during mixing. The mixture was transferred into Amaxa certified cuvettes and placed into the Nucleofector cuvette holder and program U-001 selected.

The program was allowed to finish, and the samples in the cuvettes were removed immediately. 500μl pre-equilibrated culture medium was then added to each cuvette. The sample in each cuvette was then gently transferred to a corresponding well of the prepared 6-well plate, in order to establish a final volume of 2ml per well.

The cells were then incubated in a humidified 37°C / 5% $CO_2$ incubator until transfection analysis was performed. Flow cytometry analysis was performed 16-24 hours after electroporation, in order to measure CD96 expression levels. This electroporation protocol was carried out multiple times and found to reliably result in CD96 knockdown in KHYG-1 cells (see e.g. Figures 6A and 6B).

**Example 3 - Enhanced Cytotoxicity of NK Cells with a CD96 Knockdown**

KHYG-1 cells with and without the CD96 knockdown were co-cultured with K562 cells at different effector:target (E:T) ratios.

Cytotoxicity was measured 4 hours after co-culture, using the DELFIA EuTDA Cytotoxicity Kit from PerkinElmer (Catalog number: AD0116).

Target cells K562 were cultivated in RPMI-1640 medium containing 10% FBS, 2mM L-glutamine and antibiotics. 96-well V-bottom plates (catalog number: 83.3926) were bought from SARSTEDT. An Eppendorf centrifuge 5810R (with plate rotor) was used to spin down the plate. A VARIOSKAN FLASH (with ScanIt software 2.4.3) was used to measure the fluorescence signal produced by lysed K562 cells.

K562 cells were washed with culture medium and the number of cells adjusted to $1\times10^6$ cells/mL with culture medium. 2-4mL of cells was added to 5μl of BATDA reagent and incubated for 10 minutes at 37°C. Within the cell, the ester bonds are hydrolysed to form a hydrophilic ligand, which no longer passes through the membrane. The cells were centrifuged at 1500RPM for 5 mins to wash the loaded K562 cells. This was repeated 3-5 times with medium containing 1mM Probenecid (Sigma P8761). After the final wash the cell pellet was resuspended in culture medium and adjusted to about 5 $\times 10^4$ cells/mL.

Wells were set up for detection of background, spontaneous release and maximum release. 100μL of loaded target cells (5,000 cells) were transferred to wells in a V-bottom plate and 100μL of effector cells (KHYG-1 cells) were added at varying cell concentrations, in order to produce effector to target ratios ranging from 1:1 to 20:1. The plate was centrifuged at 100xg for 1 minute and incubated for 4 hours in a humidified 5% $CO_2$ atmosphere at 37°C. For maximum release wells 10μL of lysis buffer was added to each well 15 minutes before harvesting the medium. The plate was centrifuged at 500xg for 5 minutes.

20μL of supernatant was transferred to a flat-bottom 96 well plate 200μL of pre-warmed Europium solution added. This was incubated at room temperature for 15 mins using a plate shaker. As K562 cells are lysed by the KHYG-1 cells, they release ligand into the medium. This ligand then reacts with the Europium solution to form a fluorescent chelate that directly correlates with the amount of lysed cells.

The fluorescence was then measured in a time-resolved fluorometer by using VARIOSKAN FLASH. The specific release was calculated using the following formula:

% specific release = Experiment release – Spontaneous release / Maximum release – Spontaneous release

Statistical analysis was performed using Graphpad Prism 6.04 software. A paired t test was used to compare the difference between siRNA CD96 knockdown KHYG-1 cells and control groups (n=3).

The specific release was found to be significantly increased in co-cultures containing the CD96 knockdown KHYG-1 cells. This was the case at all E:T ratios (see Figure 7).

As fluorescence directly correlates with cell lysis, it was confirmed that knocking down CD96 expression in KHYG-1 cells resulted in an increase in their ability to kill K562 cancer target cells.

**Example 4 - Enhanced Cytotoxicity of NK Cells with a CD328 (Siglec-7) Knockdown**

SiRNA-mediated knock-down of CD328 in NK-92 cells

Materials, reagents and instruments

Control siRNA (catalog number: sc-37007) and CD328 siRNA (catalog number: sc-106757) were bought from Santa Cruz Biotechnology. To achieve transfection efficiencies of up to 90% with high cell viability (>75%) in NK-92 cells with the Nucleofector™ Device (Nucleofector II, Lonza), a Nucleofector™ Kit T from Lonza was used. RPMI-1640 containing 10% FBS, 2mM L-glutamine, antibiotics free, was used for post-Nucleofection culture. Mouse anti-human CD328-APC (catalog number: 339206) was bought from Biolegend.

Protocol

To make 10μM of siRNA stock solution

- Resuspend lyophilized siRNA duplex in 66μl of the RNAse-free water (siRNA dilution buffer: sc-29527) to FITC-control/control-siRNA, in 330μl of the RNAse-free water for the target gene siRNA (siRNA CD328).
- Heat the tube to 90°C for 1 minute.
- Incubate at 37 °C for 60 minutes.
- Store siRNA stock at -20 °C if not used directly.
- One Nucleofection sample contains (for 100μl standard cuvette)
- Cell number: $2\times 10^6$ cells
- siRNA: 4μl of 10μM) stock
- Nucleofector solution: 100μl

Nucleofection

- Cultivate the required number of cells. (Passage one or two day before Nucleofection, cells must be in logarithmic growth phase).
- Prepare siRNA for each sample.
- Pre-warm the Nucleofector solution to room temperature (100μl per sample).
- Pre-warm an aliquot of culture medium containing serum and supplements at 37 °C in a 50ml tube. Prepare 6-well plates by filling with 1.5ml of cullture medium containing serum and supplements and pre-incubate plates in a humidified 37 °C /5% CO2 incubator.
- Take an aliquot of cell culture and count the cells to determine the cell density.
- Centrifuge the required number of cells at 1500rpm for 5 min. Discard supernatant completely so that no residual medium covers the cell pellet.
- Resuspend the cell pellet in room temperature Nucleofector Solution to a final concentration of $2\times10^6$ cells/100μl. Avoid storing the cell suspension longer than 15-20 min in Nucleofector Solution, as this reduces cell viability and gene transfer efficiency.
- Mix 100μl of cell suspension with siRNA.
- Transfer the sample into an amaxa certified cuvette. Make sure that the sample covers the bottom of the cuvette, avoid air bubbles while pipetting. Close the cuvette with the blue cap.
- Select the appropriate Nucleofector program (A-024 for NK-92 cells). Insert the cuvette into the cuvette holder (Nucleofector II: rotate the carousel clockwise to the final position) and press the "x" button to start the program.
- To avoid damage to the cells, remove the samples from the cuvette immediately after the program has finished (display showing "OK"). Add 500μl of the pre-warmed culture medium into the cuvette and transfer the sample into the prepared 6-well plate.
- Incubate cells in a humidified 37°C/5% $CO_2$ incubator. Perform flow cytometric analysis and cytotoxicity assay after 16-24 hours.

Results: we followed the above protocol and performed flow cytometry analysis of CD328 expression level in NK-92 cells. The results of one representative experiment is shown in Fig. 8, confirming successful knockdown.

Knocking down CD328 enhances cytotoxicity

Materials, reagents and instruments

DELFIA EuTDA cytotoxicity kit based on fluorescence enhancing ligand (Catalog nmber: AD0116) was bought from PerkinElmer. Target cells K562 were cultivated in RPMI-1640 medium containing 10% FBS, 2mM L-glutamine and antibiotics. 96-well V-bottom plates (catalog number: 83.3926) were bought from SARSTEDT. Eppendrof centrifuge 5810R (with plate rotor) was used to spin down the plate. VARIOSKAN FLASH (with Scanlt software 2.4.3) was used to measure the fluorescence signal produced by lysed K562 cells.

Protocol

- Load target K562 cells with the fluorescence enhancing ligand DELFIA BATDA reagent
- Wash K562 cells with medium, adjust the number of cells to $1\times10^6$ cells/mL with culture medium. Add 2-4 mL of cells to 5 μl of BATDA reagent, incubate for 10 minutes at 37°C.
- Spin down at 1500RPM for 5minutes to wash the loaded K562 cells for 3-5 times with medium containing 1mM Probenecid (Sigma P8761).
- After the final wash resuspend the cell pellet in culture medium and adjust to about $5\times10^4$ cells/mL.

Cytotoxicity assay

- Set up wells for detection of background, spontaneously release and maximum release.
- Pipette 100μL of loaded target cells (5,000 cells) to a V-bottom plate.
- Add 100μL of effector cells (NK-92) of varying cell concentrations. Effector to target ratio ranges from 1:1 to 20:1.
- Spin down the plate at 100xg of RCF for 1 minute.
- Incubate for 2 hours in a humidified 5% CO2 atmosphere at 37 °C. For maximum release wells, add 10 μL of lysis buffer to each well 15 minutes before harvesting the medium.
- Spin down the plate at 500xg for 5 minutes.

- Transfer 20 μL of supernatant to a flat-bottom 96 well plate, add 200 μL of pre-warmed Europium solution, incubate at room temperature for 15 minutes using plateshaker.
- Measure the fluorescence in a time-resolved fluorometer by using VARIOSKAN FLASH. The specific release was calculated using the following formula:
- % specific release = Experiment release - Spontaneous release / Maximum release - Spontaneous release

Results: we followed the above to determine the effect on cytotoxicity of the CD328 knockdown. The results of one representative experiment are shown in figure 9. As seen, cytotoxicity against target cells was increased in cells with the CD328 knockdown.

**Example 5** - **Protocol for Blood Cancer Therapy by Knockdown / Knockout of Checkpoint Inhibitory Receptors**

As demonstrated in the above Examples, checkpoint inhibitory receptor function can be knocked down or knocked out in a variety of ways. The following protocol was developed for use in treating patients with blood cancer:
Following diagnosis of a patient with a cancer suitable to be treated with the methods described herein, an aliquot of modified NK cells can be thawed and cultured prior to administration to the patient.
Alternatively, a transient mutation can be prepared using e.g. siRNA within a day or two, as described above. The MaxCyte Flow Electroporation platform offers a suitable solution for achieving fast large-scale transfections in the clinic.
The removal of certain checkpoint inhibitory receptors may be more beneficial than others. This is likely to depend on the patient and the cancer. For this reason, the cancer is optionally biopsied and the cancer cells are grown in culture ex *vivo.* A range of NK cells with different checkpoint inhibitory receptor modifications can thus be tested for cytotoxicity against the specific cancer. This step can be used to select the most appropriate NK cell or derivative thereof for therapy.
Following successful modification, the cells are resuspended in a suitable carrier (e.g. saline) for intravenous and/or intratumoural injection into the patient.

**Example 6** - **KHYG-1 Knock-in of TRAIL / TRAIL variant**

KHYG-1 cells were transfected with both TRAIL and TRAIL variant, in order to assess their viability and ability to kill cancer cells following transfection.
The TRAIL variant used is that described in WO 2009/077857. It is encoded by the wildtype TRAIL gene containing the D269H/E195R mutation. This mutation significantly increases the affinity of the TRAIL variant for DR5, whilst reducing the affinity for both decoy receptors (DcR1 and DcR2).

Baseline TRAIL Expression

Baseline TRAIL (CD253) expression in KHYG-1 cells was assayed using flow cytometry.
Mouse anti-human CD253-APC (Biolegend catalog number: 308210) and isotype control (Biolegend catalog number: 400122) were used to stain cell samples and were analyzed on a BD FACS Canto II flow cytometer.
KHYG-1 cells were cultured in RPMI 1640 medium containing 10% FBS, 2mM L-glutamine, penicillin (100 U/mL)/streptomycin (100 mg/mL) and IL-2 (10ng/mL). 0.5-1.0 $\times 10^6$ cells/test were collected by centrifugation (1500rpm $\times$ 5 minutes) and the supernatant was aspirated. The cells (single cell suspension) were washed with 4 mL ice cold FACS Buffer (PBS, 0.5-1% BSA, 0.1% NaN3 sodium azide). The cells were re-suspended in 100 μL ice cold FACS Buffer, add 5uL antibody was added to each tube and incubated for 30 minutes on ice. The cells were washed 3 times by centrifugation at 1500 rpm for 5 minutes. The cells were then re-suspended in 500 μL ice cold FACS Buffer and temporarily kept in the dark on ice.
The cells were subsequently analyzed on the flow cytometer (BD FACS Canto II) and the generated data were processed using FlowJo 7.6.2 software.
As can be seen in Fig. 10, FACS analysis showed weak baseline expression of TRAIL on the KHYG-1 cell surface.

TRAIL / TRAIL variant Knock-in by Electroporation

Wildtype TRAIL mRNA and TRAIL variant (D269H/195R) mRNA was synthesized by TriLink BioTechnologies, aliquoted and stored as -80°C. Mouse anti-human CD253-APC (Biolegend catalog number: 308210) and isotype control (Biolegend catalog number: 400122), and Mouse anti-human CD107a-PE (eBioscience catalog number: 12-1079-42) and isotype control (eBioscience catalog number: 12-4714) antibodies were used to stain cell samples and were analyzed on a BD FACS Canto II flow cytometer. DNA dye SYTOX-Green (Life Technologies catalog number: S7020; 5 mM Solution in DMSO) was used. To achieve transfection efficiencies of up to 90% with high cell viability in KHYG-1 cells with the Nucleofector™ Device (Nucleofector II, Lonza), a Nucleofector™ Kit T from Lonza was used. Antibiotics-free RPMI 1640 containing 10% FBS, L-glutamine (2mM) and IL-2 (10ng/mL) was used for post-Nucleofection culture.

KHYG-1 and NK-92 cells were passaged one or two days before Nucleofection, as the cells must be in the logarithmic growth phase. The Nucleofector solution was pre-warmed to room temperature (100 μl per sample), along with an aliquot of culture medium containing serum and supplements at 37°C in a 50 mL tube. 6-well plates were prepared by filling with 1.5 mL culture medium containing serum and supplements and pre-incubated in a humidified 37°C / 5% $CO_2$ incubator. An aliquot of cell culture was prepared and the cells counted to determine the cell density. The required number of cells was centrifuged at 1500rpm for 5 min, before discarding the supernatant completely. The cell pellet was re-suspended in room temperature Nucleofector Solution to a final concentration of $2\times10^6$ cells/100μl (maximum time in suspension = 20 minutes). 100 μl cell suspension was mixed with 10 μg mRNA (volume of RNA < 10 μL). The sample was transferred into an Amaxa-certified cuvette (making sure the sample covered the bottom of the cuvette and avoiding air bubbles). The appropriate Nucleofector program was selected (i.e. U-001 for KHYG-1 cells). The cuvettes were then inserted into the cuvette holder. 500 μl pre-warmed culture medium was added to the cuvette and the sample transferred into a prepared 6-well plate immediately after the program had finished, in order to avoid damage to the cells. The cells were incubated in a humidified 37°C / 5% $CO_2$ incubator. Flow cytometric analysis and cytotoxicity assays were performed 12-16 hours after electroporation. Flow cytometry staining was carried out as above.

As can be seen in Fig.s 11 and 12, expression of TRAIL / TRAIL variant and CD107a (NK activation marker) increased post-transfection, confirming the successful knock-in of the TRAIL genes into KHYG-1 cells.

Fig. 13 provides evidence of KHYG-1 cell viability before and after transfection via electroporation. It can be seen that no statistically significant differences in cell viability are observed following transfection of the cells with TRAIL / TRAIL variant, confirming that the expression of wildtype or variant TRAIL is not toxic to the cells. This observation contradicts corresponding findings in NK-92 cells, which suggest the TRAIL variant gene knock-in is toxic to the cells (data not shown). Nevertheless, this is likely explained by the relatively high expression levels of TRAIL receptors DR4 and DR5 on the NK-92 cell surface (see Fig. 14).

<u>Effects of TRAIL / TRAIL variant on KHYG-1 Cell Cytotoxicity</u>

Mouse anti-human CD2-APC antibody (BD Pharmingen catalog number: 560642) was used. Annexin V-FITC antibody (ImmunoTools catalog number: 31490013) was used. DNA dye SYTOX-Green (Life Technologies catalog number: S7020) was used. A 24-well cell culture plate (SARSTEDT AG catalog number: 83.3922) was used. Myelogenous leukemia cell line K562, multiple myeloma cell line RPMI8226 and MM1.S were used as target cells. K562, RPMI8226, MM1.S were cultured in RPMI 1640 medium containing 10% FBS, 2mM L-glutamine and penicillin (100 U/mL)/streptomycin (100 mg/mL).

As explained above, KHYG-1 cells were transfected with TRAIL / TRAIL variant.

The target cells were washed and pelleted via centrifugation at 1500rpm for 5 minutes. Transfected KHYG-1 cells were diluted to $0.5\times10^6$/mL. The target cell density was then adjusted in pre-warmed RPMI 1640 medium, in order to produce effector:target (E:T) ratios of 1:1.

0.5 mL KHYG-1 cells and 0.5 mL target cells were then mixed in a 24-well culture plate and placed in a humidified 37°C / 5% $CO_2$ incubator for 12 hours. Flow cytometric analysis was then used to assay KHYG-1 cell cytotoxicity; co-cultured cells (at different time points) were washed and then stained with CD2-APC antibody (5 μL/test), Annexin V-FITC (5 μL/test) and SYTOX-Green (5 μL/test) using Annexin V binding buffer.

Data were further analyzed using FlowJo 7.6.2 software. CD2-positive and CD2-negative gates were set, which represent KHYG-1 cell and target cell populations, respectively. The Annexin V-FITC and SYTOX-Green positive cells in the CD2-negative population were then analyzed for TRAIL-induced apoptosis.

Fig.s 15, 16 and 17 show the effects of both KHYG-1 cells expressing TRAIL or TRAIL variant on apoptosis for the three target cell lines: K562, RPMI8226 and MM1.S, respectively. It is apparent for all target cell populations that TRAIL expression on KHYG-1 cells increased the level of apoptosis, when compared to normal KHYG-1 cells (not transfected with TRAIL). Moreover, TRAIL variant expression on KHYG-1 cells further increased apoptosis in all target cell lines, when compared to KHYG-1 cells transfected with wildtype TRAIL.

Cells of the invention, expressing the TRAIL variant, offer a significant advantage in cancer therapy, due to exhibiting higher affinities for the death receptor DR5. When challenged by these cells, cancer cells are prevented from developing defensive strategies to circumvent death via a certain pathway. Thus cancers cannot effectively circumvent TRAIL-induced cell death by upregulating TRAIL decoy receptors, as the NK cell are modified so that they remain cytotoxic in those circumstances.

**Example 7 - Protocol for Blood Cancer Therapy using NK Cells with TRAIL Variants Knocked-in**

KHYG-1 cells were transfected with TRAIL variant, as described above in Example 6. The following protocol was developed for use in treating patients with blood cancer:
Following diagnosis of a patient with a cancer expressing IL-6, IL-6R or gp130, a DR5-inducing agent, e.g. Bortezomib, is administered, prior to administration of the modified NK cells, and hence is used at low doses to upregulate expression of DR5 on the cancer, making modified NK cell therapy more effective.
An aliquot of modified NK cells is then thawed, cultured and administered to the patient.
Since the TRAIL variant expressed by the NK cells used in therapy has a lower affinity for decoy receptors than wildtype TRAIL, there is increased binding of death receptors on the cancer cell surface, and hence more cancer cell apoptosis as a result.
Another option, prior to implementation of the above protocol, is to biopsy the cancer and culture cancer cells ex *vivo*. This step can be used to identify those cancers expressing particularly high levels of decoy receptors, and/or low levels of death receptors, in order to help determine whether a DR5-inducing agent is appropriate for a given patient. This step may also be carried out during therapy with the above protocol, as a given cancer might be capable of adapting to e.g. reduce its expression of DR5, and hence it may become suitable to treat with a DR5-inducing agent part-way through therapy.

**Example 8 - Low Dose Bortezomib Sensitizes Cancer Cells to NK Cells Expressing TRAIL Variant**

Bortezomib (Bt) is a proteasome inhibitor (chemotherapy-like drug) useful in the treatment of Multiple Myeloma (MM). Bortezomib is known to upregulate DR5 expression on several different types of cancer cells, including MM cells.
KHYG-1 cells were transfected with TRAIL variant, as described above in Example 6, before being used to target MM cells with or without exposure to Bortezomib.

Bortezomib-induced DR5 expression

Bortezomib was bought from Millennium Pharmaceuticals. Mouse anti-human DR5-AF647 (catalog number: 565498) was bought from BD Pharmingen. The stained cell samples were analyzed on BD FACS Canto II.

(1) MM cell lines RPMI8226 and MM1.S were grown in RPMI1640 medium (Sigma, St Louis, MO, USA) supplemented with 2 mM L-glutamine, 10 mM HEPES, 24 mM sodium bicarbonate, 0.01% of antibiotics and 10% fetal bovine serum (Sigma, St Louis, MO, USA), in 5% CO2 atmosphere at 37°C.
(2) MM cells were seeded in 6-well plates at $1\times10^6$/mL, 2mL/well.
(3) MM cells were then treated with different doses of Bortezomib for 24 hours.
(4) DR5 expression in Bortezomib treated/untreated MM cells was then analyzed by flow cytometry (Fig. 18).

Low dose Bortezomib treatment was found to increase DR5 expression in both MM cell lines (Fig. 18). DR5 upregulation was associated with a minor induction of apoptosis (data not shown). It was found, however, that DR5 expression could not be upregulated by high doses of Bortezomib, due to high toxicity resulting in most of the MM cells dying.

Bortezomib-induced sensitization of cancer cells

KHYG-1 cells were transfected with the TRAIL variant (TRAIL D269H/E195R), as described above in Example 6.

(1) Bortezomib treated/untreated MM1.S cells were used as target cells. MM1.S cells were treated with 2.5nM of Bortzeomib or vehicle (control) for 24 hours.
(2) 6 hours after electroporation of TRAIL variant mRNA, KHYG-1 cells were then cultured with MM cells in 12- well plate. After washing, cell concentrations were adjusted to $1\times10^6$/mL, before mixing KHYG-1 and MM1.S cells at 1:1 ratio to culture for 12 hours.
(3) Flow cytometric analysis of the cytotoxicity of KHYG-1 cells was carried out. The co-cultured cells were collected, washed and then stained with CD2-APC antibody (5 uL/test), AnnexinV-FITC (5 uL/test) and SYTOX-Green (5 uL/test) using AnnexinV binding buffer.
(4) Data were further analyzed using FlowJo 7.6.2 software. CD2-negative population represents MM1.S cells. KHYG-1 cells are strongly positive for CD2. Finally, the AnnexinV-FITC and SYTOX-Green positive cells in the CD2-negative population were analyzed.

Flow cytometric analysis of apoptosis was performed in Bortezomib-pretreated/untreated MM1.S cells co-cultured with KHYG-1 cells electroporated with/without TRAIL variant (Fig. 19).

It was found that Bortezomib induced sensitivity of MM cells to KHYG-1 cells expressing the TRAIL variant. The data therefore indicated that an agent that induced DR5 expression was effective in the model in increasing cytotoxicity against cancer cells, and hence may be useful in enhancing cancer therapy.

**Example 9 - Confirmation of Induced Apoptosis by the TRAIL Variant**

Despite the conclusive evidence of increased NK cell cytotoxicity resulting from TRAIL variant expression in the previous Examples, we wished to confirm whether the increased cytotoxicity resulted from inducing cancer cell apoptosis (most likely) or by inadvertently activating the NK cells to exhibit a more cytotoxic phenotype and hence kill cancer cells via perforin secretion.

Concanamycin A (CMA) has been demonstrated to inhibit perforin-mediated cytotoxic activity of NK cells, mostly due to accelerated degradation of perforin by an increase in the pH of lytic granules. We investigated whether the cytotoxicity of KHYG-1 cells expressing the TRAIL variant could be highlighted when perforin-mediated cytotoxicity was partially abolished with CMA.

<u>CMA-induced reduction of perforin expression</u>

Mouse anti-human perforin-AF647 (catalog number: 563576) was bought from BD pharmingen. Concanamycin A (catalog number: SC-202111) was bought from Santa Cruz Biotechnology. The stained cell samples were analyzed using a BD FACS Canto II.

(1) KHYG-1 cells were cultured in RPMI1640 medium containing 10%FBS (fetal bovine serum), 2mM L-glutamine, penicillin (100 U/mL)/streptomycin (100 mg/mL), and IL-2 (10ng/mL).
(2) KHYG-1 cells (6 hours after electroporation, cultured in penicillin/streptomycin free RPMI1640 medium) were further treated with 100nM CMA or equal volume of vehicle (DMSO) for 2 hours.
(3) The cells were collected ($1\times10^6$ cells/test) by centrifugation (1500rpm $\times$ 5 minutes) and the supernatant was aspirated.
(4) The cells were fixed in 4% paraformaldehyde in PBS solution at room temperature for 15 minutes.
(5) The cells were washed with 4 mL of FACS Buffer (PBS, 0.5-1% BSA, 0.1% sodium azide) twice.
(6) The cells were permeabilized with 1mL of PBS/0.1% saponin buffer for 30 minutes at room temperature.
(7) The cells were washed with 4 mL of PBS/0.1% saponin buffer.
(8) The cells were re-suspended in 100 uL of PBS/0.1% saponin buffer, before adding 5uL of the antibody to each tube and incubating for 30 minutes on ice.
(9) The cells were washed with PBS/0.1% saponin buffer 3 times by centrifugation at 1500 rpm for 5 minutes.
(10) The cells were re-suspended in 500 uL of ice cold FACS Buffer and kept in the dark on ice or at 4°C in a fridge briefly until analysis.
(11) The cells were analyzed on the flow cytometer (BD FACS Canto II). The data were processed using FlowJo 7.6.2 software.

CMA treatment significantly decreased the perforin expression level in KHYG-1 cells (Fig. 20) and had no negative effects on the viability of KHYG-1 cells (Fig. 21).

<u>Cytotoxicity of NK cell TRAIL variants in the presence of CMA</u>

KHYG-1 cells were transfected with the TRAIL variant (TRAIL D269H/E195R), as described above in Example 6.

(1) MM1.S cells were used as target cells.
(2) 6 hours after electroporation of TRAIL mRNA, KHYG-1 cells were treated with 100mM CMA or an equal volume of vehicle for 2 hours.
(3) The KHYG-1 cells were washed with RPMI1640 medium by centrifugation, and resuspended in RPMI1640 medium containing IL-2, adjusting cell concentrations to $1\times10^6$/mL.
(4) The MM1.S cells were re-suspended in RPMI1640 medium containing IL-2 adjusting cell concentrations to $1\times10^6$/mL.
(5) The KHYG-1 and MM1.S cells were mixed at 1:1 ratio and co-cultured for 12 hours.
(6) Flow cytometric analysis of the cytotoxicity of KHYG-1 cells was carried out. The co-cultured cells were washed and stained with CD2-APC antibody (5 uL/test).

(7) After washing, further staining was performed with AnnexinV-FITC (5 uL/test) and SYTOX-Green (5 uL/test) using AnnexinV binding buffer.

(8) Data were further analyzed using FlowJo 7.6.2 software. CD2-negative population represents MM1.S cells. KHYG-1 cells are strongly positive for CD2. The AnnexinV-FITC and SYTOX-Green positive cells in CD2-negative population were then analyzed.

It was again shown that NK cells expressing the TRAIL variant show higher cytotoxicity than control cells lacking expression of the TRAIL variant (Fig. 22). In this Example, however, it was further shown that CMA was unable to significantly diminish the cytotoxic activity of NK cells expressing TRAIL variant, in contrast to the finding for control NK cells treated with CMA.

NK cells without the TRAIL variant (control or mock NK cells) were shown to induce 48% cancer cell death in the absence CMA and 35.9% cancer cell death in the presence of CMA (Fig. 22). NK cells expressing the TRAIL variant were able to induce more cancer cell death than control NK cells both in the presence and absence of CMA. In fact, even with CMA present, NK cells expressing TRAIL variant induced more cancer cell death than control NK cells in the absence of CMA.

This data thus shows the importance of the TRAIL variant in increasing NK cell cytotoxicity against cancer cells via a mechanism less susceptible to perforin-related downregulation. Since perforin is used commonly by NK cells to kill target cells, and many cancer cells have developed mechanisms for reducing NK cell perforin expression, in order to evade cytotoxic attack, the NK cells of the invention represent a powerful alternative less susceptible to attenuation by cancer cells.

**Example 10 - Combined Expression of Mutant TRAIL Variant and Knockdown of Checkpoint Inhibitory Receptor CD96 in KHYG-1 Cells**

Increases in NK cell cytotoxicity were observed when knocking down checkpoint inhibitory receptor CD96 expression and also when expressing TRAIL variant. We also tested combining the two genetic modifications to provoke a synergistic effect on NK cell cytotoxicity.

CD96 expression was knocked down in KHYG-1 cells, as described in Example 2.

KHYG-1 cells were transfected with the TRAIL variant (TRAIL D269H/E195R), as described above in Example 6.

(1) 12 hours after electroporation KHYG-1 cells were co-cultured with target cells (K562 or MM1.S) at a concentration of $1\times10^6$/mL in 12-well plates (2mL/well) for 12 hours. The E:T ratio was 1:1.

(2) 12 hours after co-culture, the cells were collected, washed, stained with CD2-APC, washed again and further stained with AnnexinV-FITC (5 uL/test) and SYTOX-Green (5 uL/test) using AnnexinV binding buffer.

(3) Cell samples were analyzed using a BD FACS canto II flow cytometer. Data were further analyzed using FlowJo 7.6.2 software. CD2-negative population represents MM1.S cells. KHYG-1 cells are strongly positive for CD2. The AnnexinV-FITC and SYTOX-Green positive cells in the CD2-negative population were then analyzed.

Simultaneously knocking down CD96 expression and expressing TRAIL variant in KHYG-1 cells was found to synergistically enhance the cells' cytotoxicity against both K562 target cells (Fig. 23) and MM1.S target cells (Fig. 24). This was indicated by the fact that in both target cell groups, more cell death resulted from the simultaneous genetic modification than resulted from the individual modifications in isolation.

At the same time, further evidence showing knockdown of CD96 increases NK cell cytotoxicity was obtained (Fig. 23 & 24), in addition to further evidence showing expression of the TRAIL mutant/variant increases NK cell cytotoxicity (Fig. 23 & 24).

Example 11 - Direct and Indirect Effects of IL-6 on NK Cell Cytotoxicity

Materials and Methods

Recombinant Human IL-2 (Catalog: 200-02) and IL-6 (Catalog: 200-06) were bought from PEPROTECH. The IL-2 was reconstituted in 100mM acetic acid solution, aliquoted and stored at -80°C. The IL-6 was reconstituted in PBS containing 0.1% BSA, aliquoted and stored at -80°C.

RPMI1640 medium (Catalog: R8758) was bought from SIGMA-ALDRICH. Fetal bovine serum was bought from SIGMA-ALDRICH (Catalog: F7524). 100X Penicillin-Streptomycin solution stabilized with 10,000 units penicillin and 10mg streptomycin/mL (Catalog: P4333) was bought from SIGMA-ALDRICH. Horse serum for cell culture (Catalog: H1138-500ML) was bought from SIGMA-ALDRICH. Alpha MEM medium (Catalog: 12561056) was bought from Thermo Fisher Scientific.

PE labeled mouse anti-human CD126 (IL-6 receptor alpha chain) (Catalog: 551850), PE labeled mouse anti-human CD130 (gp130, IL-6 receptor-associated signal transducer) (Catalog#:555757), PE-labeled Mouse IgG1 k isotype control (Catalog: 555749), APC-labeled mouse anti-human CD2 (Catalog: 560642), FITC-labeled mouse anti-human CD2 (Catalog: 555326), APC-labeled mouse anti-human PD-L1 (Catalog: 563741) and APC-labeled mouse anti-human PD-L2 (Catalog: 557926) were bought from BD Pharmingen. APC-labeled mouse anti-human PD1 antibody (Catalog: 329907) was bought from Biolegend. Phosphor-Stat3(Ser727) mouse mAb (Catalog:9136), Phosphor-Shp-1(Tyr564) rabbit mAb (Catalog; 8849), Phosphor-Shp-2(Tyr580) rabbit mAb (Catalog: 5431), Phospho-p44/42 MAPK (Erk1/2) (Thr202/Tyr204) rabbit mAb (Catalog: 4370) and p44/42 MAPK (Erk1/2) rabbit mAb were bought from Cell Signaling Technology. Phosphor-Stat3(Tyr705) mouse mAb (Catalog: sc-81523) and rabbit anti-human Stat3 polyclonal antibody (Catalog: sc-7179) were bought from Santa Cruz Biotechnology. Mouse anti-beta actin (Catalog: A5441) was bought from SIGMA-ALDRICH. Functional IL-6 blocking antibody (Catalog: 501110) and the related isotype control antibody (Catalog: 400414) were bought from Biolegend.

DNA dye SYTOX® green (Catalog: s34860) for flow cytometric analysis of dead cells was bought from Life Technologies. Annexin V-FITC antibody (Catalog: 556419) for flow cytometric analysis of apoptotic cells was bought from BD Pharmingen.

NK cell line KHYG-1 was cultivated and maintained in RPMI1640 medium containing 10% FBS, 100 U/mL of penicillin / 100 mg/mL of streptomycin and 10ng/mL of IL-2. NK cell line NK-92 was cultured in alpha MEM containing 10% horse serum, 10% FBS, 100 U/mL of penicillin / 100 mg/mL of streptomycin and 10ng/mL of IL-2. Every 2-3 days, the medium for culturing KHYG-1 and NK-92 cells was changed or the cells were split at 1:2 or 1:3 dilution.

K562, U937, HL60, Raji, RPMI8226, U266, MM1.S, NCI-H929 and KMS11 cells were cultured in RPMI-1640 supplemented with 10% FBS and 100 U/mL of penicillin / 100 mg/mL of streptomycin.

IL-6 Receptor Expression

Flow cytometry was used to quantify expression levels of the IL-6 receptor and gp130 on various effector and target cells.

Cells (in log phase) were harvested by centrifugation (1500 rpm for 5 min) and a density of 1 million cells/test was used. The cells were washed with ice-cold PBS containing 0.1% BSA and 0.1% sodium azide. Cells were finally exposed to a PE-labeled CD126, CD130 or isotype control antibody (2.5μL/test) in 50μL of PBS containing 0.1% BSA and 0.1% sodium azide on ice for 30 min. After washing cells twice with ice-cold PBS, samples were acquired on a FACS Canto II (BD Biosciences). The results were analyzed using FlowJo 7.6.1 software

Figures 31 -44 show IL-6 receptor (CD126) and gp130 (CD130) expression on KHYG-1, NK-92, RPMI8226, MM1.S, NCI-H929, U266, KMS11 and K562 cells.

KHYG-1 cells expressed low levels of CD126 (Fig.s 31 and 32) and CD130 (Fig.s 33 and 34).

NK-92 cells expressed low levels of CD126 (Fig. 35) and CD130 (Fig. 36).

MM cells (U266, RPMI8226, NCI-H929, KMS11 and MM1.S) expressed relatively high levels of CD126 and CD130 (Fig.s 37 - 41, respectively).

Leukemic K562 cells, however, were CD126-negative (Fig.s 42 and 43) but expressed relatively high levels of CD130 (Fig. 44).

Direct Inhibition of NK Cell Cytotoxicity by IL-6

KHYG-1, RPMI8226, MM1.S and K562 cells were cultured and maintained as described above. Cells were harvested in log phase, washed and re-suspended in pre-warmed appropriate mediums, before adjusting the cell concentration to 1 million/mL. The concentration of target cells was adjusted according to the E:T (effector: target) ratio. 0.5 mL KHYG-1 cells and 0.5 mL target cells were added to one well of a 24-well plate. IL-6 was added to the wells at final concentration of 100ng/mL. The plates were then placed in a humidified 37°C / 5% $CO_2$ incubator for 12 hours (6 or 4 hours for K562 cells). The same mixture of KHYG-1 and target cells at time point 0hr were used as a control.

Flow cytometry was used to measure the cytotoxicity of KHYG-1 cells. The co-cultured cells were collected (at different time points), washed and then stained with CD2-APC antibody (2.5 uL/test) first, then stained with AnnexinV-FITC (2.5 uL/test) and SYTOX-Green (0.5 uL/test) using AnnexinV binding buffer. Data were further analyzed using FlowJo 7.6.1 software. CD2-positive and CD2-negative gates were set which represent KHYG-1 cells and target cells, respectively. KHYG-1 cells were 100% positive for CD2, but K562, RPMI8226 and MM1.S cells were CD2-negative. Finally, the percentage of AnnexinV-FITC and SYTOX-Green positive cells (dead cells) in CD2-negative population was analyzed.

Figures 25 - 30 show that IL-6 suppressed KHYG-1 cell cytotoxicity against RPMI8226, MM1.S and K562 target cells at an E:T ratio of 1:1.

In the presence of IL-6 (100ng/mL), the percentage of dead target cells decreased when co-cultured with KHYG-1 cells. This was the case for RPMI8226 cells after a 12hr incubation (Fig.s 25 and 26), MM1.S cells after a 12hr incubation (Fig.s 27 and 28) and K562 cells after a 6hr incubation (Fig. 29) or a 4hr incubation (Fig. 30). Since K562 cells are CD126 negative, the results show that the inhibitory effects on KHYG-1 cell cytotoxicity were directly mediated through the IL-6 receptor on KHYG-1 cells.

In order to further investigate the mechanism behind the observed IL-6-induced inhibition of KHYG-1 cytotoxicity, KHYG1 cells were stimulated with 50ng/mL of IL-6 in the presence of IL-2 for 24 hours, then NKG2D (activating receptor) and NKG2A (inhibitory receptor) expression levels were analyzed by flow cytometry. Negative control means FMO. APC anti-human NKG2D Antibody (catalog number #320807) was bought from Biolegend. APC anti-human NKG2A Antibody (catalog number FAB1059A) was bought from R&D systems.

As seen in Fig. 70, IL-6 directly inhibits KHYG-1 cell cytotoxicity by decreasing expression levels of the activating receptor NKG2D (70A), while increasing expression of the inhibitory receptor NKG2A (70B).

Indirect Inhibition of NK Cell Cytotoxicity by IL-6

MM cells in log phase were seeded at 0.5 million/mL in RPMI1640 medium containing 10% FBS. A final concentration of 100ng/mL IL-6 or same volume of vehicle (PBS) was used to treat MM cells for 48 hours. Then MM cells were harvested, washed, and stained with PD-L1 and PD-L2 antibodies (2.5 uL/test). Flow cytometric data were acquired using a FACS Canto II, and the results were further analyzed by FACSDIVA 8.0.1 software.

Figures 45 - 56 show that IL-6 increased expression of PD-L1 and PD-L2 on RPMI8226, NCI-H929, MM1.S and U266 cells.

IL-6 induced upregulation of PD-L1 was observed on RPMI8226 cells (Fig. 45), NCI-H929 cells (Fig. 47), MM1.S cells (Fig.'s 49 and 50) and U266 cells (Fig.'s 53 and 54).

IL-6 induced upregulation of PD-L2 was observed on RPMI8226 cells (Fig. 46), NCI-H929 cells (Fig. 48), MM1.S cells (Fig.'s 51 and 52) and U266 cells (Fig.'s 55 and 56).

As PD-L1 and PD-L2 are well-known to bind the checkpoint inhibitory receptor (cIR) PD-1 on NK cells, these data clearly show a second (indirect) mechanism through which IL-6 suppresses NK cell cytotoxicity by acting also on the cancer cells.

These two elucidated mechanisms indicated IL-6 as a key cytokine involved in cancer cell survival.

Effect of IL-6 Antagonism

U266 cells in log phase were seeded at 0.5 million/mL in RPMI1640 medium containing 10% FBS. A final concentration of 10μg/mL rat anti-human IL-6 mAb or isotype control antibody was added to block IL-6 secreted by U266 cells. At a time point of 48 hours, U266 cells were harvested, washed, and stained with PD-L1 and PD-L2 antibodies (2.5 uL/test). Flow cytometric data were acquired using a FACS Canto II, and the results were further analyzed using FACSDIVA 8.0.1 software.

Figures 57 - 60 show that PD-L1 expression on U266 cells was significantly decreased in the presence of an IL-6 blocking antibody.

Figures 61 - 64 show that PD-L2 expression on U266 cells was significantly decreased in the presence of an IL-6 blocking antibody.

Antagonism of IL-6 signaling is therefore shown to be achievable by using an IL-6 blocking antibody.

These data confirm that IL-6 signaling is responsible for regulating expression of PD-L1 and PD-L2 on the cancer cell.

Blocking IL-6 signaling, as per the invention, therefore has use in the treatment of cancer, since both the direct and indirect IL-6 induced suppression of NK cell cytotoxicity is prevented. Furthermore, any direct proliferative or anti-apoptotic effects of IL-6 on the cancer cell are also prevented by blocking IL-6 signaling.

In order to further demonstrate this, KHYG-1 cells were stimulated with U266 cells as above in the presence of 2 μg/mL of IL-6 blocking mAb (LEAF™ Purified anti-human IL-6 antibody, Biolegend, catalog number #501110) or the same dose of isotype control antibody (Biolegend, catalog number #400413).

As seen in Fig. 69, blocking IL-6 using the mAb recovered KHYG-1 cytotoxicity against U266 cells (E:T ratio of 1:1; 12hr incubation). Thus, in addition to the above data for RPMI8226, MM1.S and K562 target cells, this showed that inhibiting IL-6 signaling increases the ability of KHYG-1 cells to kill target cancer cells in another cancer cell line.

IL-6 Signaling in NK Cells

KHYG-1 cells were cultivated and maintained as mentioned above. When testing the effects of IL-6 alone, KHYG-1 cells were starved in RPMI160 medium supplemented with 10%FBS (no IL-2) for 6 hours, then stimulated with 10ng/mL of IL-2 and/or 100ng/mL of IL-6.

KHYG-1 cells growing in normal conditions need IL-2 to promote cell proliferation and maintain cytotoxicity.

As shown in figure 65, it was found that IL-2 alone activated p-STAT3 and p-P44/42 but decreased expression of p-SHP1 and p-SHP2.

As shown in figure 66, IL-6 alone activated p-STAT3, p-SHP1 and p-SHP2 but decreased expression of p-P44/42.

As shown in figure 67, in the presence of IL-2, IL-6 remained capable of decreasing p-P44/42 expression and increasing p-SHP1 and p-SHP2 expression.

It has been demonstrated in models (including NK cells) that p-STAT3 is a major downstream effector of the IL-6 signaling pathway and the level of p-P44/42 is positively associated with NK cell cytotoxicity.

The above data show that p-SHP1 and p-SHP2 play an important role in regulating the levels of p-P44/42. Furthermore, it is herein shown that IL-6 anti-cytotoxic signaling overcomes that of IL-2 pro-cytotoxic signaling, leading to an overall decrease in NK cell cytotoxicity, and can effectively be reversed - thus IL-6 antagonism can be offered as a cancer therapy.

Cancer Cell Induced Upregulation of PD-1 expression on NK Cells

KHYG-1 cells were cultivated and maintained in RPMI1640 medium containing 10% FBS, 100 U/mL penicillin / 100 mg/mL streptomycin and 10ng/mL IL-2. K562, U937, HL60, Raji, RPMI8226, U266 and MM1.S cells were cultured in RPMI1640 supplemented with 10% FBS and 100 U/mL penicillin / 100 mg/mL streptomycin. Cells were harvested in log phase, washed and re-suspended in pre-warmed RPMI1640 medium containing IL-2 (medium for culturing KHYG-1 cells), before adjusting the cell concentration to 1 million/mL. The concentration of target cells was adjusted according to the E:T (effector:target) ratio. 0.5 mL of KHYG-1 cells and 0.5 mL of target cells was added to one well of a 24-well plate and cultured for 24 hours. The cells were harvested, washed and stained with CD2-FITC (2.5 uL/test) and PD1-APC (2.5 uL/test) antibodies. Samples were acquired using a FACS Canto II, and analyzed using FACSDiva 8.0.1 software. CD2-positive and CD2-negative gates represent KHYG-1 cells and target cells, respectively. KHYG-1 cells are 100% positive for CD2, but MM cells and other malignant blood cancer cell lines are CD2-negative. PD-1 expression in the CD2-positive population (i.e. KHYG-1 cells) was thus analyzed.

As can be seen from figure 68, flow cytometric analysis of PD-1 expression in KHYG-1 cells co-cultured with different blood cancer cell lines (E:T ratio = 1:1) revealed that PD-1 expression was significantly induced by all of the tested blood cancer cell lines.

These data highlight the suppressive effects of cancer cells on NK cell cytotoxicity, as higher PD-1 expression on NK cells leads to a greater degree of cytotoxic inhibition by those cancers expressing PD-L1 and/or PD-L2.

It is thus shown herein that cancer cells upregulate expression of cIR PD-1 on NK cells, whilst IL-6 both directly decreases NK cell cytotoxicity and upregulates expression of PD-L1 and PD-L2 on cancer cells. The increased PD-1 expression on NK cells and increased PD-L1 and PD-L2 expression on cancer cells work together to significantly suppress NK cytotoxicity. In addition, the IL-6 directly promotes cancer cell proliferation.

Blocking IL-6 signaling, as shown above, has therapeutic application in reducing the survival of cancer cells, especially those cancer cells expressing IL-6 receptors.

IL-6 Antagonist Treatment Protocol

The following protocol was developed for use in treating patients with multiple myeloma. Nevertheless, it is apparent that the invention is suitable for treating patients with many different cancers including IL-6R-expressing cancers.

Following diagnosis of a patient with an IL-6R positive cancer, in this case multiple myeloma, an aliquot of NK cells is thawed and cultured prior to administration to the patient in an effective dose. The aliquoted cells may be modified as described elsewhere herein. Alternatively, a transient transfection can be prepared using e.g. viral means, electroporation etc. For electroporation, the MaxCyte Flow Electroporation platform offers a suitable solution for achieving fast large-scale transfections in the clinic. After NK cells are transfected, they are cultured to allow for expression of the modification and then administered intravenously to the patient.

Prior to, simultaneously with or subsequent to administration of NK cells, an IL-6 antagonist is administered in an effective dose to the patient. This IL-6 antagonist may be one antagonist or a combination thereof. The IL-6 antagonist(s) may bind IL-6, IL-6R, gp130 etc., provided that the result of binding reduces (antagonizes) IL-6 signaling.

The invention thus provides antagonism of IL-6 signaling in NK cell-based cancer therapy.

## Claims

1. A natural killer (NK) cell or cell line in combination with an antibody antagonist against IL-6, IL-6R or gp130 for use in treating a solid tissue tumor.

2. An NK cell or cell line for use according to claim 1, wherein the solid tissue tumor is selected from a liver tumor, including hepatocellular carcinoma; a lung tumor; non-small cell lung cancer; a pancreatic tumor, including pancreatic adenocarcinoma or acinar cell carcinoma of the pancreas; a colon cancer, stomach cancer, kidney cancer, including renal cell carcinoma (RCC) and transitional cell carcinoma (TCC, also known as urothelial cell carcinoma); ovarian cancer; prostate cancer; breast cancer; and cervical cancer

3. An NK cell or cell line for use according to claim 1 or claim 2, wherein the solid tissue tumor expresses PDL-1 and/or PDL-2.

4. An NK cell or cell line for use according to any preceding claim, wherein the NK cell or cell line has been genetically modified to have reduced expression of one or more checkpoint inhibitory receptors selected from CD96 (TACTILE), CD152 (CTLA4), CD223 (LAG-3), CD279 (PD-1), CD328 (SIGLEC7), SIGLEC9, TIGIT and TIM-3.

5. An NK cell or cell line for use according to claim 4, wherein the checkpoint inhibitory receptor is PD-1.

6. An NK cell or cell line for use according to any preceding claim, wherein the antibody antagonist binds to IL-6.

7. An NK cell or cell line for use according to claim 6, wherein the IL-6 antibody is selected from siltuximab, olokizumab (CDP6038), elsilimomab, BMS-945429 (ALD518), MH-166 and sirukumab (CNTO 136).

8. An NK cell or cell line for use according to any of claims 1-5, wherein the antibody antagonist binds to IL-6R.

9. An NK cell or cell line for use according to claim 8, wherein the IL-6R antibody is selected from tocilizumab, sarilumab, PM-1 and AUK12-20.

10. An NK cell or cell line for use according to any preceding claim in combination with a separate anti-cancer therapy that utilises endogenous NK cells as immune effector cells.

11. An NK cell or cell line for use according to either claim 10, wherein the separate anti-cancer therapy is antibody dependent cell-mediated cytotoxicity (ADCC).

12. An NK cell or cell line for use according to any preceding claim, wherein the NK cell or cell line has been genetically modified to express a mutant TRAIL ligand that has an increased affinity for TRAIL receptors, e.g. DR4 and/or DR5, and/or a reduced affinity for decoy TRAIL receptors.

13. An NK cell or cell line for use according to any preceding claim, wherein the NK cell or cell line has been genetically modified to have reduced expression of an IL-6 receptor.

14. An NK cell or cell line for use according to any preceding claim, expressing a chimeric antigen receptor (CAR).

15. An NK cell or cell line for use according to claim 14, wherein the CAR binds CD38.

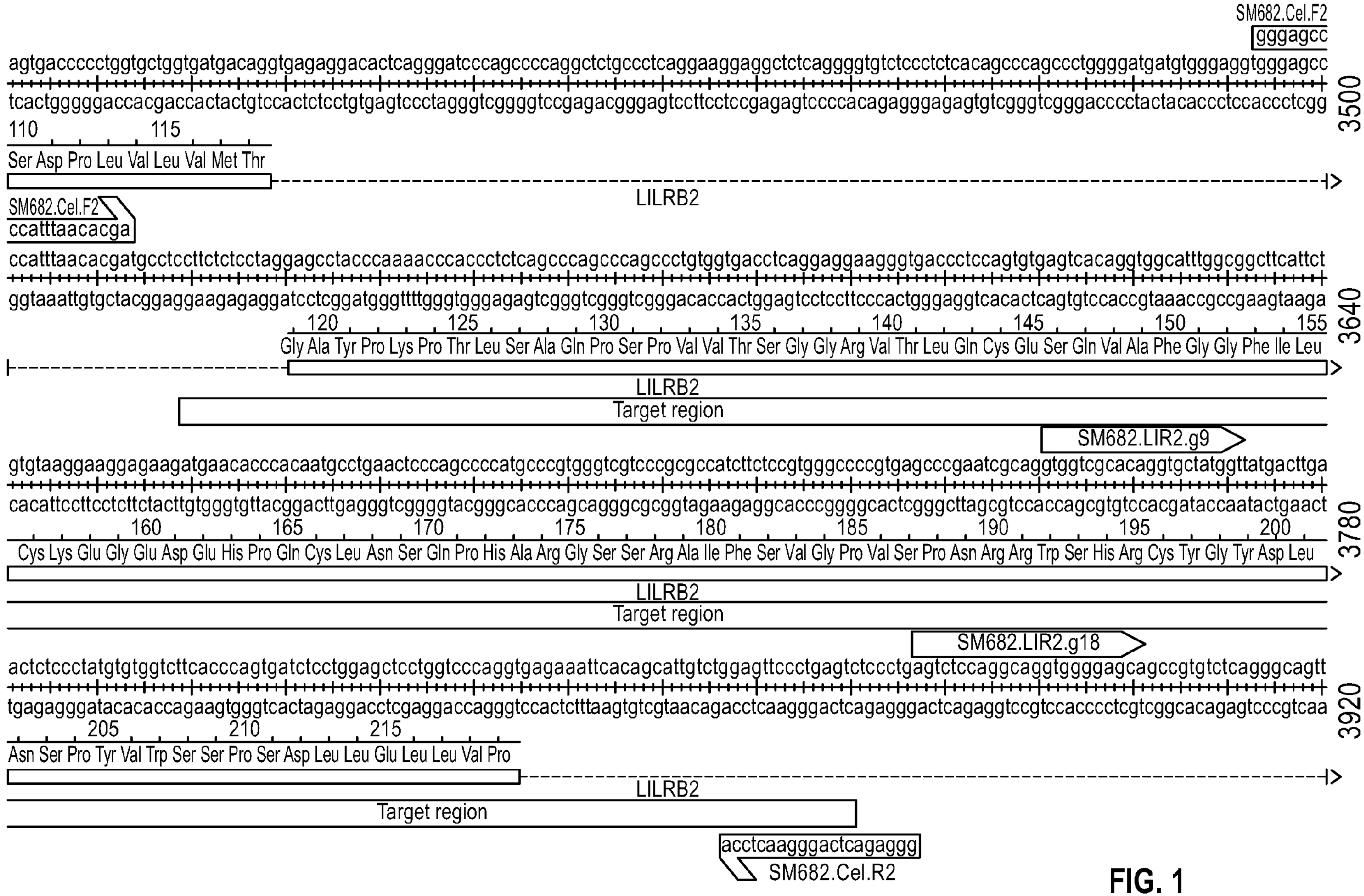
SM682.Cel.F2
gggagcc
agtgacccccctggtgctggtgatgacaggtgagaggacactcagggatcccagccccaggctctgccctcaggaaggaggctctcaggggtgtctccctctcacagcccagccctggggatgatgtgggaggtgggagcc
tcactggggggaccacgaccactactgtccactctcctgtgagtccctagggtcggggtccgagacgggagtccttcctccgagagtccccacagagggagagtgtcgggtcgggaccccctactacaccctccaccctcgg
3500
110
115
Ser Asp Pro Leu Val Leu Val Met Thr
LILRB2
SM682.Cel.F2
ccatttaacacga
ccatttaacacgatgcctccttctctcctaggagcctacccaaaacccaccctctcagcccagcccagccctgtggtgacctcaggaggaagggtgaccctccagtgtgagtcacaggtggcatttggcggcttcattct
ggtaaattgtgctacggaggaagagaggatcctcggatgggttttgggtgggagagtcgggtcgggtcgggacaccactggagtcctccttcccactgggaggtcacactcagtgtccaccgtaaaccgccgaagtaaga
3640
120
125
130
135
140
145
150
155
Gly Ala Tyr Pro Lys Pro Thr Leu Ser Ala Gln Pro Ser Pro Val Val Thr Ser Gly Gly Arg Val Thr Leu Gln Cys Glu Ser Gln Val Ala Phe Gly Gly Phe Ile Leu
LILRB2
Target region
SM682.LIR2.g9
gtgtaaggaaggagaagatgaacacccacaatgcctgaactcccagccccatgcccgtgggtcgtcccgcgccatcttctccgtgggccccgtgagcccgaatcgcaggtggtcgcacaggtgctatggttatgacttga
cacattccttcctcttctacttgtgggtgttacggacttgagggtcggggtacgggcacccagcagggcgcggtagaagaggcacccggggcactcgggcttagcgtccaccagcgtgtccacgataccaatactgaact
3780
160
165
170
175
180
185
190
195
200
Cys Lys Glu Gly Glu Asp Glu His Pro Gln Cys Leu Asn Ser Gln Pro His Ala Arg Gly Ser Ser Arg Ala Ile Phe Ser Val Gly Pro Val Ser Pro Asn Arg Arg Trp Ser His Arg Cys Tyr Gly Tyr Asp Leu
LILRB2
Target region
SM682.LIR2.g18
actctccctatgtgtggtcttcacccagtgatctcctggagctcctggtcccaggtgagaaattcacagcattgtctggagttccctgagtctccctgagtctccaggcaggtggggagcagccgtgtctcagggcagtt
tgagagggatacacaccagaagtgggtcactagaggacctcgaggaccagggtccactctttaagtgtcgtaacagacctcaagggactcagagggactcagaggtccgtccacccctcgtcggcacagagtcccgtcaa
3920
205
210
215
Asn Ser Pro Tyr Val Trp Ser Ser Pro Ser Asp Leu Leu Glu Leu Leu Val Pro
LILRB2
Target region
acctcaagggactcagaggg
SM682.Cel.R2

FIG. 1

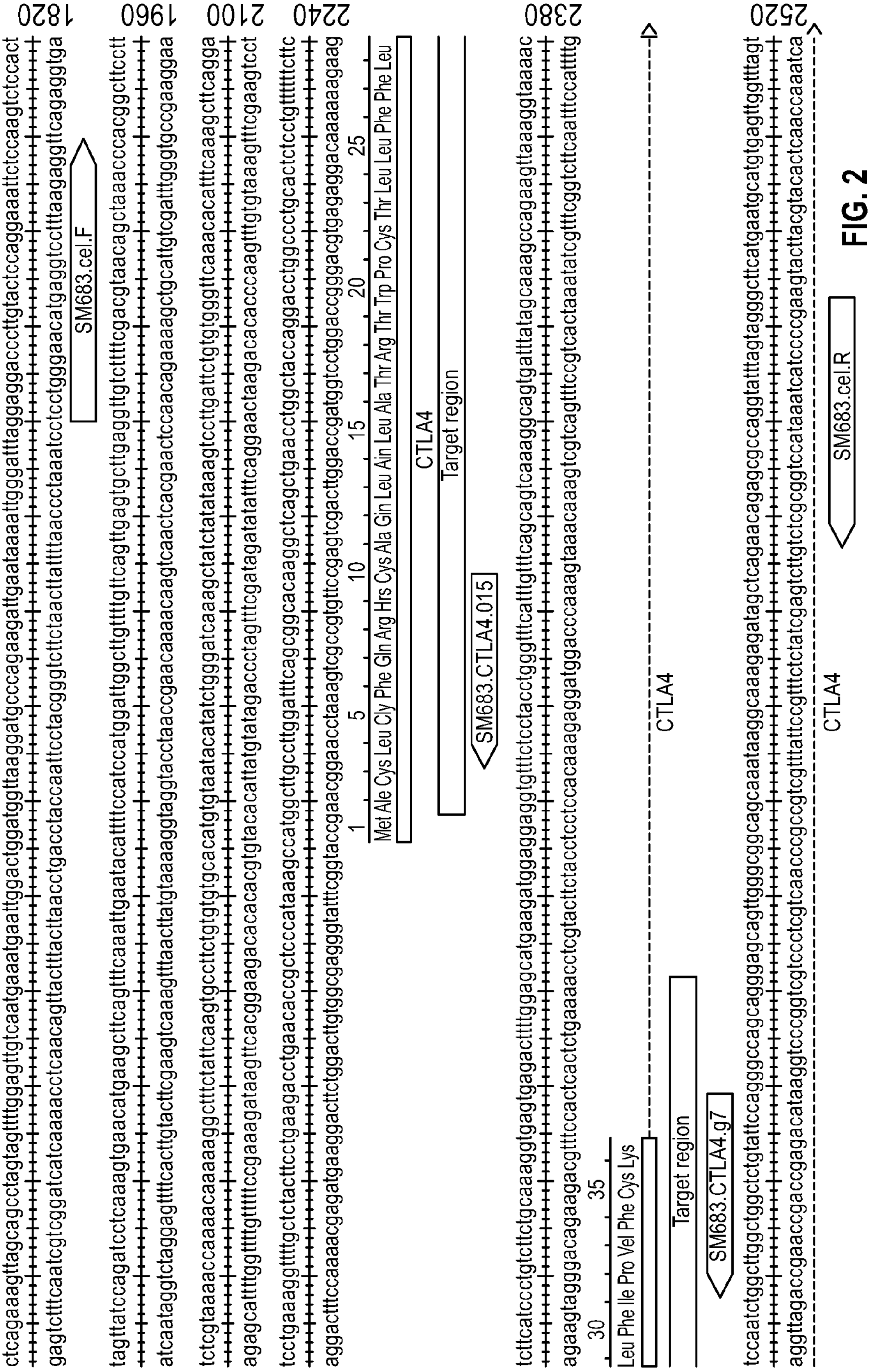
1820
1960
2100
2240
2380
2520
SM683.cel.F
Met Ale Cys Leu Cly Phe Gln Arg Hrs Cys Ala Gln Leu Ain Leu Ala Thr Arg Thr Trp Pro Cys Thr Leu Leu Phe Phe Leu
CTLA4
Target region
SM683.CTLA4.015
Leu Phe Ile Pro Vel Phe Cys Lys
Target region
SM683.CTLA4.g7
CTLA4
SM683.cel.R
CTLA4

FIG. 2

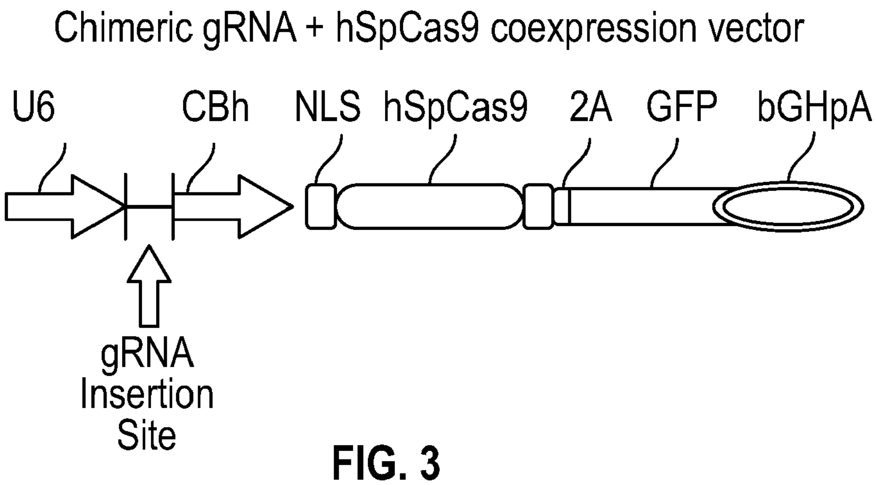
Chimeric gRNA + hSpCas9 coexpression vector
U6
CBh
NLS
hSpCas9
2A
GFP
bGHpA
gRNA Insertion Site

FIG. 3

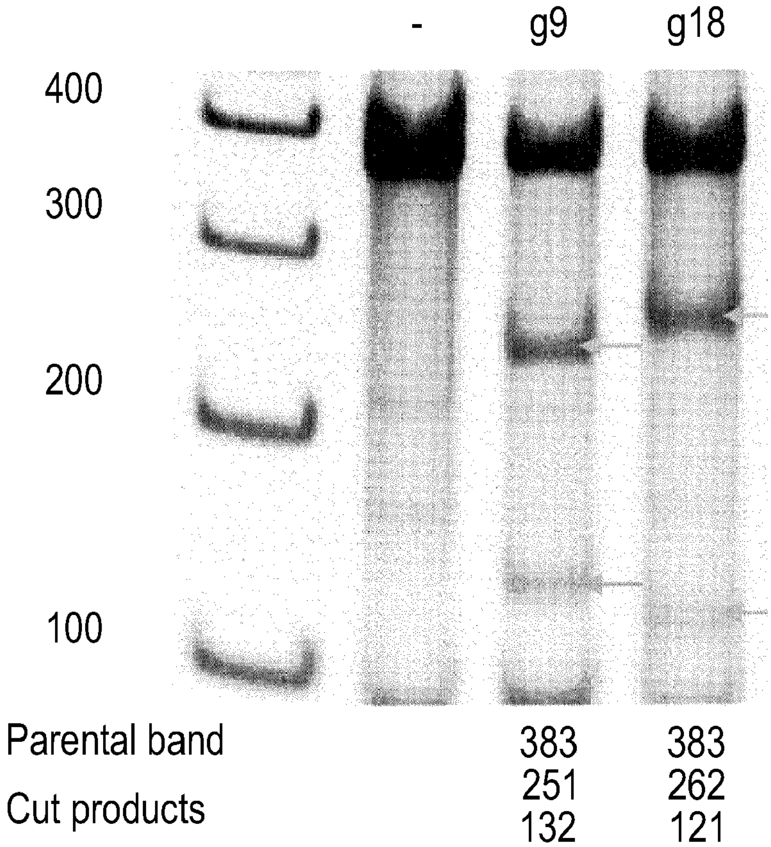
-
g9
g18
400
300
200
100
Parental band
Cut products
383
251
132
383
262
121

FIG. 4

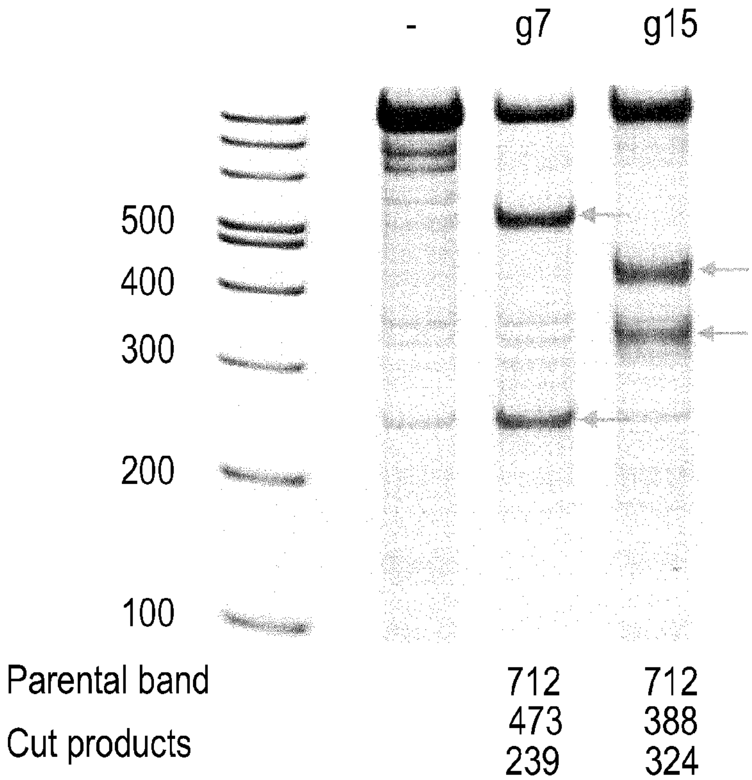
-
g7
g15
500
400
300
200
100
Parental band
712
712
Cut products
473
239
388
324

FIG. 5

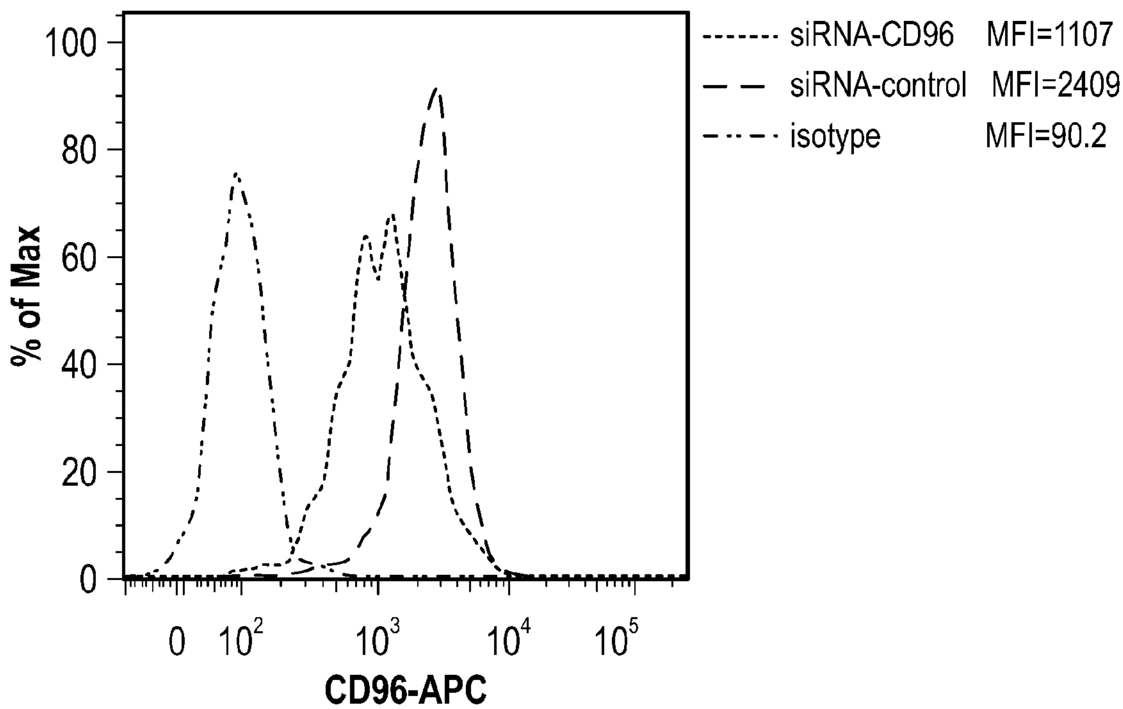
siRNA-CD96 MFI=1107
siRNA-control MFI=2409
isotype MFI=90.2
100
80
60
40
20
0
% of Max
0
$10^2$
$10^3$
$10^4$
$10^5$
CD96-APC

FIG. 6A

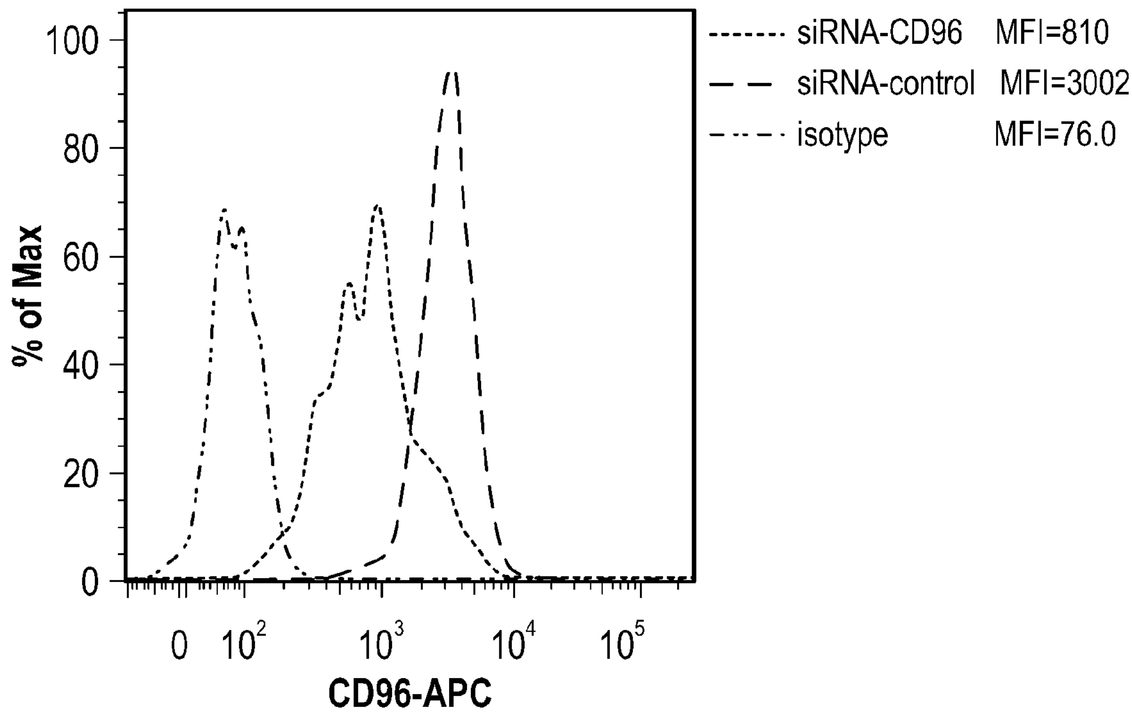

siRNA-CD96 MFI=810
siRNA-control MFI=3002
isotype MFI=76.0
100
80
60
40
20
0
% of Max
0
$10^2$
$10^3$
$10^4$
$10^5$
CD96-APC


FIG. 6B

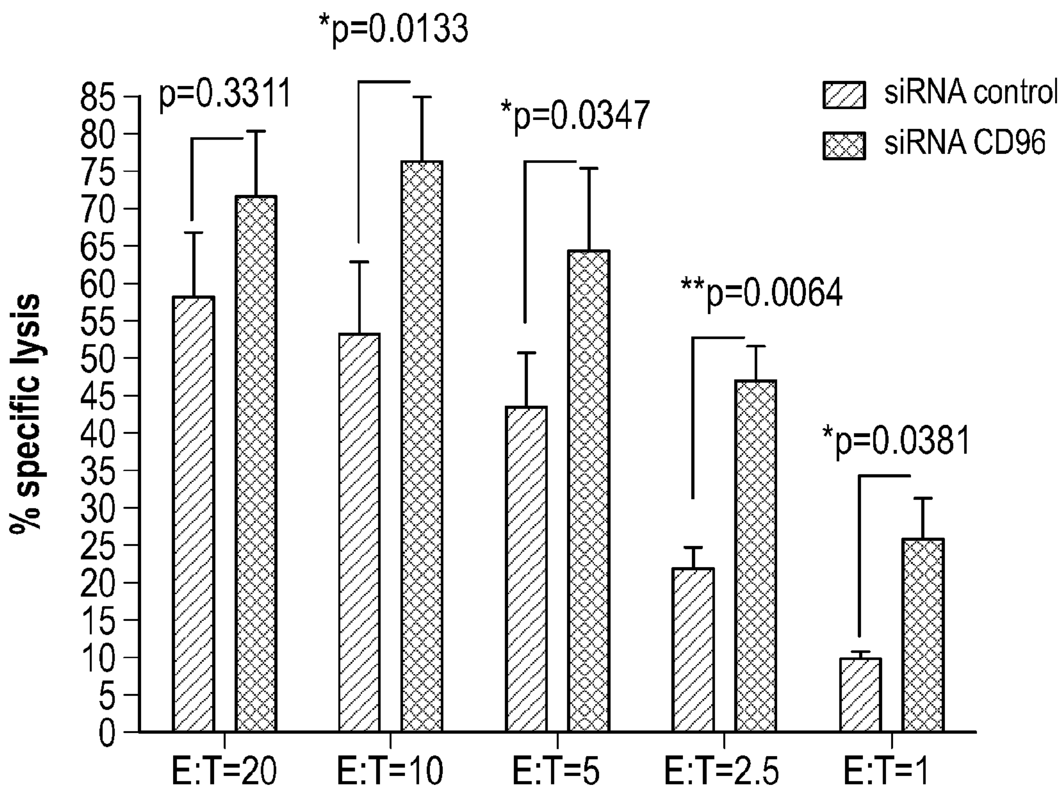

*p=0.0133
p=0.3311
*p=0.0347
**p=0.0064
*p=0.0381
siRNA control
siRNA CD96
85
80
75
70
65
60
55
50
45
40
35
30
25
20
15
10
5
0
% specific lysis
E:T=20
E:T=10
E:T=5
E:T=2.5
E:T=1


FIG. 7

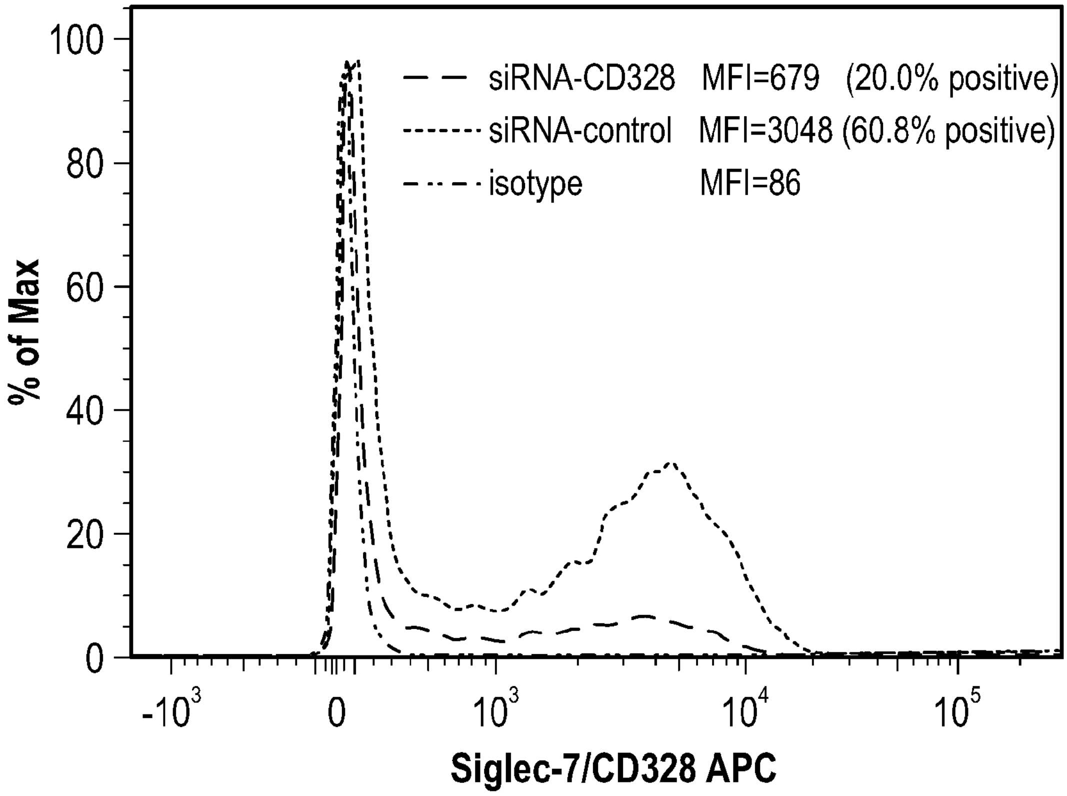
100
80
60
40
20
0
% of Max
siRNA-CD328 MFI=679 (20.0% positive)
siRNA-control MFI=3048 (60.8% positive)
isotype MFI=86
$-10^3$
0
$10^3$
$10^4$
$10^5$
Siglec-7/CD328 APC

FIG. 8

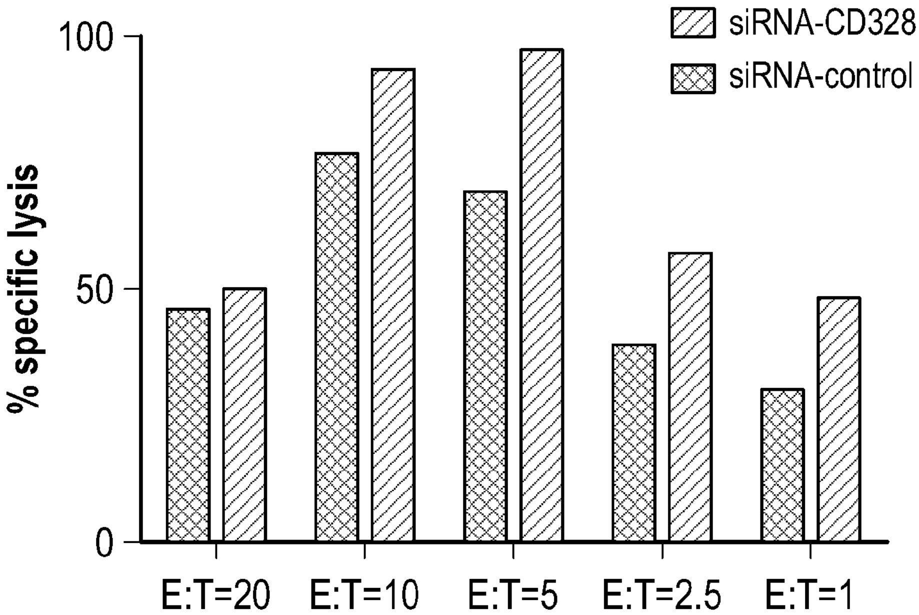
siRNA-CD328
siRNA-control
100
50
0
% specific lysis
E:T=20
E:T=10
E:T=5
E:T=2.5
E:T=1

FIG. 9

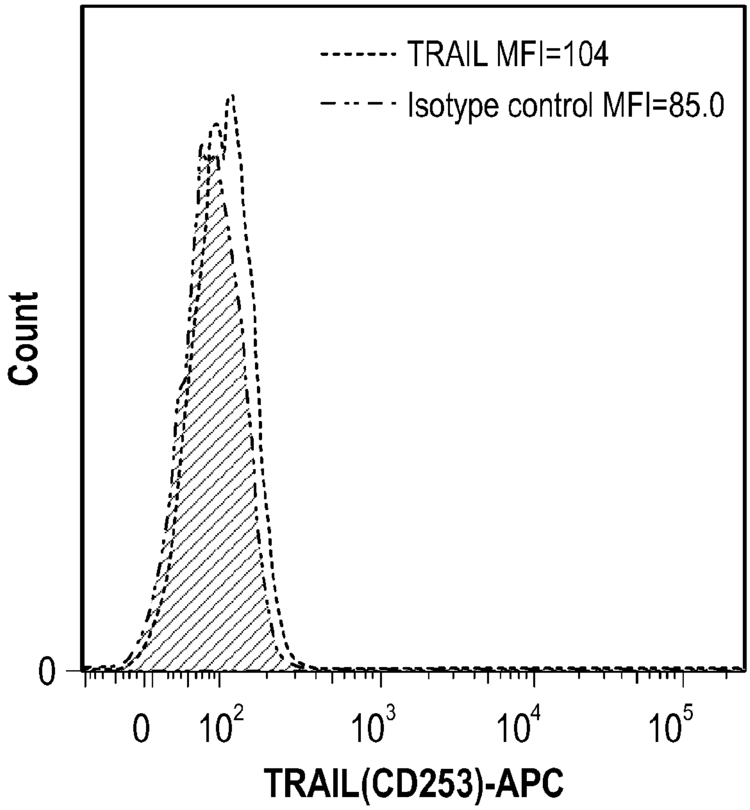
TRAIL MFI=104
Isotype control MFI=85.0
Count
0
0
10^2
10^3
10^4
10^5
TRAIL(CD253)-APC

FIG. 10

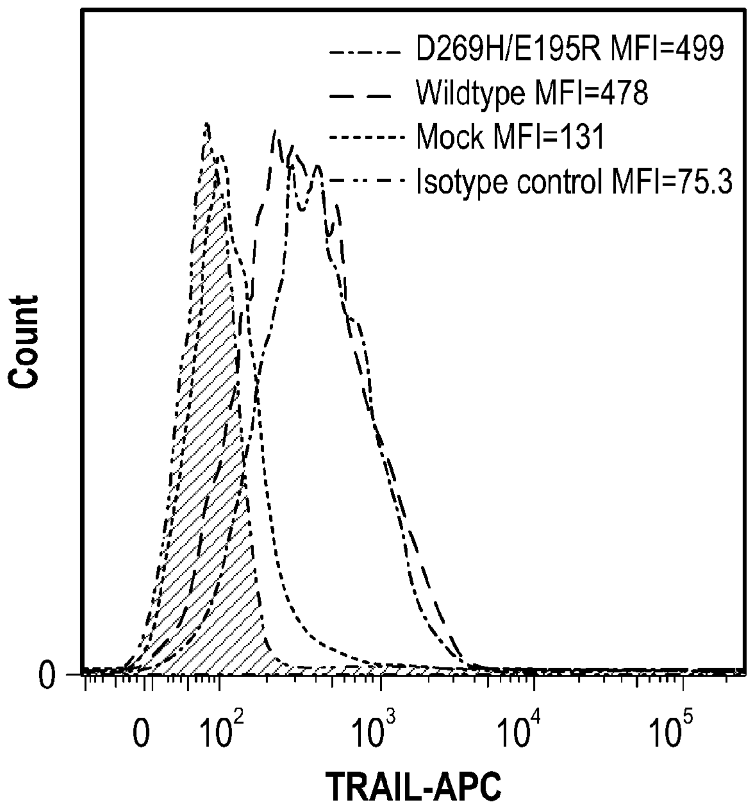
D269H/E195R MFI=499
Wildtype MFI=478
Mock MFI=131
Isotype control MFI=75.3
Count
0
0
10^2
10^3
10^4
10^5
TRAIL-APC

FIG. 11

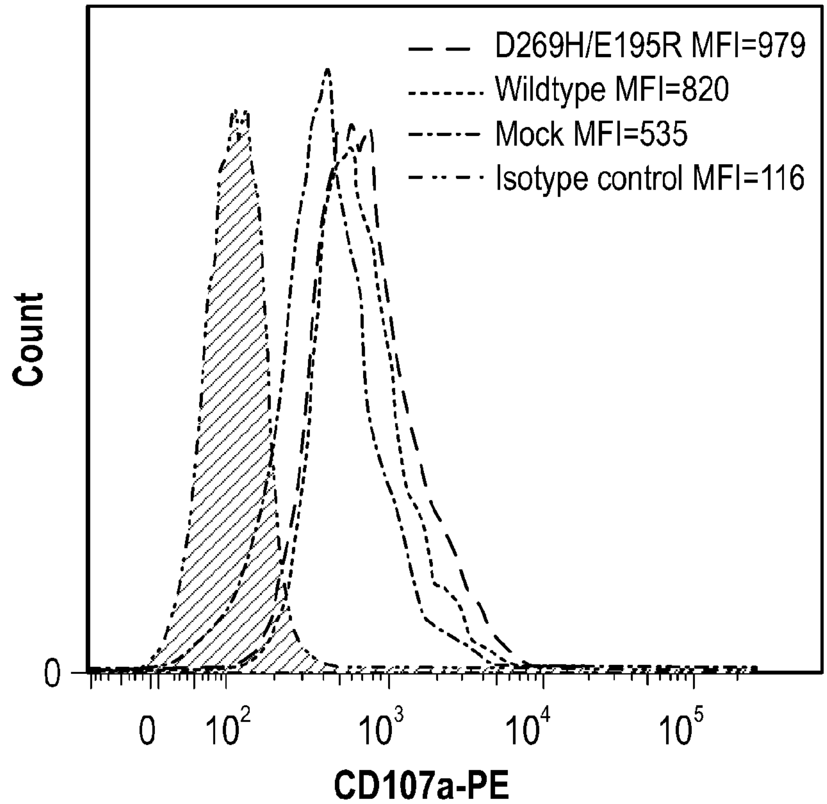
D269H/E195R MFI=979
Wildtype MFI=820
Mock MFI=535
Isotype control MFI=116
Count
0
0
$10^2$
$10^3$
$10^4$
$10^5$
CD107a-PE

FIG. 12

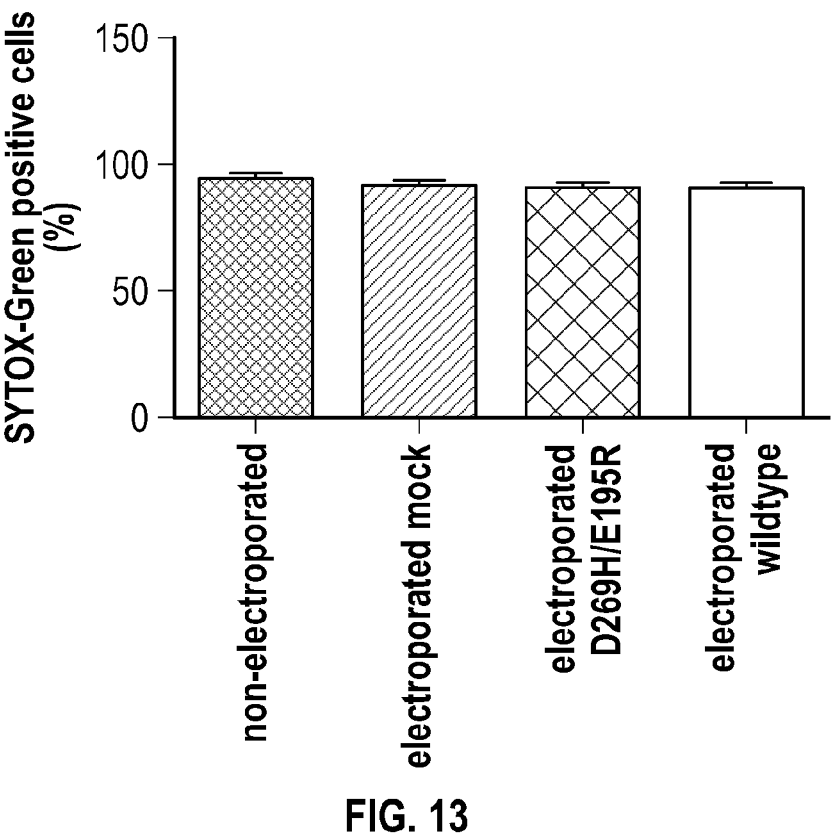
SYTOX-Green positive cells (%)
150
100
50
0
non-electroporated
electroporated mock
electroporated D269H/E195R
electroporated wildtype

FIG. 13

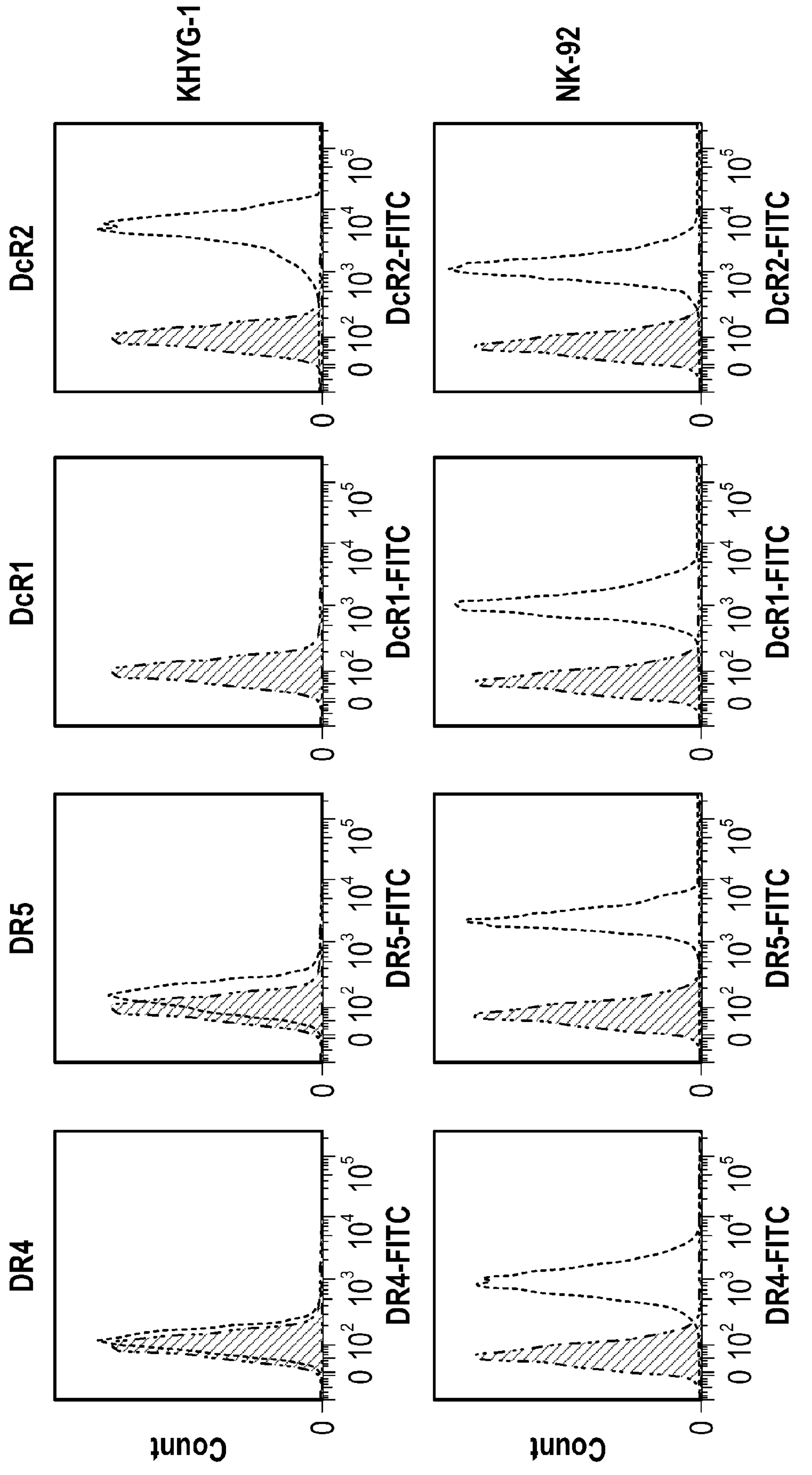

KHYG-1
NK-92
DR4
DR5
DcR1
DcR2
Count
DR4-FITC
DR5-FITC
DcR1-FITC
DcR2-FITC


FIG. 14

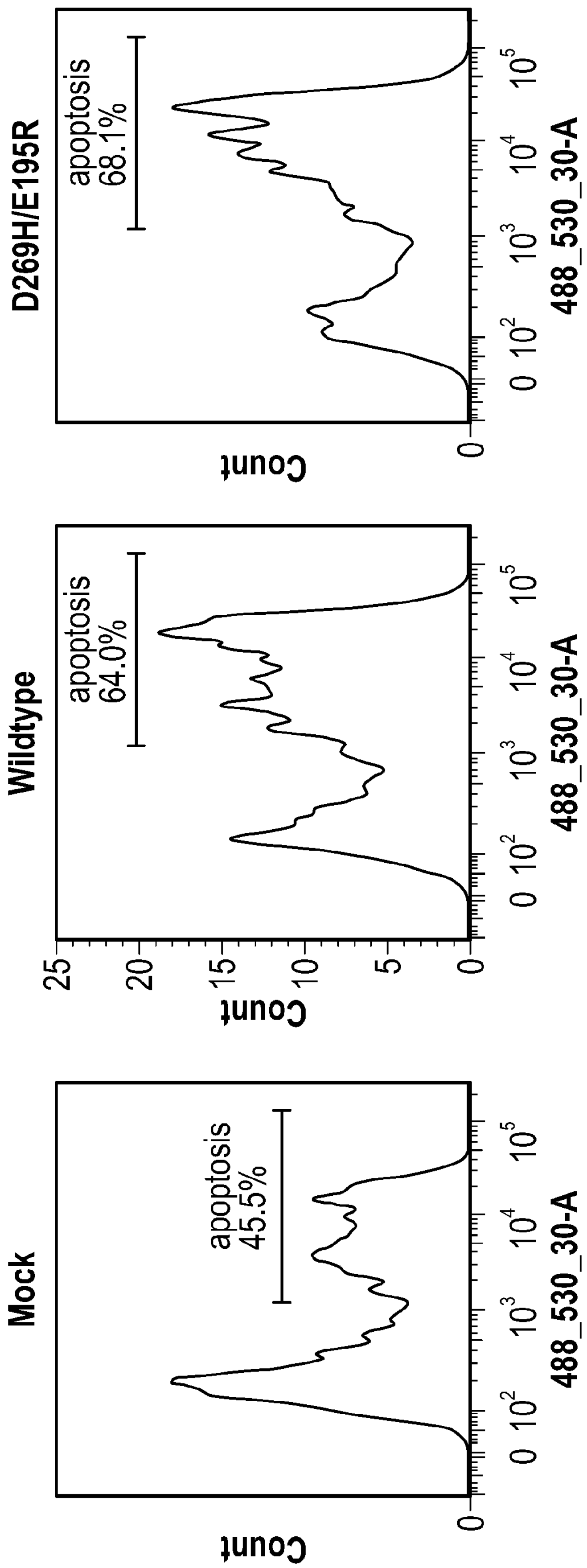

Mock
apoptosis
45.5%
Count
0
0 10^2 10^3 10^4 10^5
488_530_30-A
Wildtype
apoptosis
64.0%
Count
25 20 15 10 5 0
0 10^2 10^3 10^4 10^5
488_530_30-A
D269H/E195R
apoptosis
68.1%
Count
0
0 10^2 10^3 10^4 10^5
488_530_30-A


FIG. 15

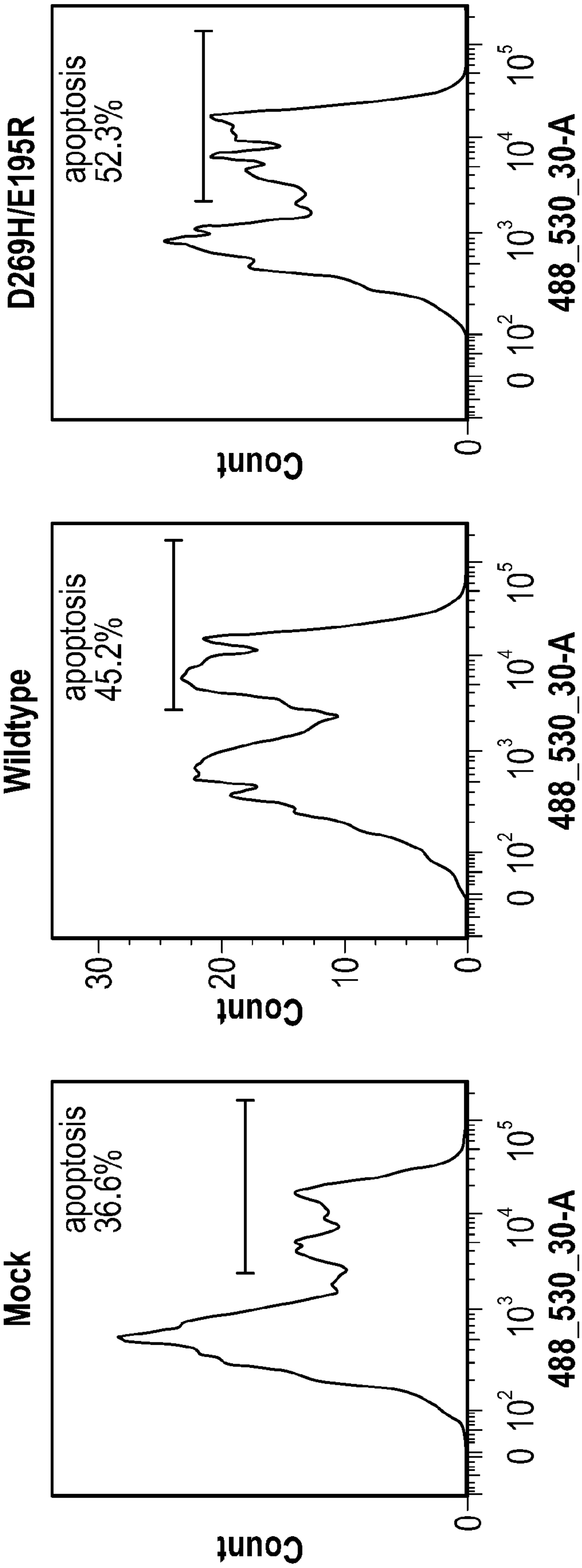
Mock
apoptosis
36.6%
Count
0
0 10^2 10^3 10^4 10^5
488_530_30-A
Wildtype
apoptosis
45.2%
Count
0
10
20
30
0 10^2 10^3 10^4 10^5
488_530_30-A
D269H/E195R
apoptosis
52.3%
Count
0
0 10^2 10^3 10^4 10^5
488_530_30-A

FIG. 16

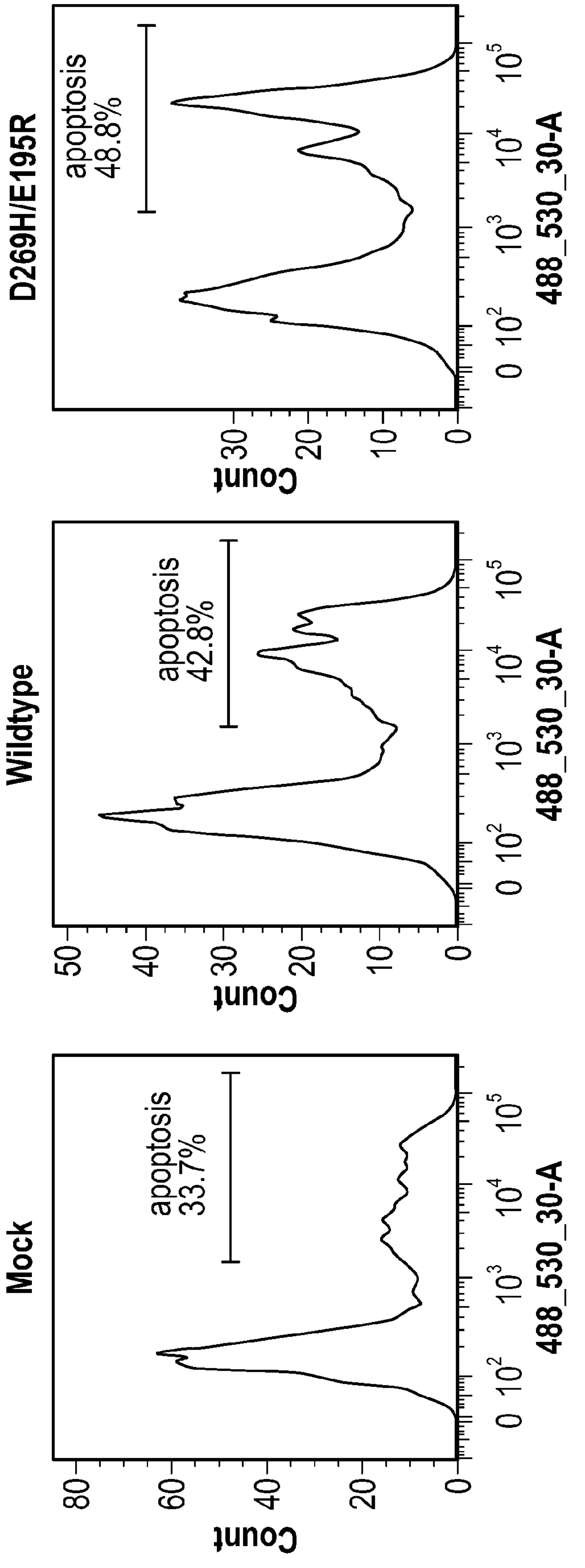
Mock
apoptosis 33.7%
Count
488_530_30-A
Wildtype
apoptosis 42.8%
Count
488_530_30-A
D269H/E195R
apoptosis 48.8%
Count
488_530_30-A

FIG. 17

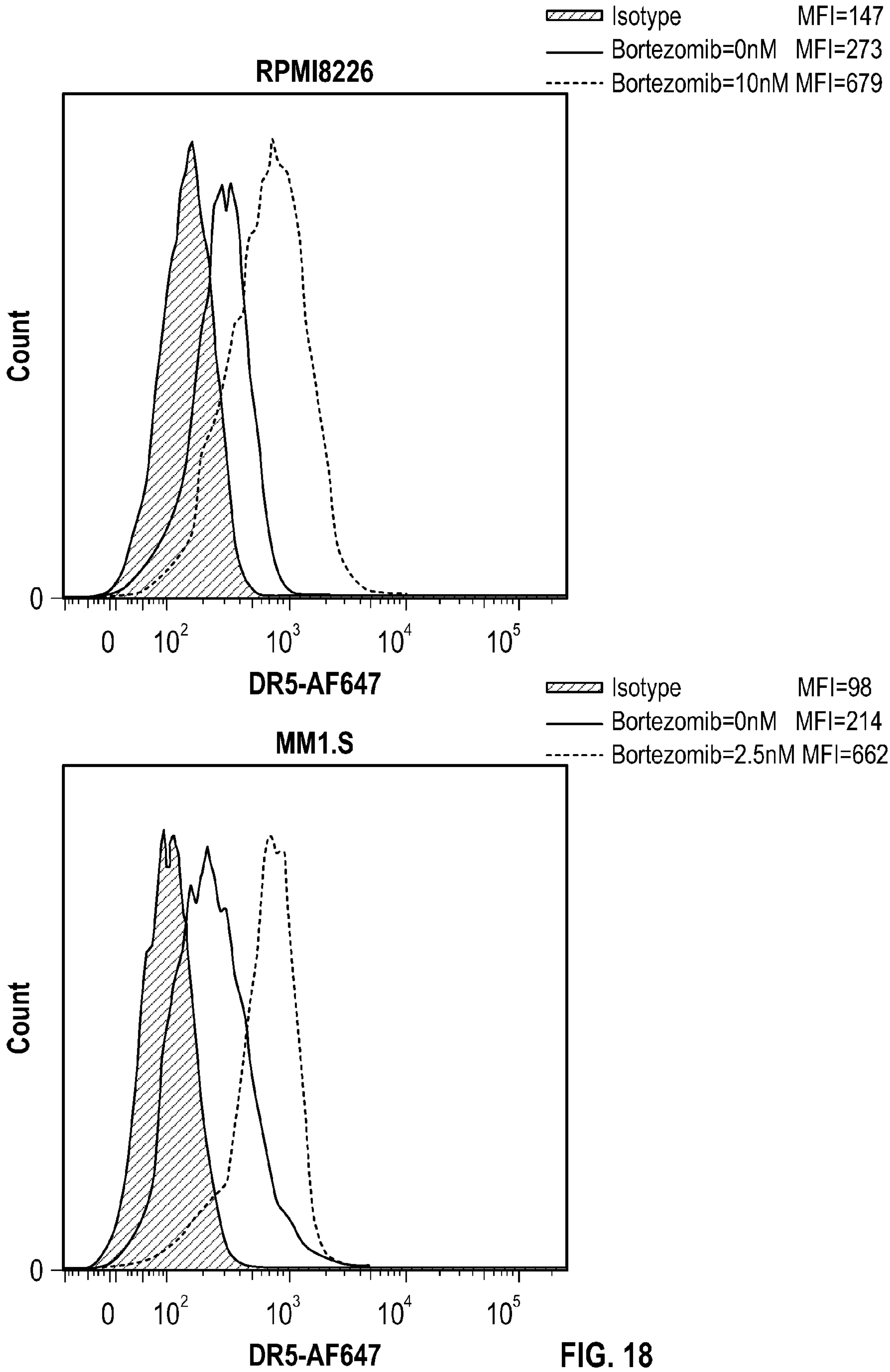
Isotype MFI=147
Bortezomib=0nM MFI=273
Bortezomib=10nM MFI=679
RPMI8226
Count
0
0
$10^2$
$10^3$
$10^4$
$10^5$
DR5-AF647
Isotype MFI=98
Bortezomib=0nM MFI=214
Bortezomib=2.5nM MFI=662
MM1.S
Count
0
0
$10^2$
$10^3$
$10^4$
$10^5$
DR5-AF647

FIG. 18

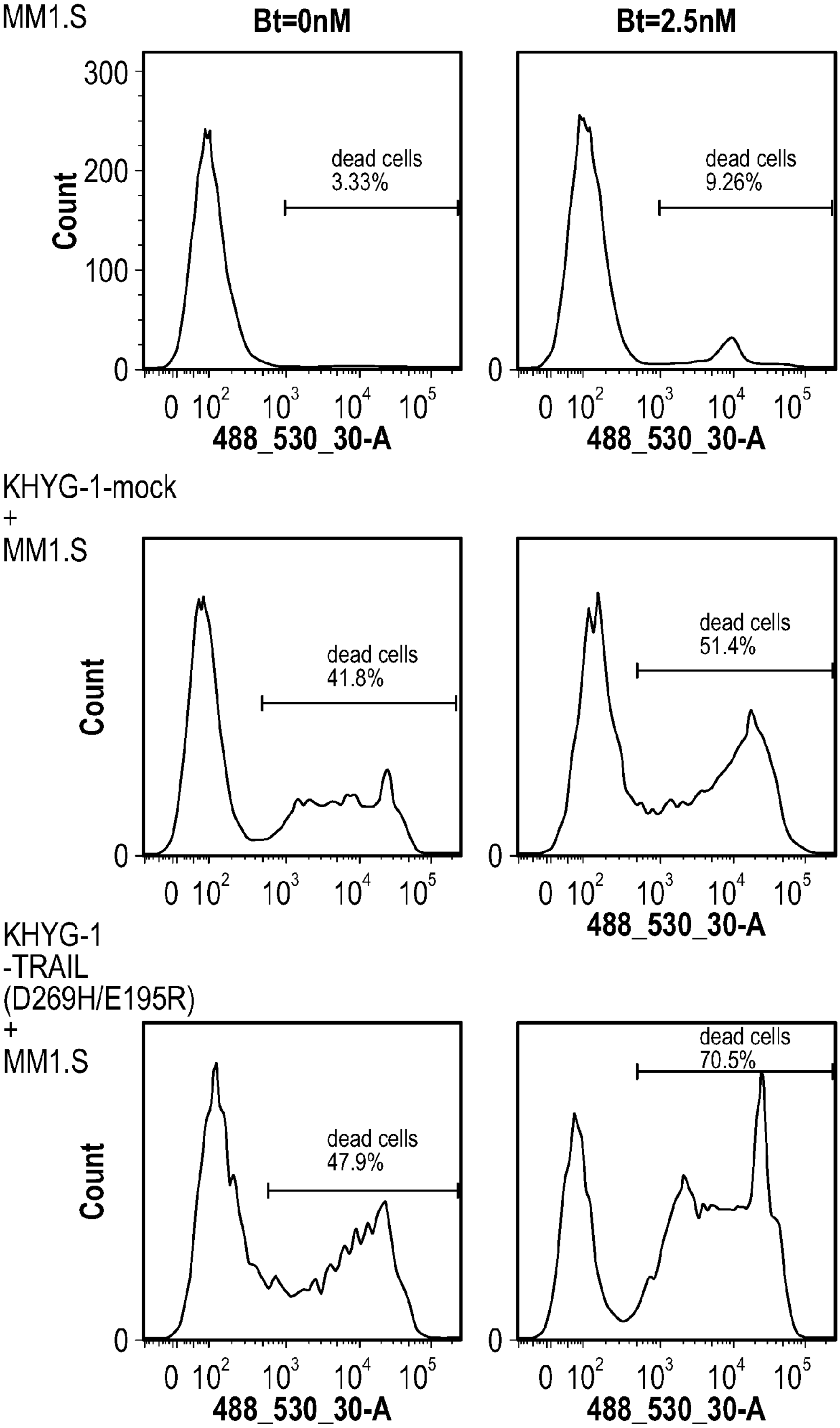
MM1.S
Bt=0nM
Bt=2.5nM
300
200
100
0
Count
dead cells
3.33%
dead cells
9.26%
0 10^2 10^3 10^4 10^5
488_530_30-A
KHYG-1-mock
+
MM1.S
dead cells
41.8%
dead cells
51.4%
KHYG-1
-TRAIL
(D269H/E195R)
+
MM1.S
dead cells
47.9%
dead cells
70.5%

FIG. 19

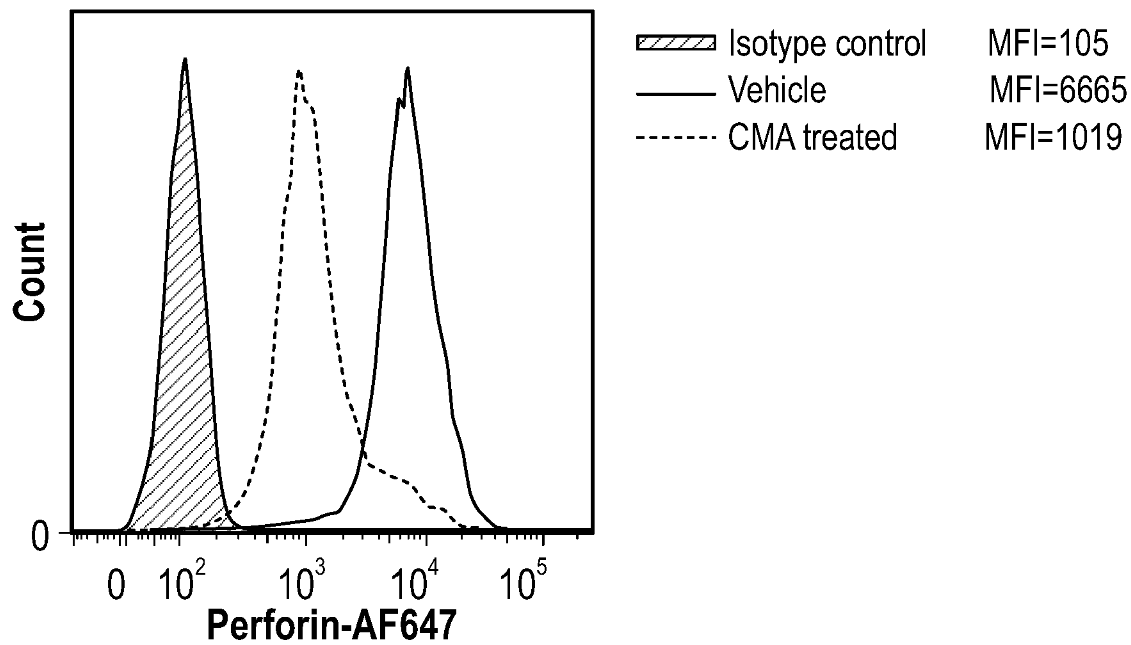
Isotype control MFI=105
Vehicle MFI=6665
CMA treated MFI=1019
Count
0
0 $10^2$ $10^3$ $10^4$ $10^5$
Perforin-AF647

FIG. 20

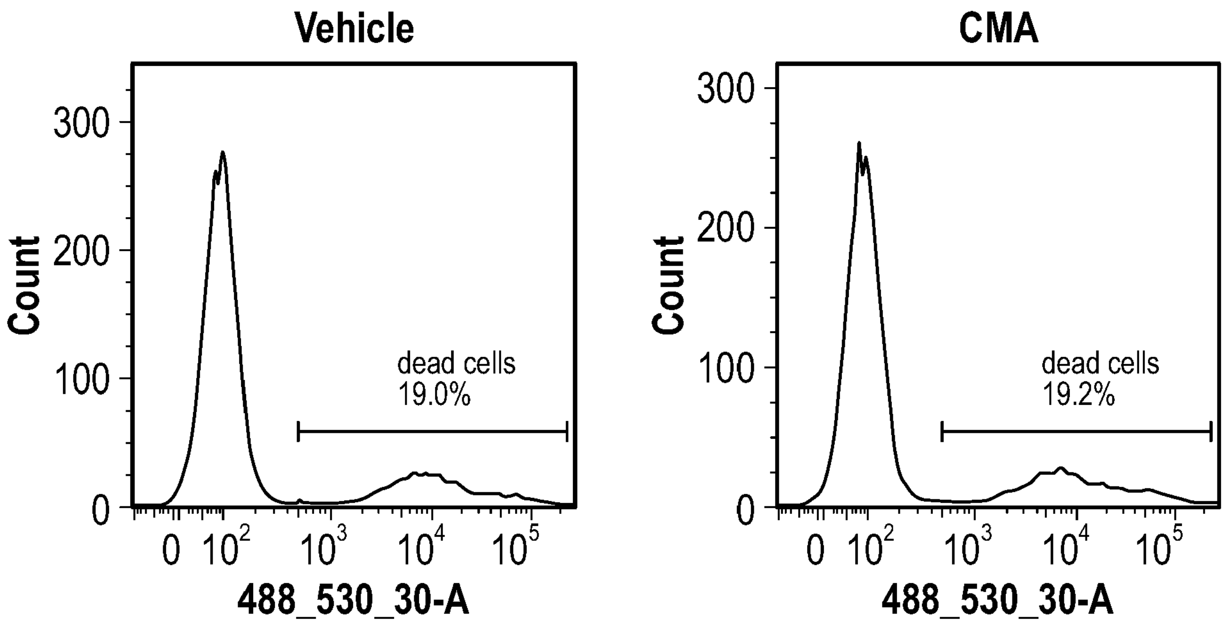
Vehicle
CMA
300
200
100
0
Count
dead cells
19.0%
dead cells
19.2%
0 $10^2$ $10^3$ $10^4$ $10^5$
488_530_30-A
488_530_30-A

FIG. 21

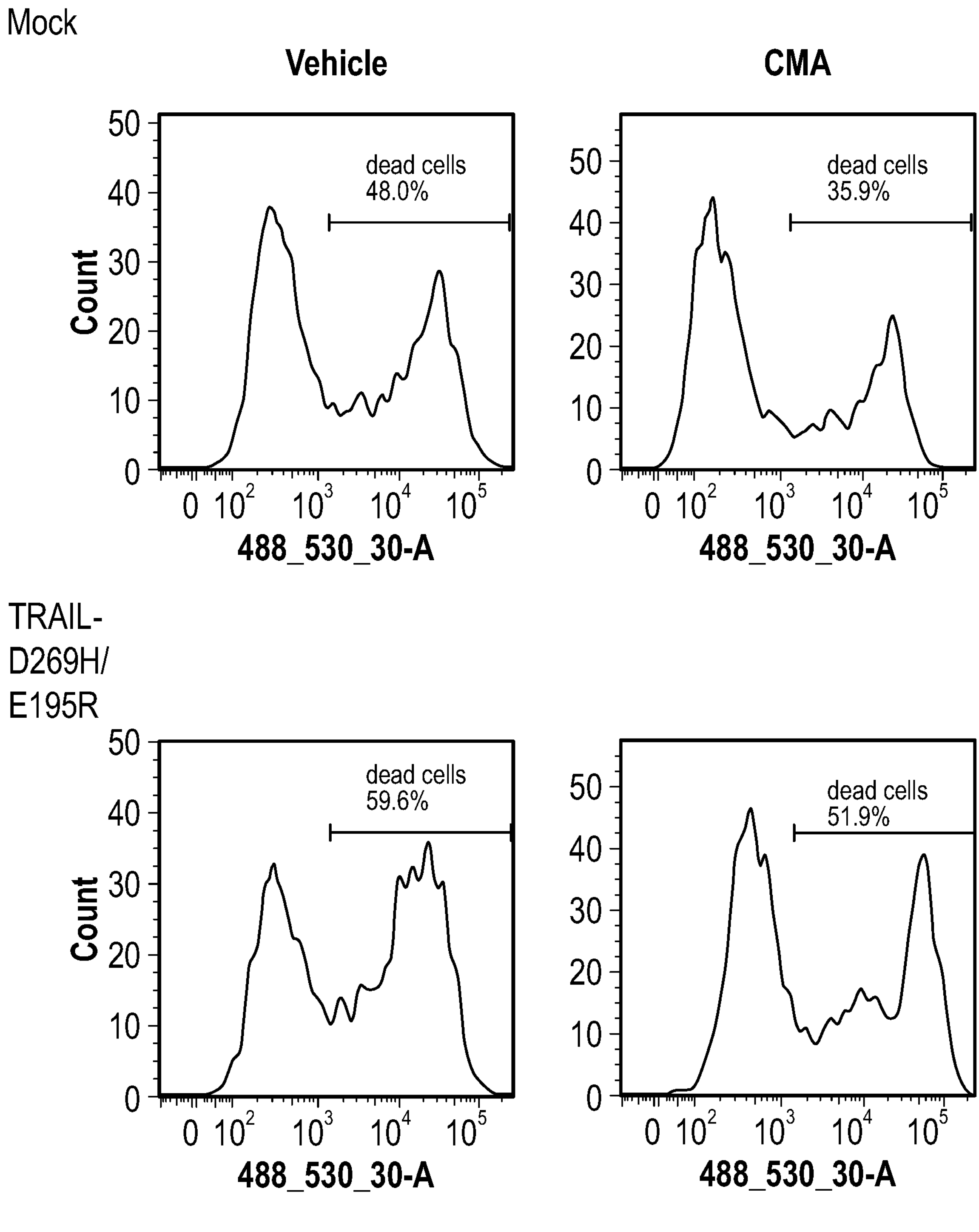
Mock
Vehicle
CMA
dead cells
48.0%
dead cells
35.9%
Count
488_530_30-A
488_530_30-A
TRAIL-
D269H/
E195R
dead cells
59.6%
dead cells
51.9%
Count
488_530_30-A
488_530_30-A

FIG. 22

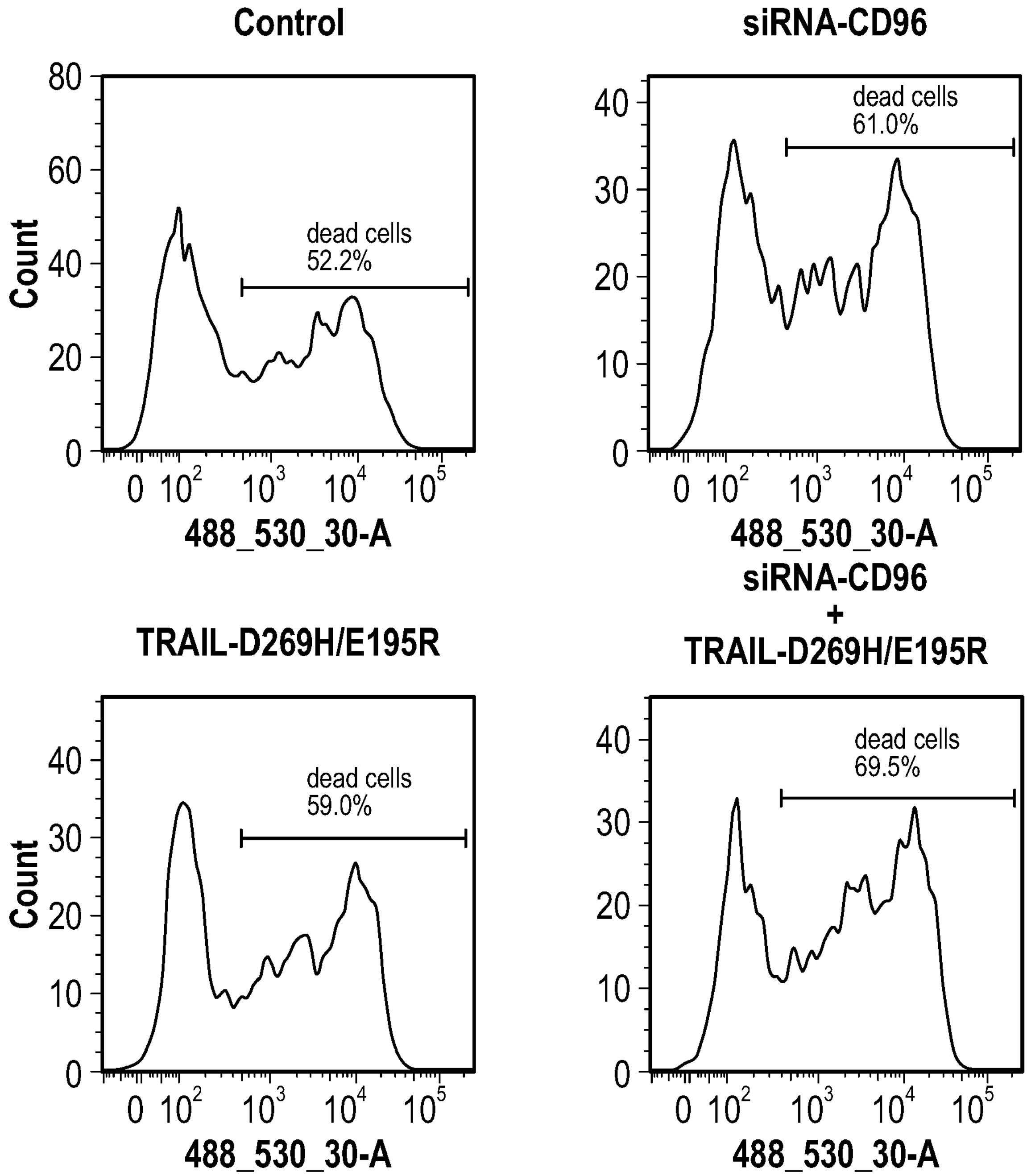
Control
80
60
40
20
0
Count
dead cells
52.2%
0 $10^2$ $10^3$ $10^4$ $10^5$
488_530_30-A
siRNA-CD96
40
30
20
10
0
dead cells
61.0%
0 $10^2$ $10^3$ $10^4$ $10^5$
488_530_30-A
TRAIL-D269H/E195R
40
30
20
10
0
Count
dead cells
59.0%
0 $10^2$ $10^3$ $10^4$ $10^5$
488_530_30-A
siRNA-CD96
+
TRAIL-D269H/E195R
40
30
20
10
0
dead cells
69.5%
0 $10^2$ $10^3$ $10^4$ $10^5$
488_530_30-A

FIG. 23

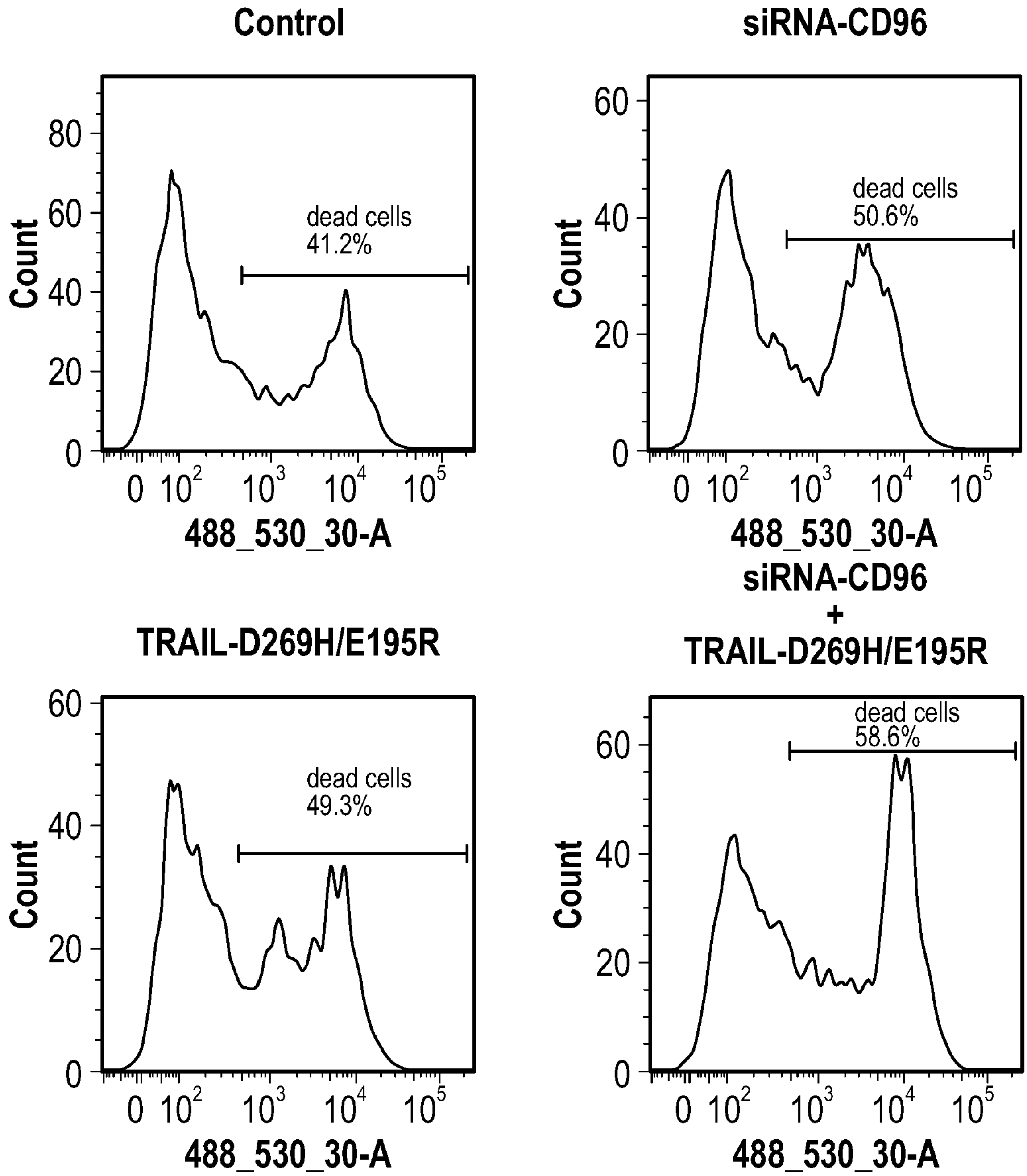
Control
dead cells
41.2%
Count
488_530_30-A
siRNA-CD96
dead cells
50.6%
Count
488_530_30-A
TRAIL-D269H/E195R
dead cells
49.3%
Count
488_530_30-A
siRNA-CD96
+
TRAIL-D269H/E195R
dead cells
58.6%
Count
488_530_30-A

FIG. 24

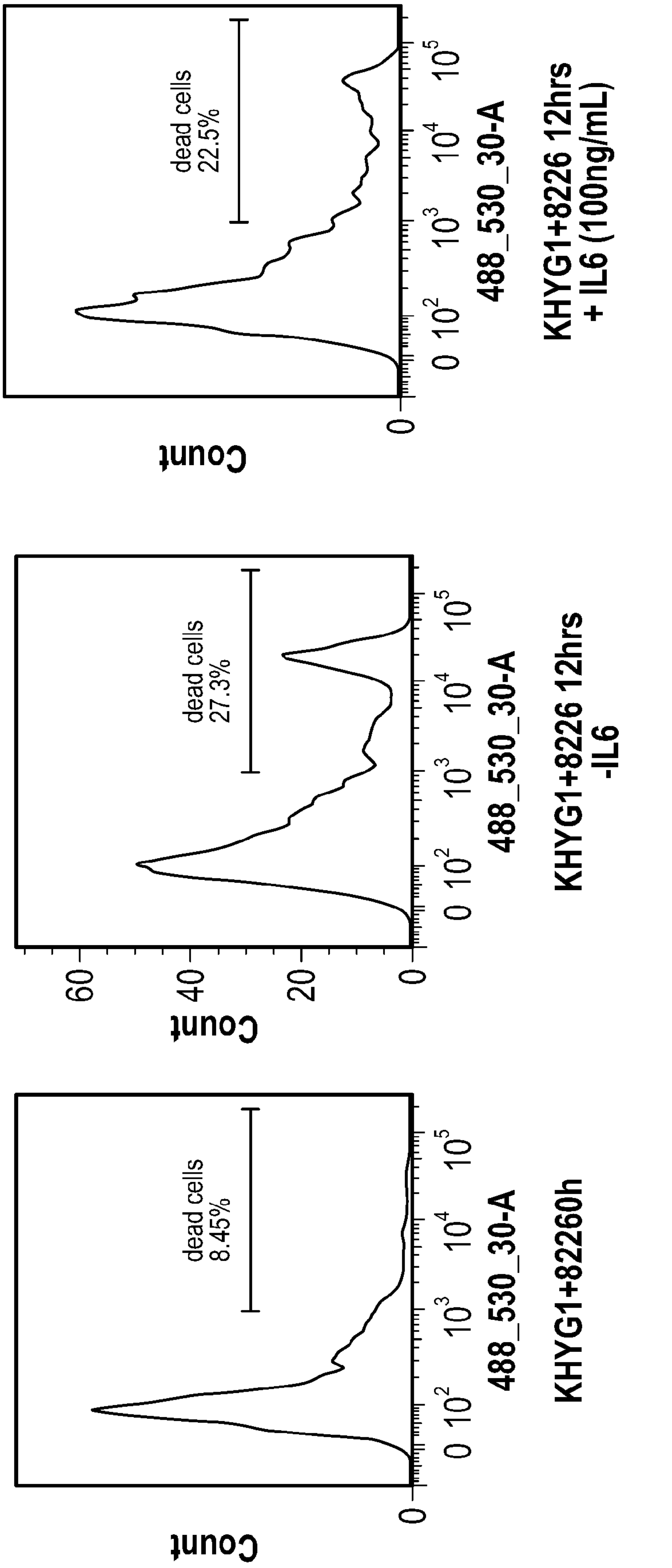
Count
0
0 10^2 10^3 10^4 10^5
dead cells
8.45%
488_530_30-A
KHYG1+82260h
Count
60
40
20
0
0 10^2 10^3 10^4 10^5
dead cells
27.3%
488_530_30-A
KHYG1+8226 12hrs
-IL6
Count
0
0 10^2 10^3 10^4 10^5
dead cells
22.5%
488_530_30-A
KHYG1+8226 12hrs
+ IL6 (100ng/mL)

FIG. 25

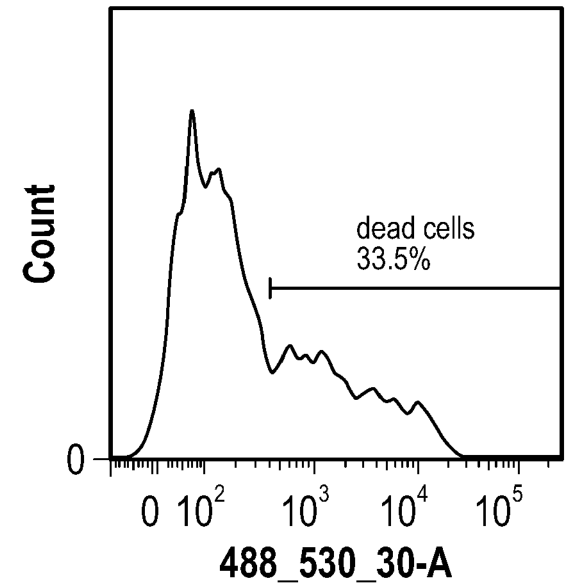


KHYG1+8226 12hrs
-IL6

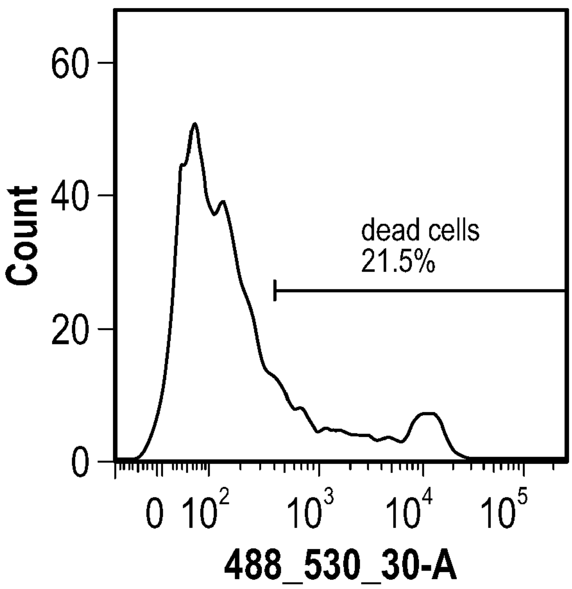


KHYG1+8226 12hrs
+ IL6 (100ng/mL)

FIG. 26

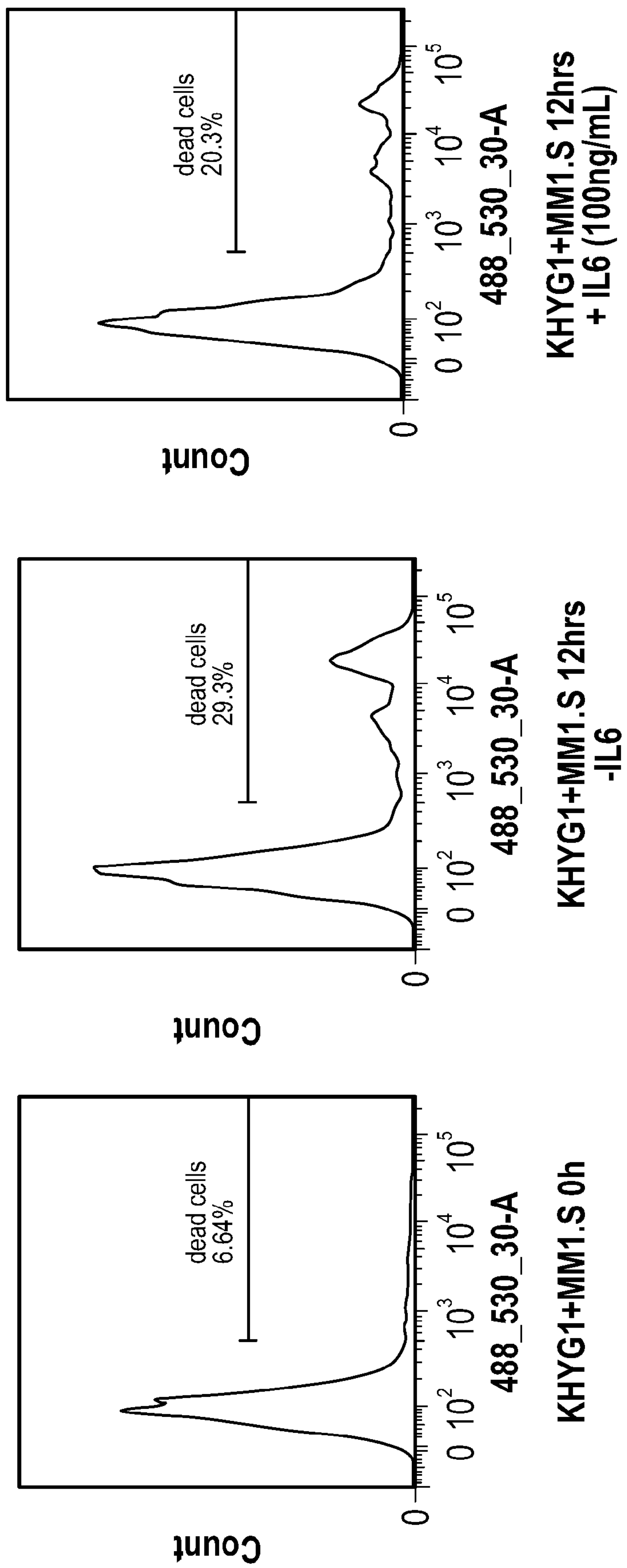
dead cells
6.64%
Count
0
0 10^2 10^3 10^4 10^5
488_530_30-A
KHYG1+MM1.S 0h
dead cells
29.3%
Count
0
0 10^2 10^3 10^4 10^5
488_530_30-A
KHYG1+MM1.S 12hrs
-IL6
dead cells
20.3%
Count
0
0 10^2 10^3 10^4 10^5
488_530_30-A
KHYG1+MM1.S 12hrs
+ IL6 (100ng/mL)

FIG. 27

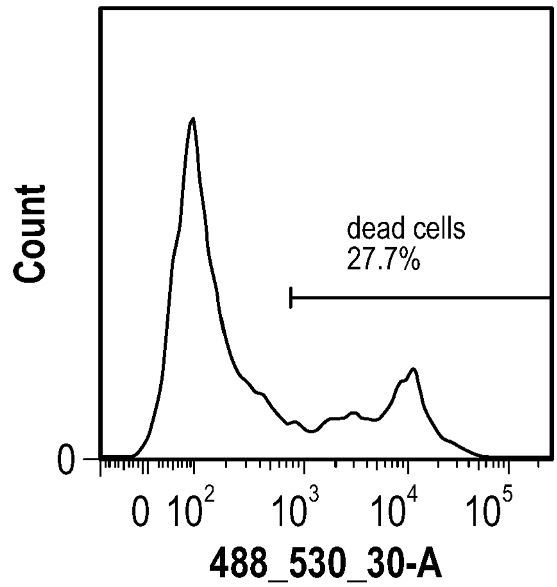


KHYG1+MM1.S 12hrs
-IL6

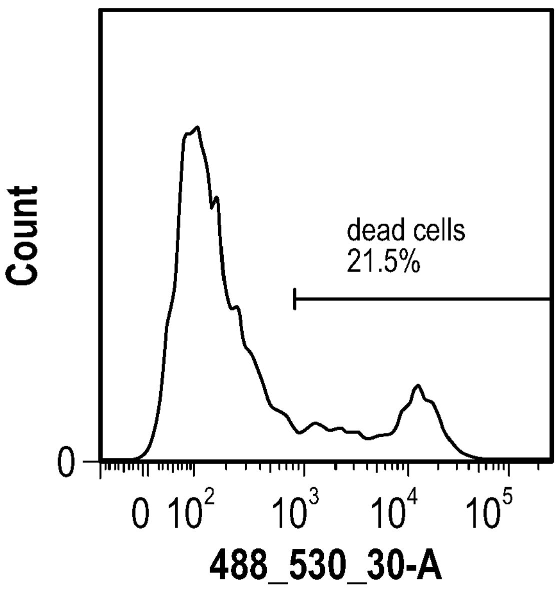


KHYG1+MM1.S 12hrs
+ IL6 (100ng/mL)

FIG. 28

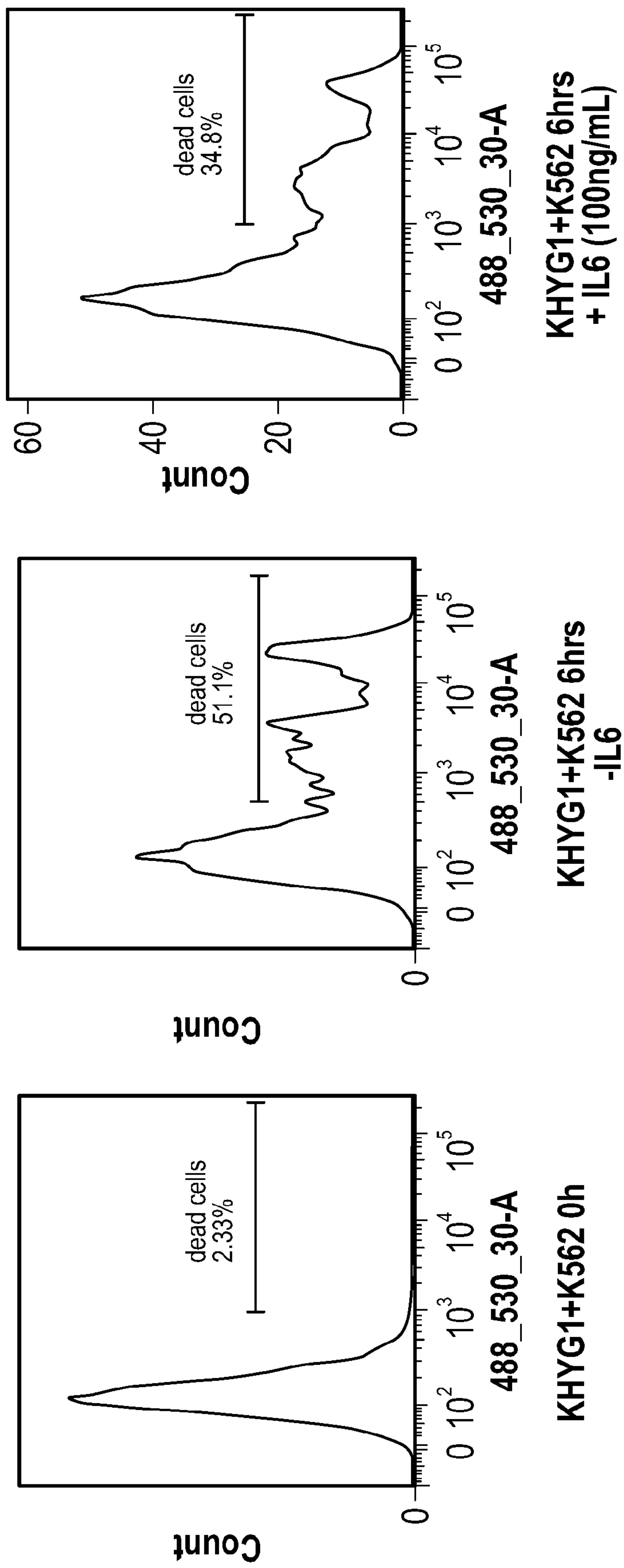
Count
0
dead cells
2.33%
0 10^2 10^3 10^4 10^5
488_530_30-A
KHYG1+K562 0h
Count
0
dead cells
51.1%
0 10^2 10^3 10^4 10^5
488_530_30-A
KHYG1+K562 6hrs
-IL6
Count
0
20
40
60
dead cells
34.8%
0 10^2 10^3 10^4 10^5
488_530_30-A
KHYG1+K562 6hrs
+ IL6 (100ng/mL)

FIG. 29

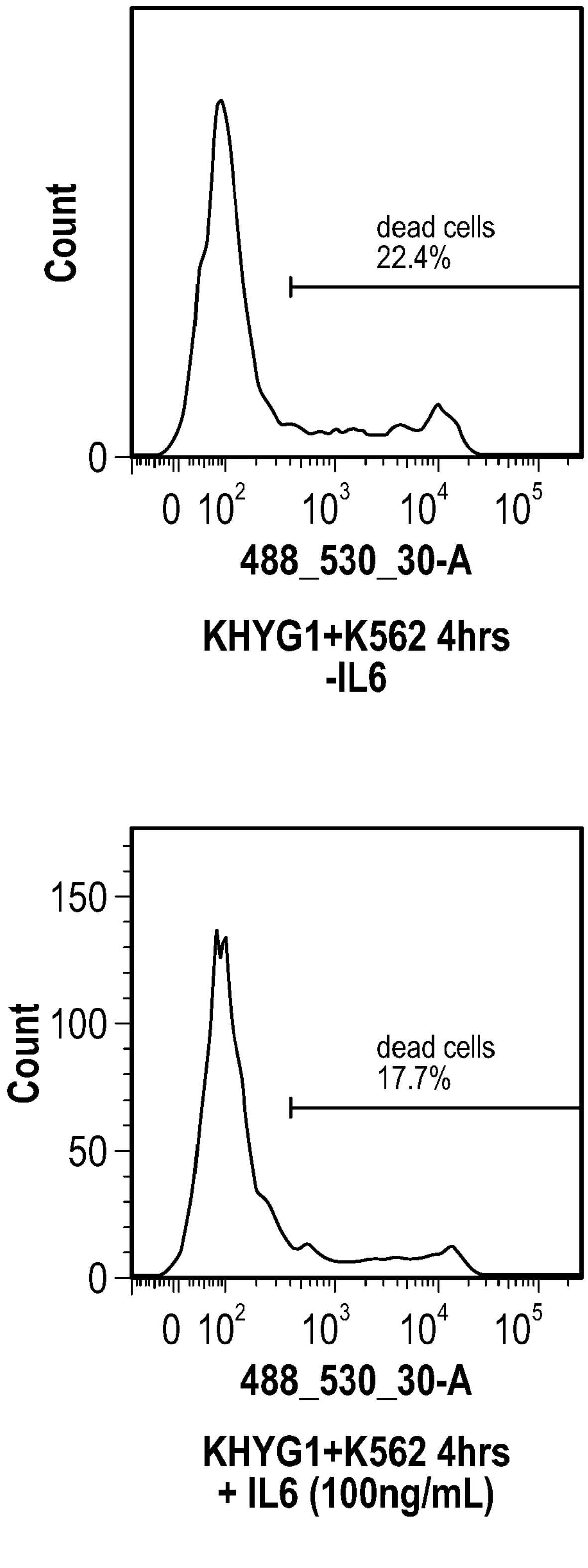
Count
dead cells
22.4%
0
0 10^2 10^3 10^4 10^5
488_530_30-A
KHYG1+K562 4hrs
-IL6
Count
150
100
50
0
dead cells
17.7%
0 10^2 10^3 10^4 10^5
488_530_30-A
KHYG1+K562 4hrs
+ IL6 (100ng/mL)

FIG. 30

| | Sample Name | Median, 488_585_42-A |
|---|---|---|
| ---------- | 20160706 KHYG1_CD126-PE_003.fcs | 148 |
| ———— | 20160706 KHYG1_BLANK_001.fcs | 113 |

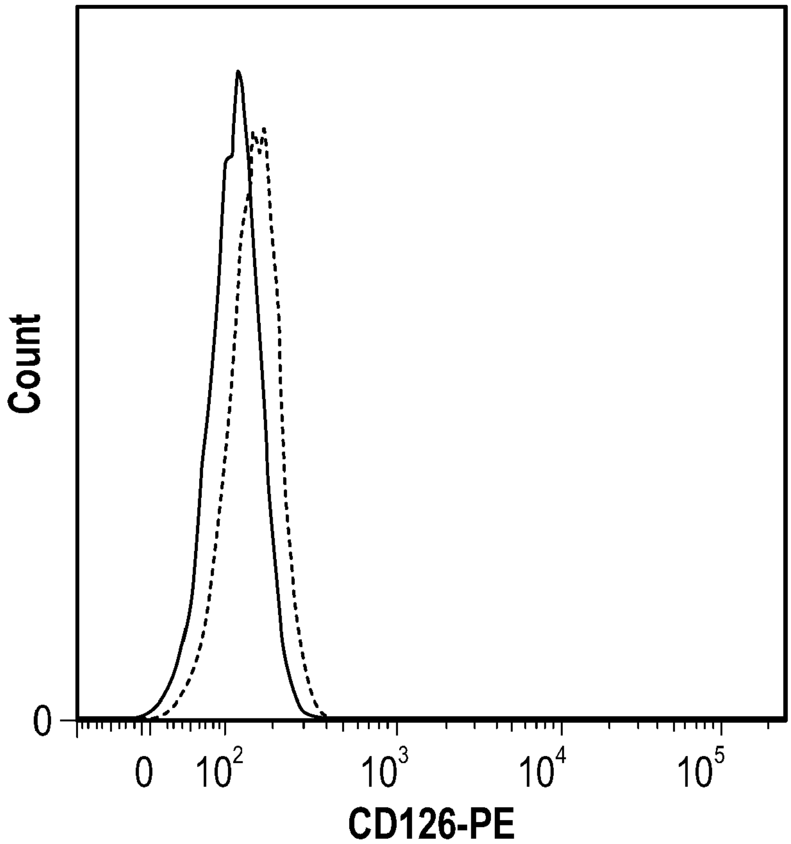


FIG. 31

| | Sample Name | Median, 488_585_42-A |
|---|---|---|
| ---------- | 20160711 KHYG1_CD126-PE_003.fcs | 167 |
| ———— | 20160711 KHYG1_BLANK_001.fcs | 127 |

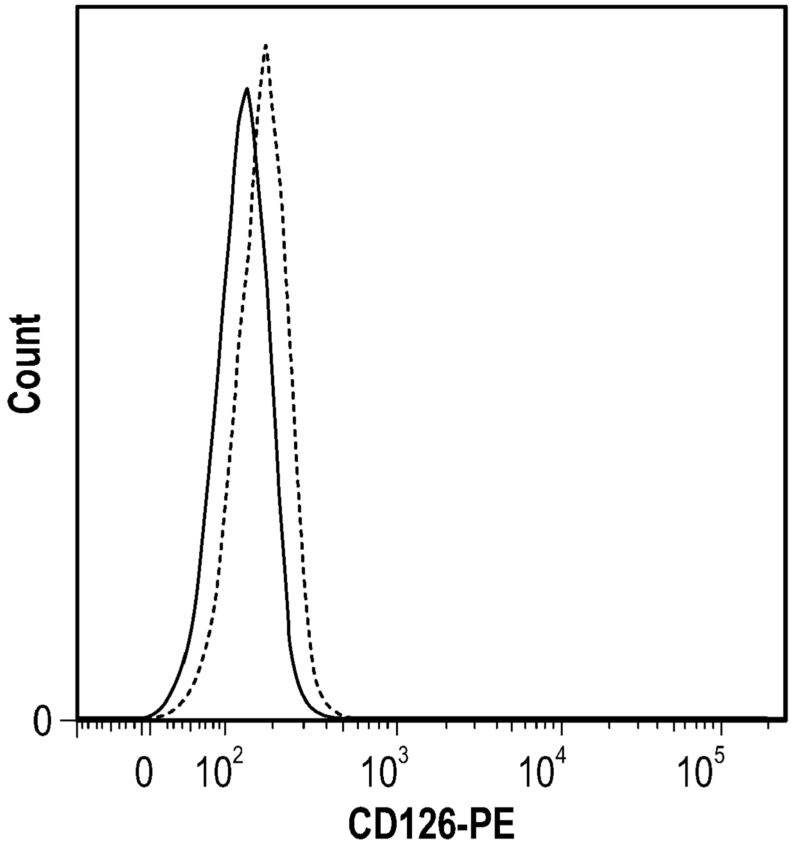


FIG. 32

| | Sample Name | Median, 488_585_42-A |
|---|---|---|
| ---------- | 20160706 KHYG1_CD130-PE_001_006.fcs | 164 |
| ———— | 20160706 KHYG1_BLANK_001_007.fcs | 121 |

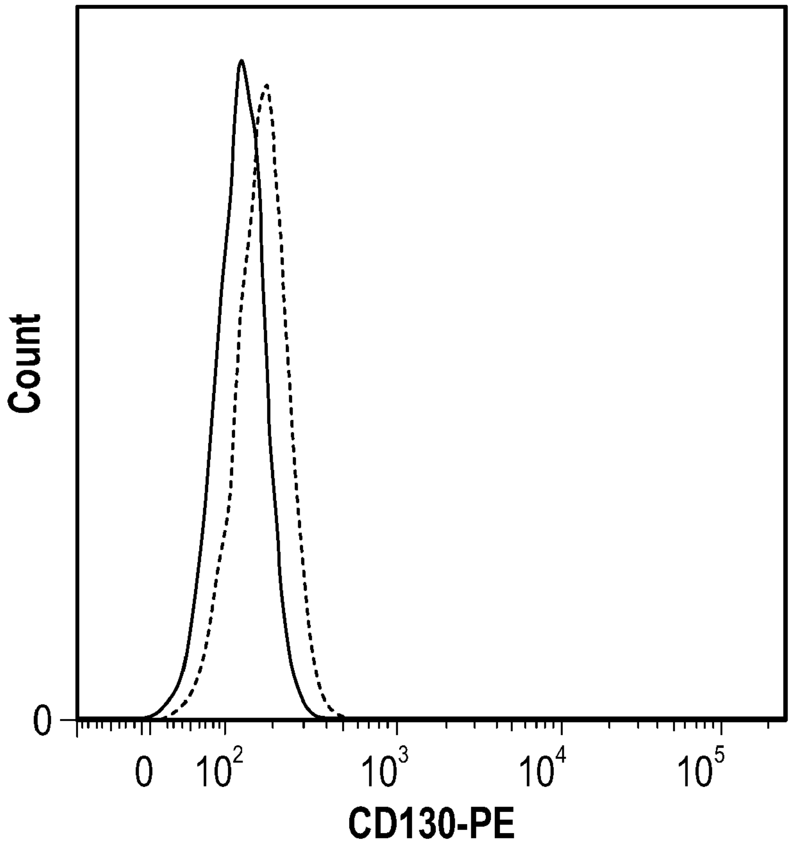


FIG. 33

| | Sample Name | Median, 488_585_42-A |
|---|---|---|
| ---------- | 20160711 KHYG1_CD130-PE_004.fcs | 186 |
| ———— | 20160711 KHYG1_BLANK_001.fcs | 127 |

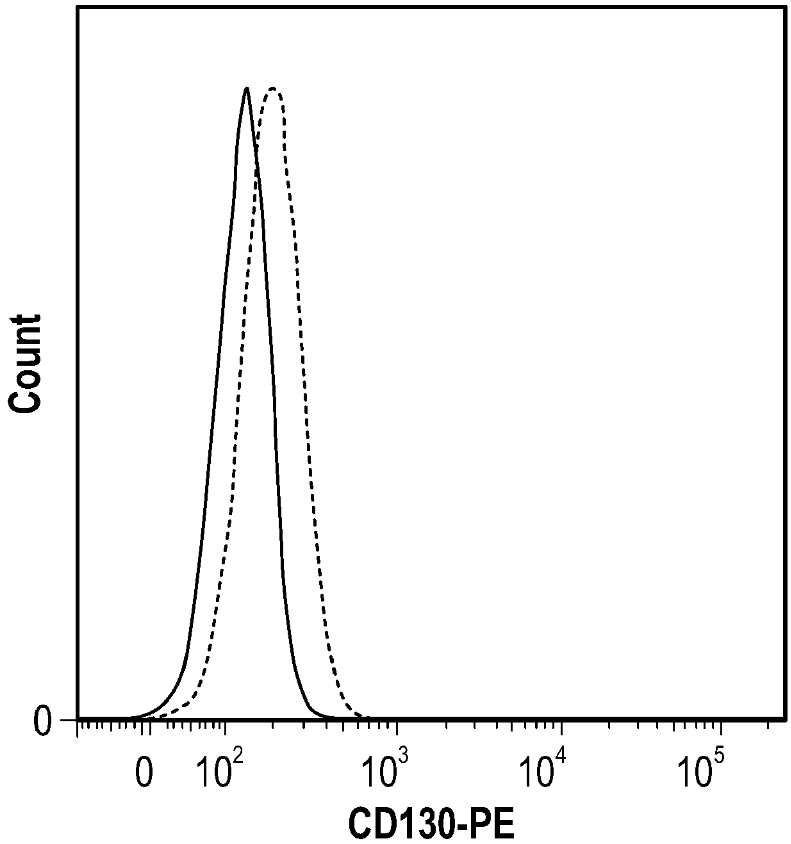


FIG. 34

| | Sample Name | Median, 488_585_42-A |
|---|---|---|
| ——— | 20160711 NK92_CD126-PE_007.fcs | 279 |
| (shaded) | 20160711 NK92_BLANK_005.fcs | 228 |

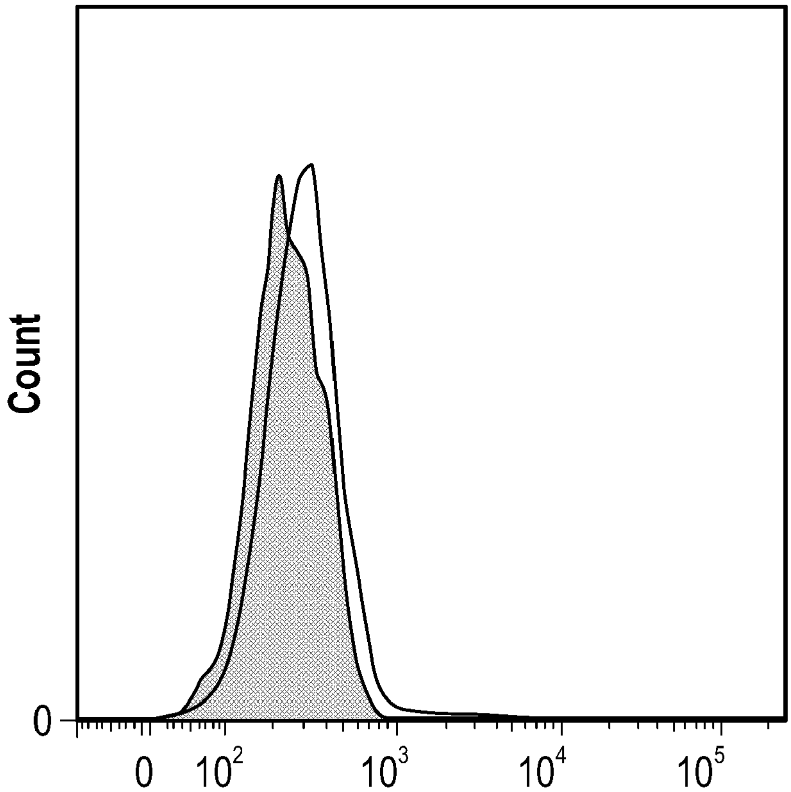


FIG. 35

| | Sample Name | Median, 488_585_42-A |
|---|---|---|
| ——— | 20160711 NK92_CD130-PE_008.fcs | 398 |
| (shaded) | 20160711 NK92_BLANK_005.fcs | 224 |

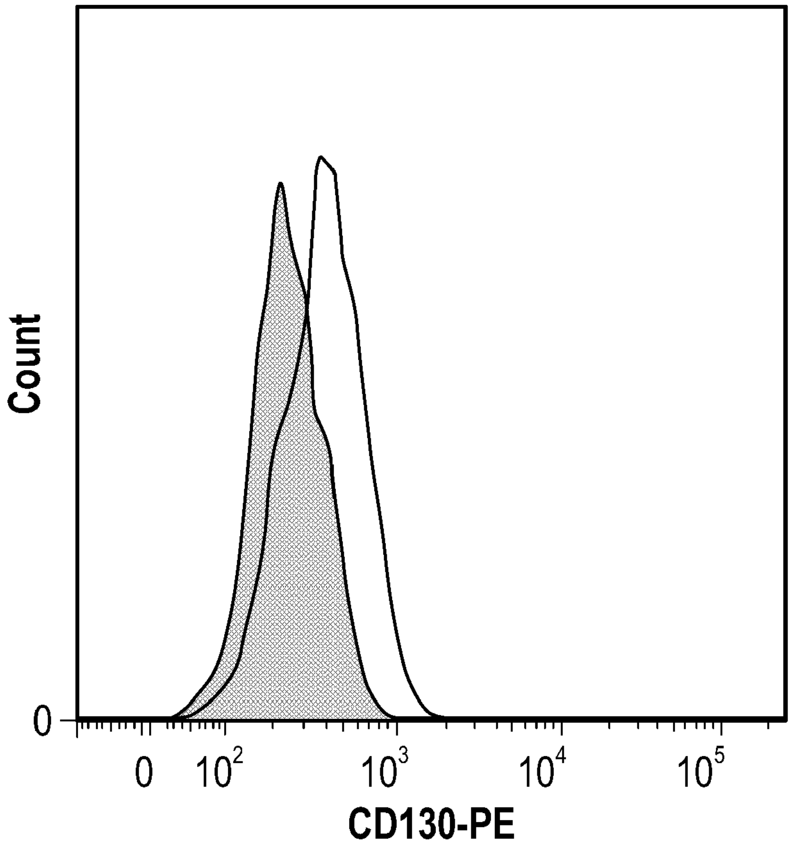


FIG. 36

| | Sample Name | Median, 488_585_42-A |
|---|---|---|
| ---------- | 20160711 U266_CD130-PE_012.fcs | 428 |
| (shaded box) | 20160711 U266_BLANK_009.fcs | 202 |
| —— | 20160711 U266_CD126-PE_011.fcs | 2205 |

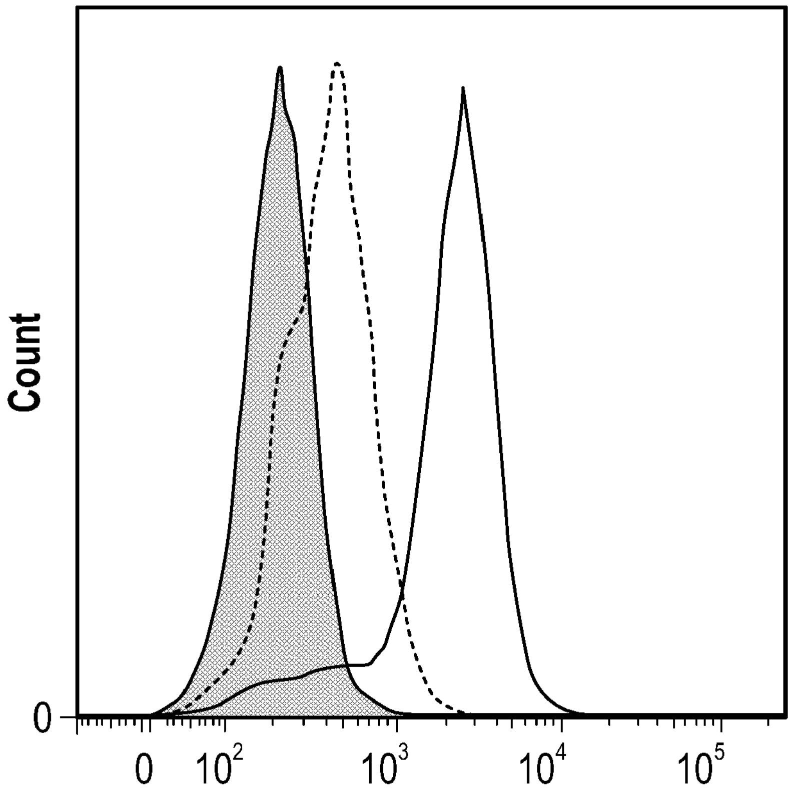


FIG. 37

| | Sample Name | Median, 488_585_42-A |
|---|---|---|
| ---------- | 8226 CD126_CD130-PE_017.fcs | 868 |
| ——— | 8226 CD126_CD126-PE_016.fcs | 1399 |
| (shaded) | 8226 CD126_BLANK_013.fcs | 130 |

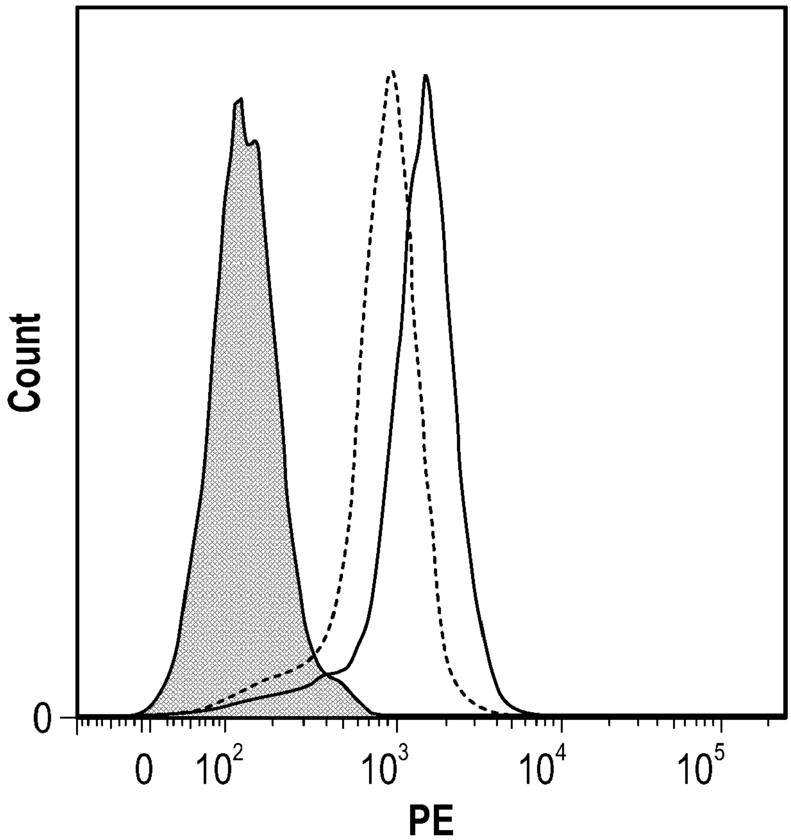


FIG. 38

| | Sample Name | Median, 488_585_42-A |
|---|---|---|
| - - - - - - | H929 CD126_CD130-PE_022.fcs | 156 |
| ——— | H929 CD126_CD126-PE_020.fcs | 978 |
| (shaded) | H929 CD126_BLANK_018.fcs | 116 |

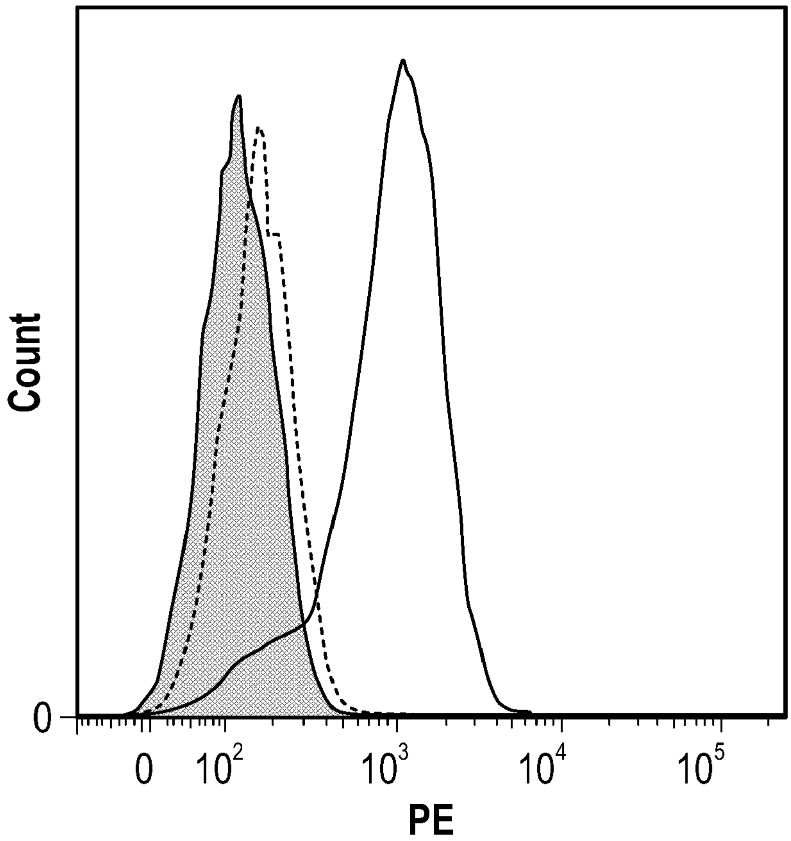


FIG. 39

| | Sample Name | Median, 488_585_42-A |
|---|---|---|
| | KMS11 CD126_CD130-PE_031.fcs | 1071 |
| | KMS11 CD126_CD126-PE_028.fcs | 190 |
| | KMS11 CD126_BLANK_026.fcs | 119 |

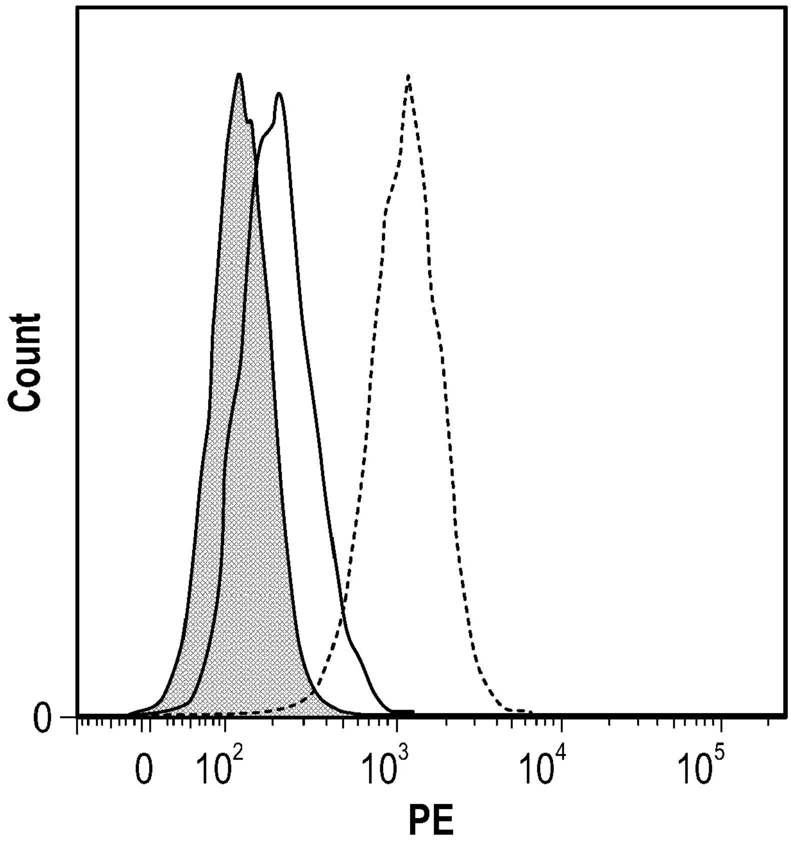


FIG. 40

| | Sample Name | Median, 488_585_42-A |
|---|---|---|
| | 20160831 MM1S_CD130-PE_004.fcs | 390 |
| | 20160831 MM1S_CD126-PE_002.fcs | 1214 |
| | 20160831 MM1S_BLANK_001.fcs | 82.1 |

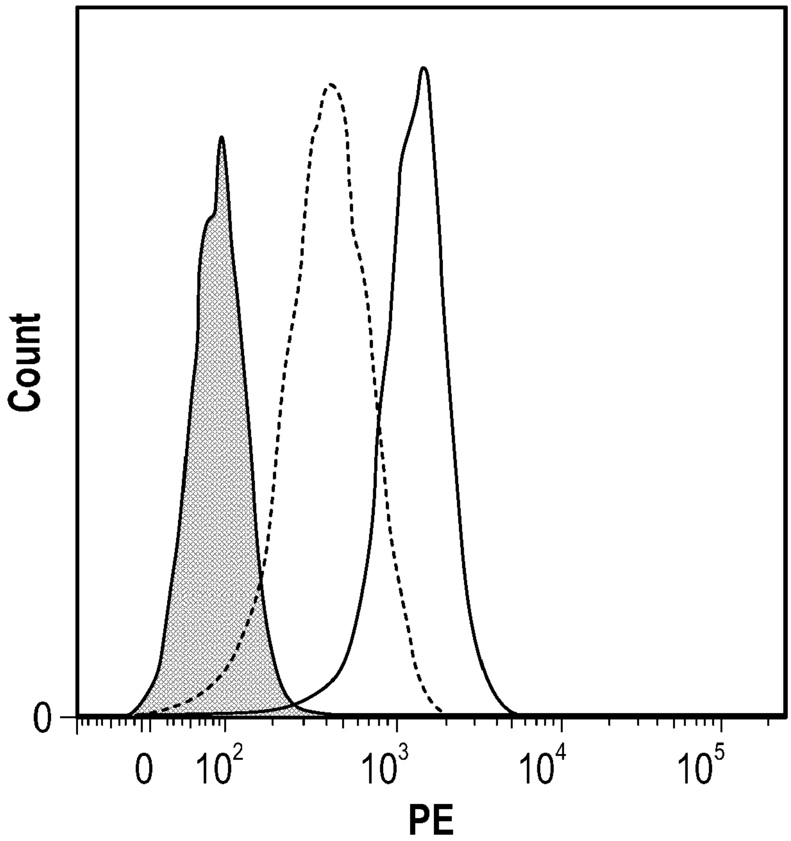


FIG. 41

| | Sample Name | Median, 488_585_42-A |
|---|---|---|
| - ·· - ·· - | K562 CD126_CD126-PE_001_012.fcs | 98.0 |
| ——— | K562 CD126_BLANK_010.fcs | 94.6 |

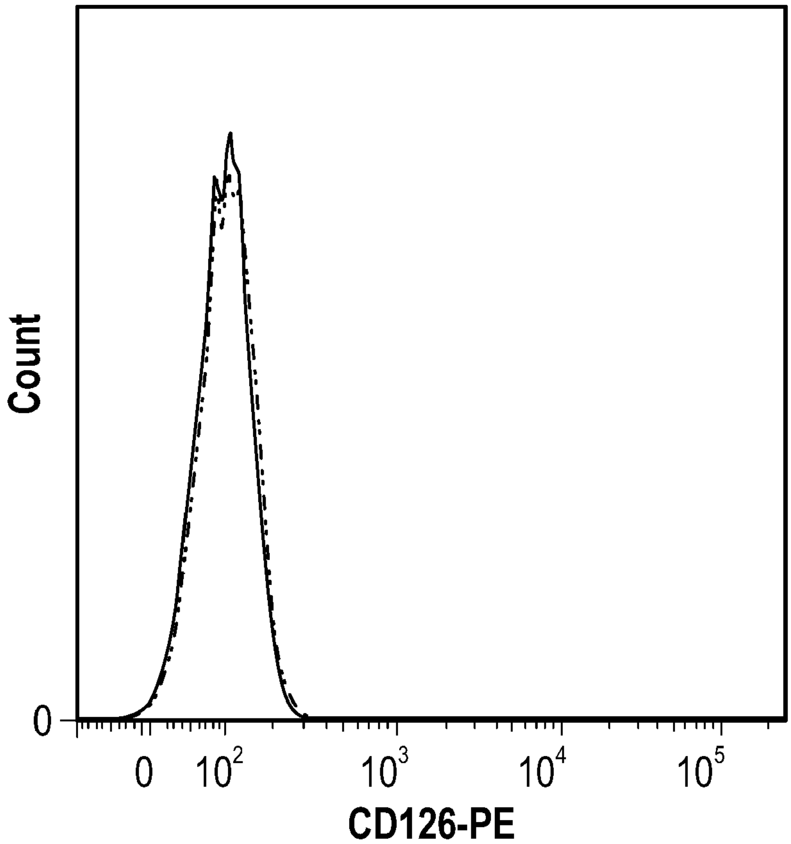


FIG. 42

| | Sample Name | Median, 488_585_42-A |
|---|---|---|
| – ·· – ·· – | 20160706 K562_CD126-PE_010.fcs | 82.3 |
| ——— | 20160706 K562_BLANK_008.fcs | 74.7 |

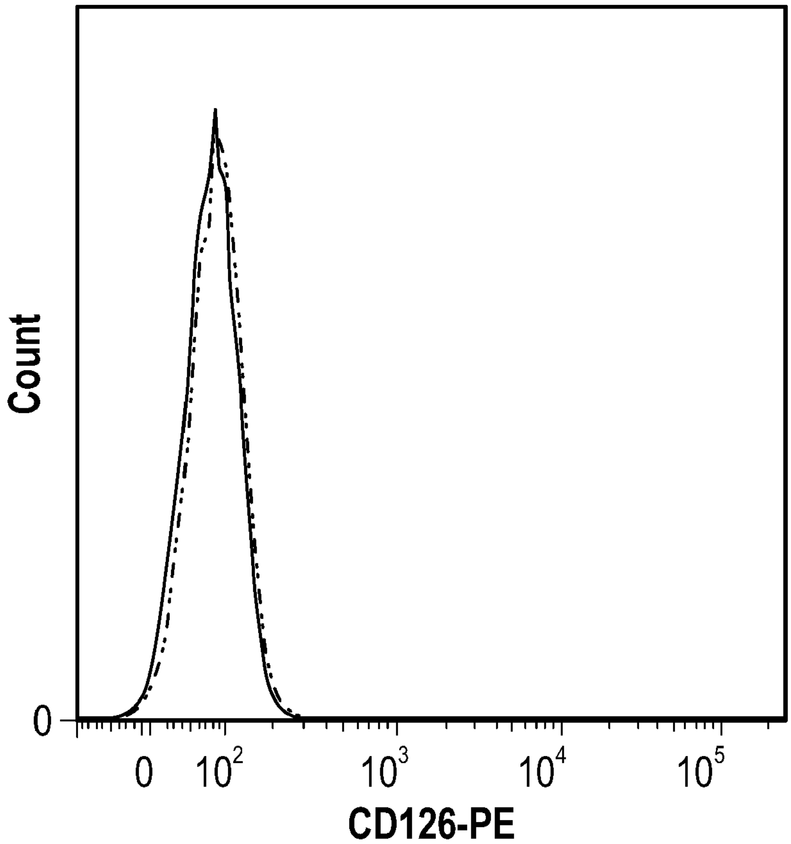


FIG. 43

| | Sample Name | Median, 488_585_42-A |
|---|---|---|
| (line) | 20160706 K562_CD130-PE_011.fcs | 120 |
| (shaded) | 20160706 K562_BLANK_001_012.fcs | 74.3 |

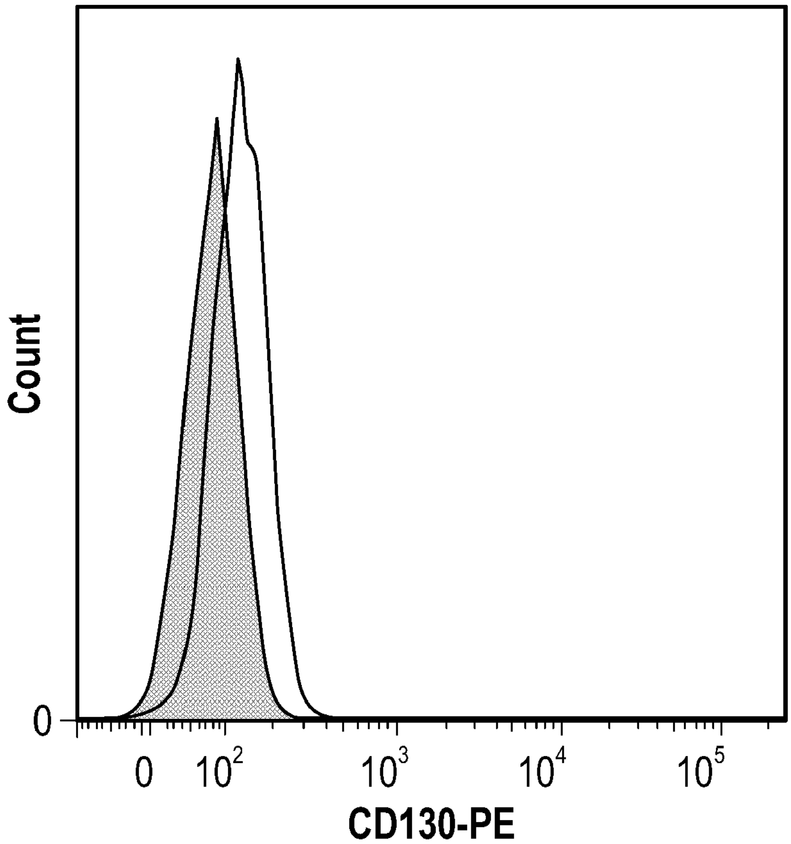


FIG. 44

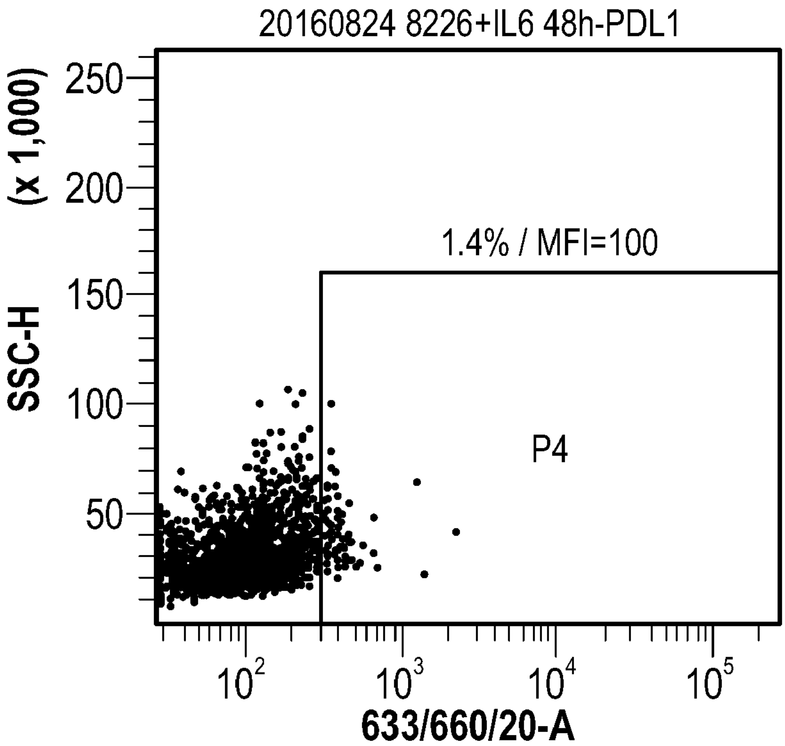


Vehicle

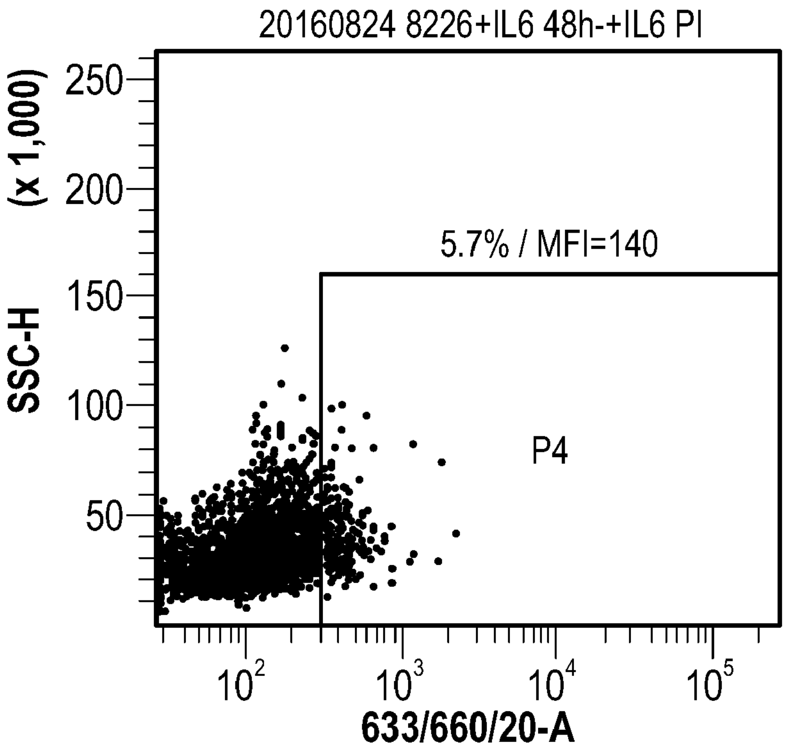


+IL-6 (100ng/mL, 48hrs)

FIG. 45

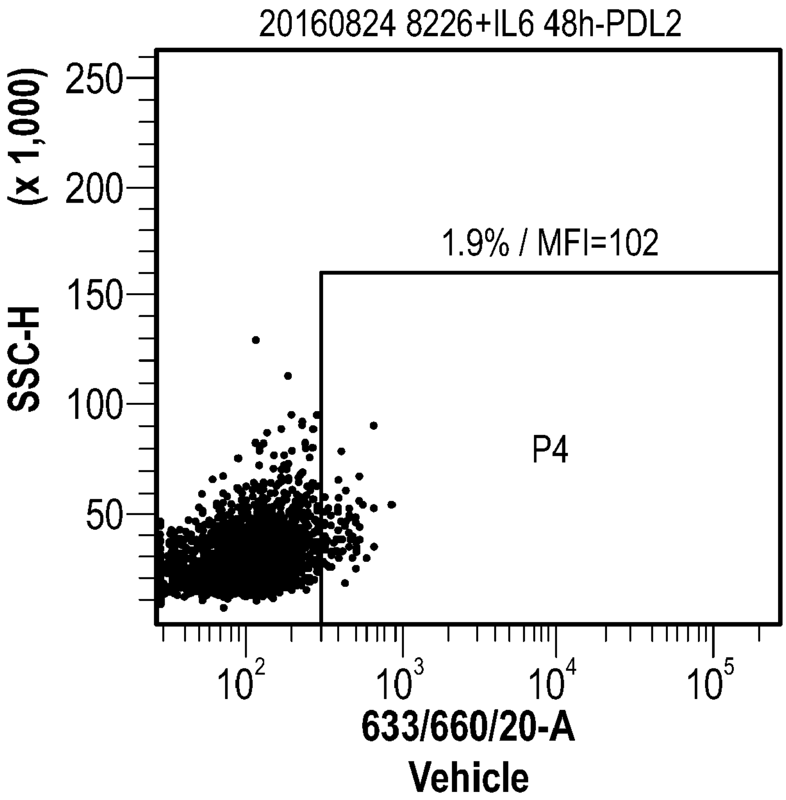
20160824 8226+IL6 48h-PDL2
SSC-H (x 1,000)
250
200
150
100
50
1.9% / MFI=102
P4
10^2
10^3
10^4
10^5
633/660/20-A
Vehicle

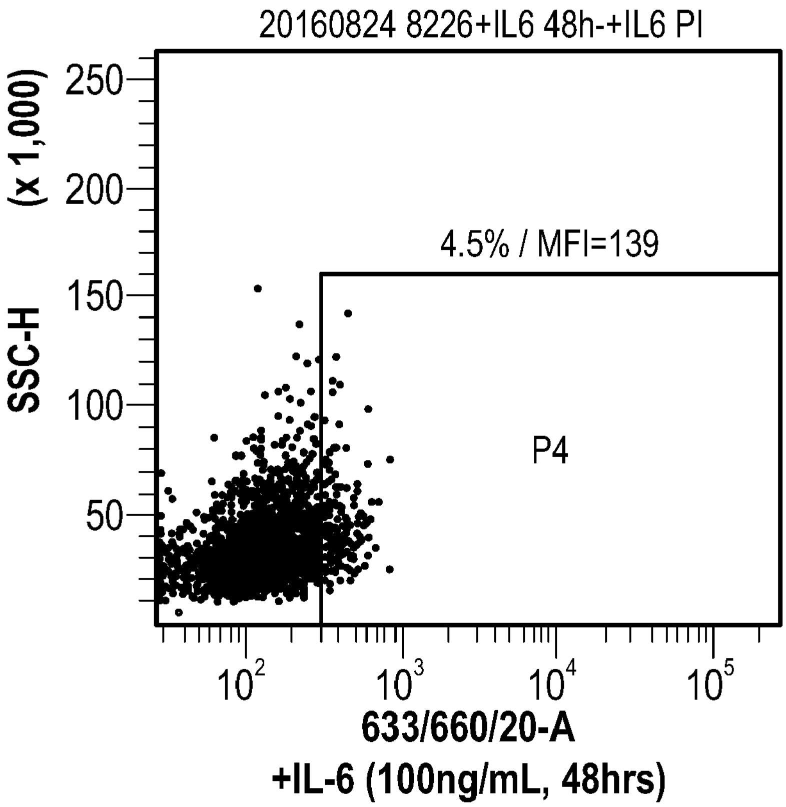
20160824 8226+IL6 48h-+IL6 PI
SSC-H (x 1,000)
250
200
150
100
50
4.5% / MFI=139
P4
10^2
10^3
10^4
10^5
633/660/20-A
+IL-6 (100ng/mL, 48hrs)

FIG. 46

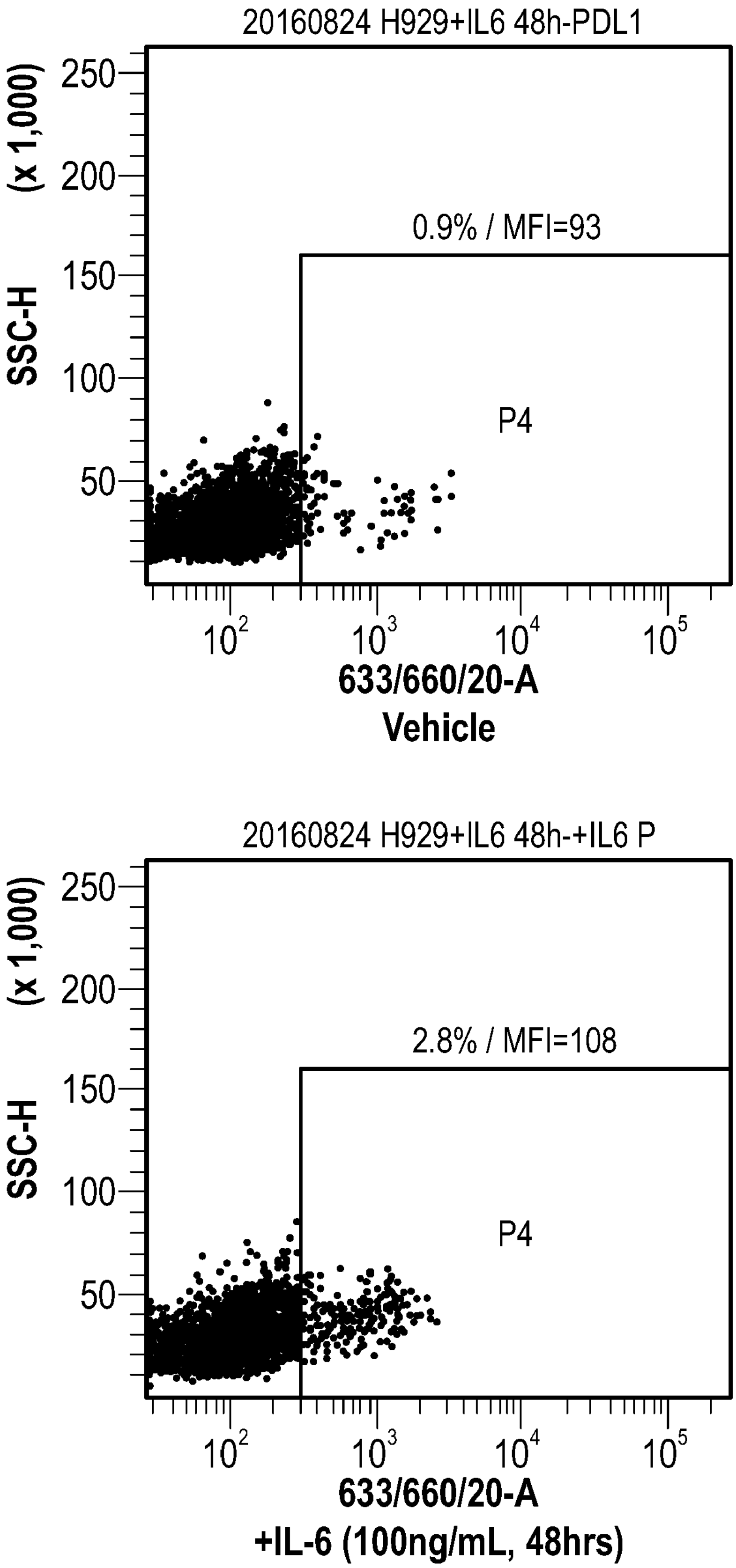
20160824 H929+IL6 48h-PDL1
SSC-H (x 1,000)
250
200
150
100
50
0.9% / MFI=93
P4
10^2
10^3
10^4
10^5
633/660/20-A
Vehicle
20160824 H929+IL6 48h-+IL6 P
SSC-H (x 1,000)
250
200
150
100
50
2.8% / MFI=108
P4
10^2
10^3
10^4
10^5
633/660/20-A
+IL-6 (100ng/mL, 48hrs)

FIG. 47

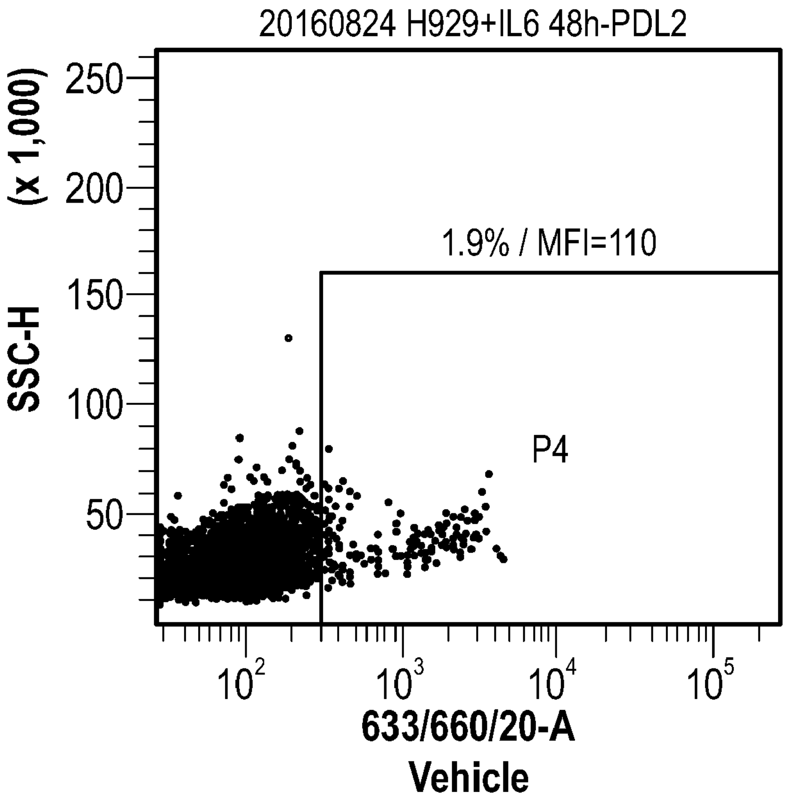
20160824 H929+IL6 48h-PDL2
SSC-H (x 1,000)
250
200
150
100
50
1.9% / MFI=110
P4
$10^2$
$10^3$
$10^4$
$10^5$
633/660/20-A
Vehicle

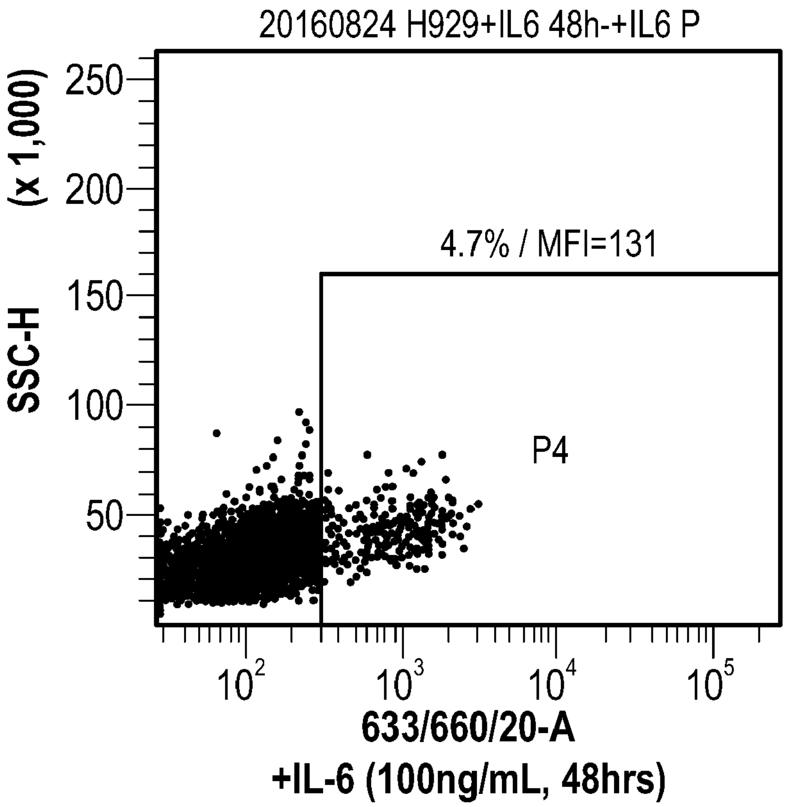
20160824 H929+IL6 48h-+IL6 P
SSC-H (x 1,000)
250
200
150
100
50
4.7% / MFI=131
P4
$10^2$
$10^3$
$10^4$
$10^5$
633/660/20-A
+IL-6 (100ng/mL, 48hrs)

FIG. 48

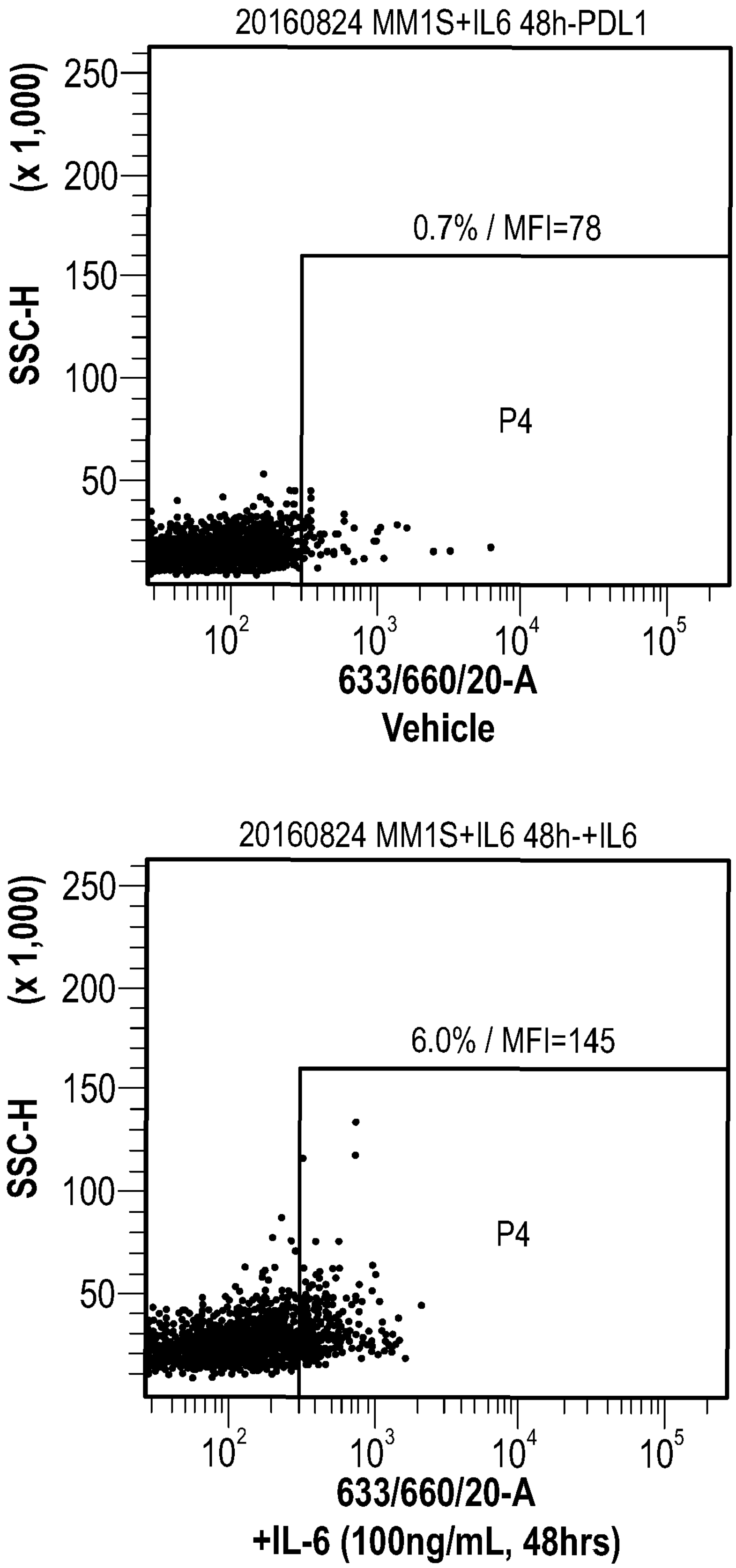
20160824 MM1S+IL6 48h-PDL1
SSC-H (x 1,000)
250
200
150
100
50
0.7% / MFI=78
P4
10^2
10^3
10^4
10^5
633/660/20-A
Vehicle
20160824 MM1S+IL6 48h-+IL6
SSC-H (x 1,000)
250
200
150
100
50
6.0% / MFI=145
P4
10^2
10^3
10^4
10^5
633/660/20-A
+IL-6 (100ng/mL, 48hrs)

FIG. 49

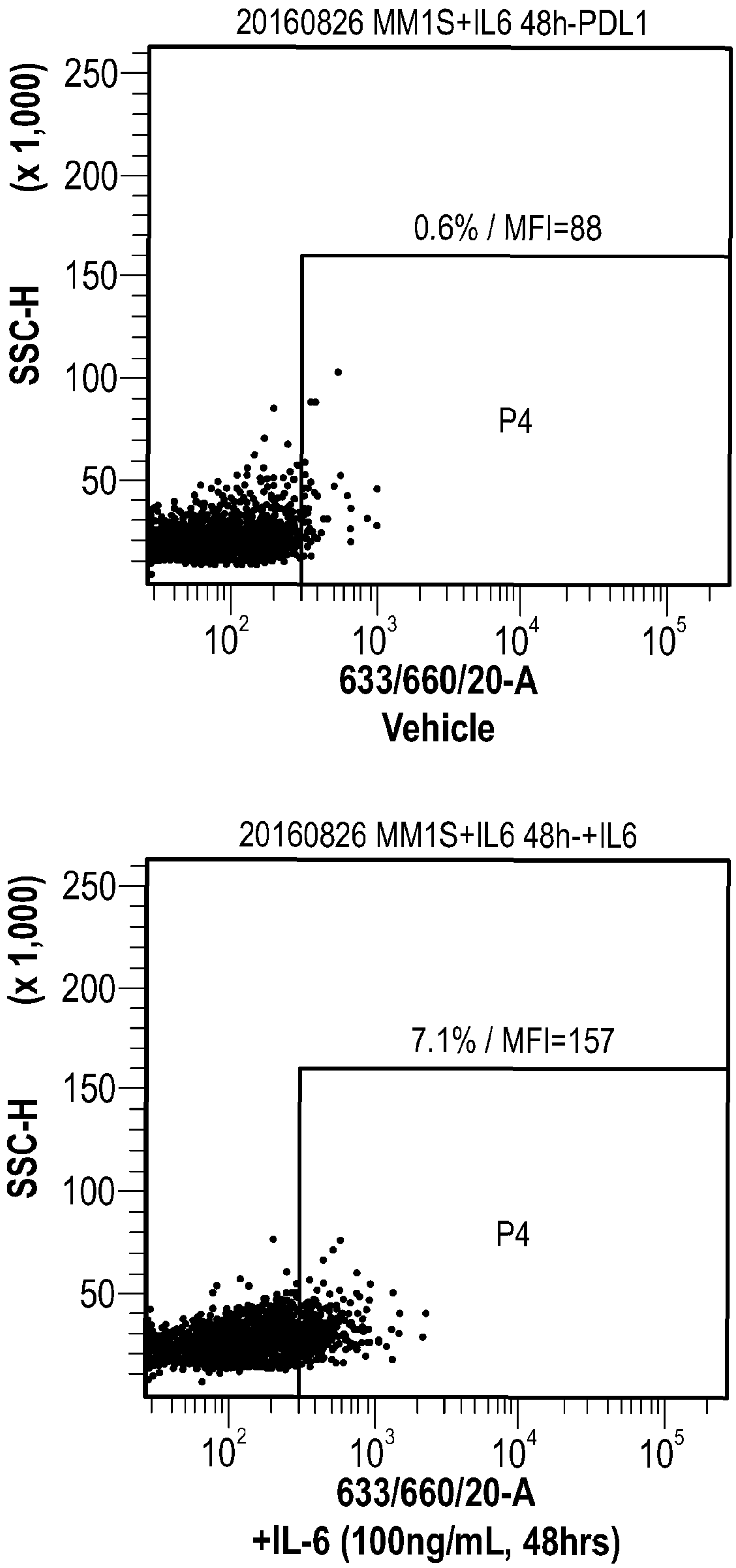
20160826 MM1S+IL6 48h-PDL1
SSC-H (x 1,000)
250
200
150
100
50
0.6% / MFI=88
P4
$10^2$
$10^3$
$10^4$
$10^5$
633/660/20-A
Vehicle
20160826 MM1S+IL6 48h-+IL6
SSC-H (x 1,000)
250
200
150
100
50
7.1% / MFI=157
P4
$10^2$
$10^3$
$10^4$
$10^5$
633/660/20-A
+IL-6 (100ng/mL, 48hrs)

FIG. 50

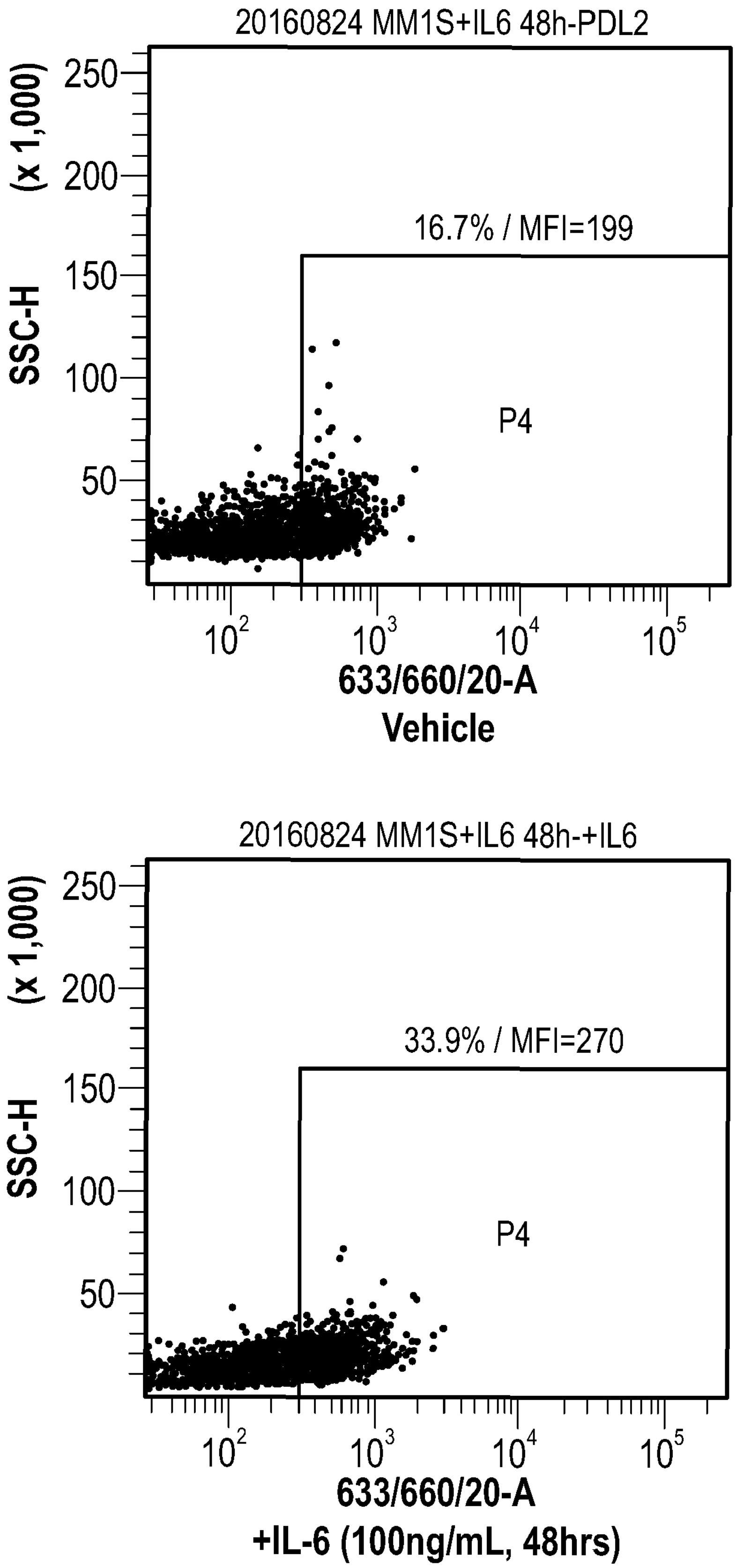
20160824 MM1S+IL6 48h-PDL2
SSC-H (x 1,000)
250
200
150
100
50
16.7% / MFI=199
P4
$10^2$
$10^3$
$10^4$
$10^5$
633/660/20-A
Vehicle
20160824 MM1S+IL6 48h-+IL6
SSC-H (x 1,000)
250
200
150
100
50
33.9% / MFI=270
P4
$10^2$
$10^3$
$10^4$
$10^5$
633/660/20-A
+IL-6 (100ng/mL, 48hrs)

FIG. 51

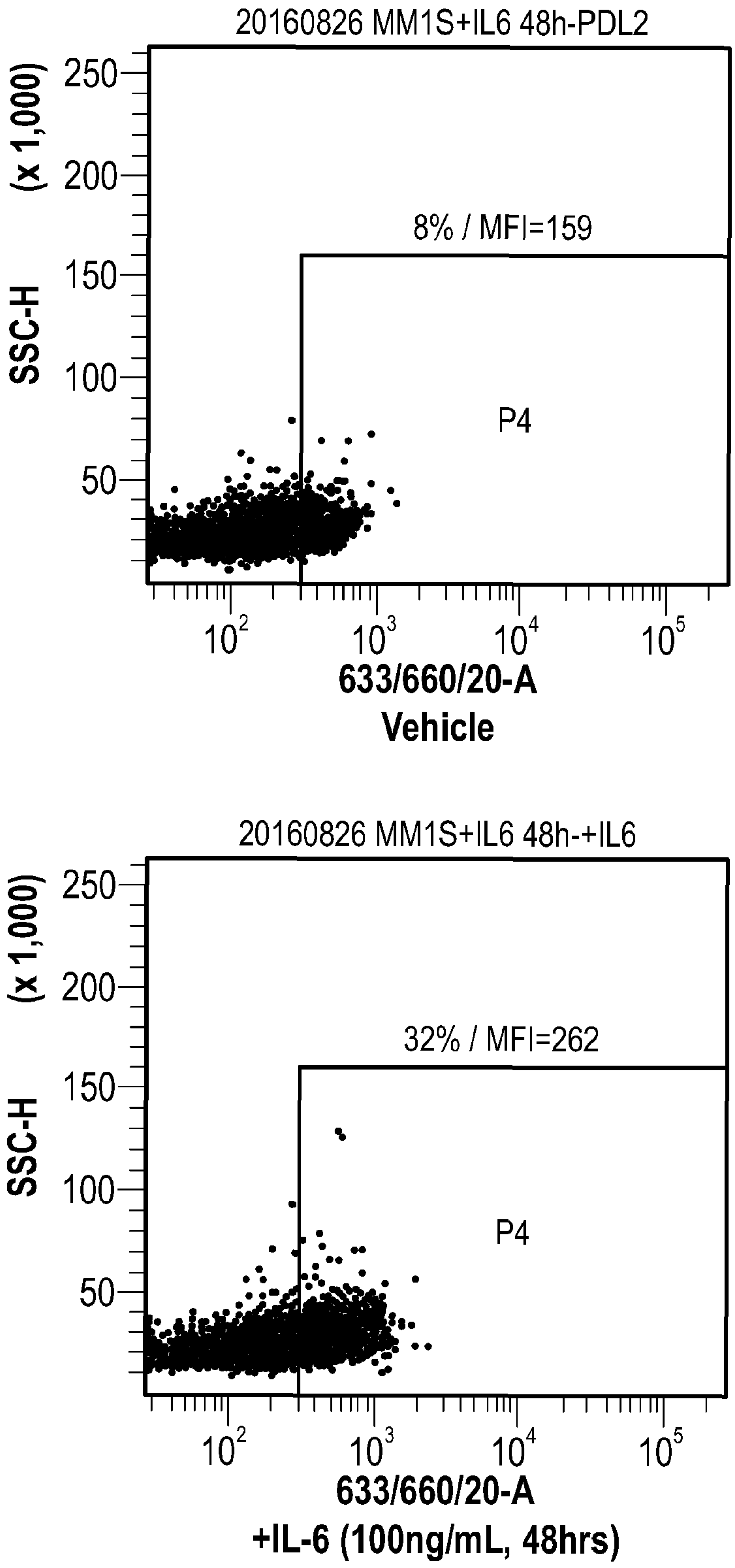
20160826 MM1S+IL6 48h-PDL2
SSC-H (x 1,000)
250
200
150
100
50
8% / MFI=159
P4
10^2
10^3
10^4
10^5
633/660/20-A
Vehicle
20160826 MM1S+IL6 48h-+IL6
SSC-H (x 1,000)
250
200
150
100
50
32% / MFI=262
P4
10^2
10^3
10^4
10^5
633/660/20-A
+IL-6 (100ng/mL, 48hrs)

FIG. 52

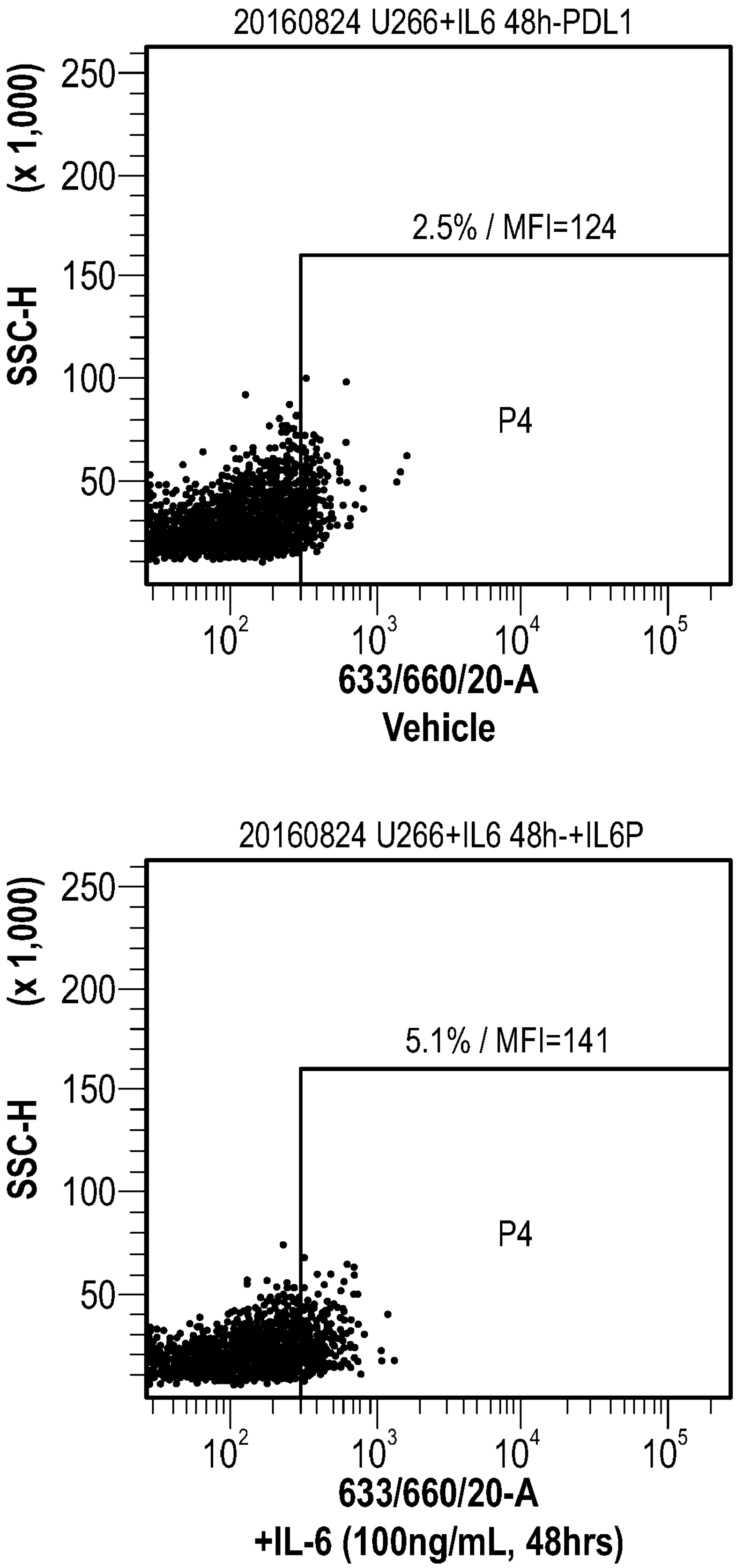
20160824 U266+IL6 48h-PDL1
SSC-H (x 1,000)
250
200
150
100
50
2.5% / MFI=124
P4
10^2
10^3
10^4
10^5
633/660/20-A
Vehicle
20160824 U266+IL6 48h-+IL6P
SSC-H (x 1,000)
250
200
150
100
50
5.1% / MFI=141
P4
10^2
10^3
10^4
10^5
633/660/20-A
+IL-6 (100ng/mL, 48hrs)

FIG. 53

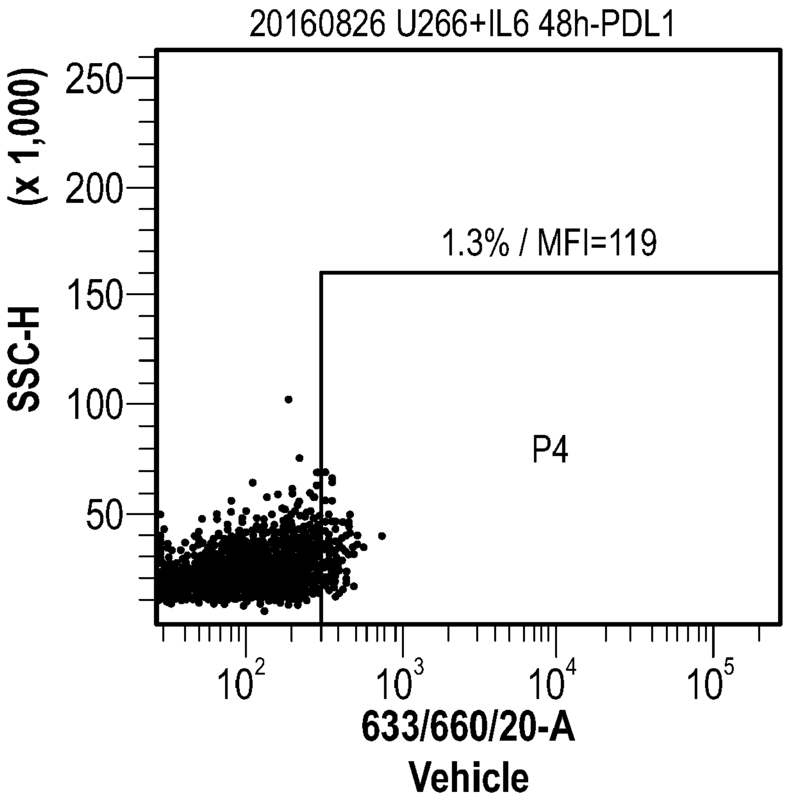
20160826 U266+IL6 48h-PDL1
SSC-H (x 1,000)
250
200
150
100
50
1.3% / MFI=119
P4
$10^2$
$10^3$
$10^4$
$10^5$
633/660/20-A
Vehicle

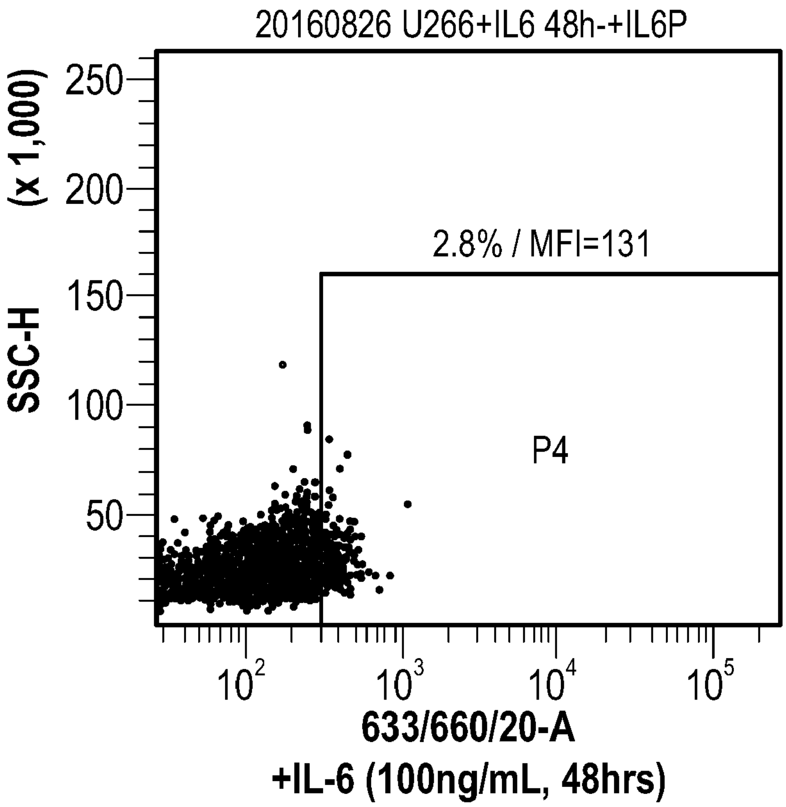
20160826 U266+IL6 48h-+IL6P
SSC-H (x 1,000)
250
200
150
100
50
2.8% / MFI=131
P4
$10^2$
$10^3$
$10^4$
$10^5$
633/660/20-A
+IL-6 (100ng/mL, 48hrs)

FIG. 54

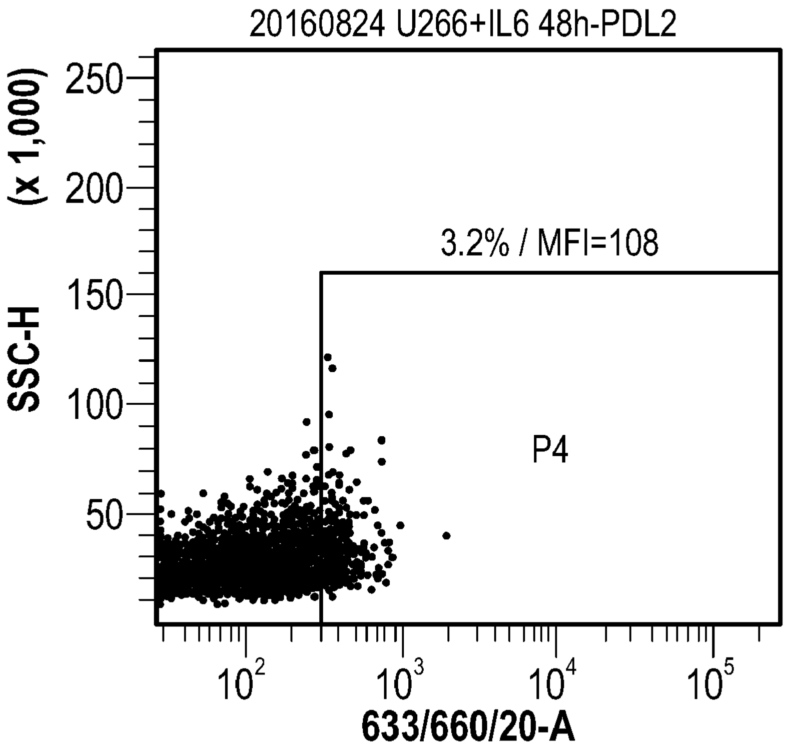

Vehicle

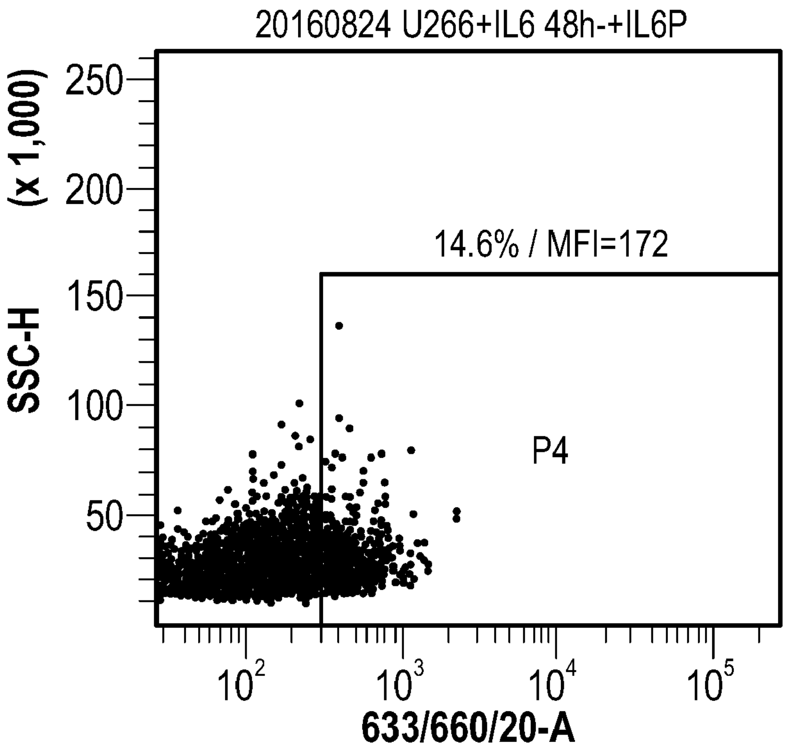

+IL-6 (100ng/mL, 48hrs)

FIG. 55

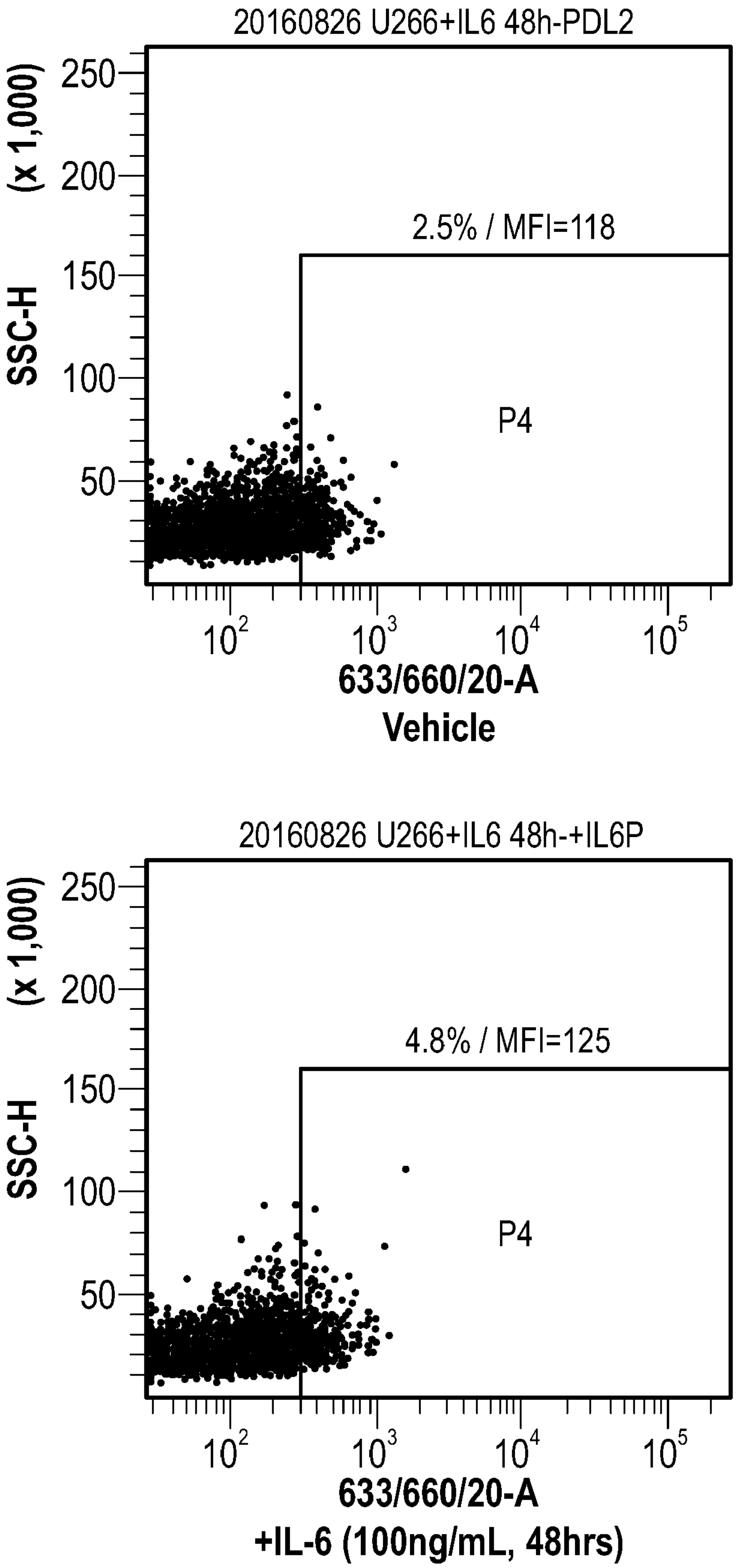
20160826 U266+IL6 48h-PDL2
SSC-H (x 1,000)
250
200
150
100
50
2.5% / MFI=118
P4
$10^2$
$10^3$
$10^4$
$10^5$
633/660/20-A
Vehicle
20160826 U266+IL6 48h-+IL6P
SSC-H (x 1,000)
250
200
150
100
50
4.8% / MFI=125
P4
$10^2$
$10^3$
$10^4$
$10^5$
633/660/20-A
+IL-6 (100ng/mL, 48hrs)

FIG. 56

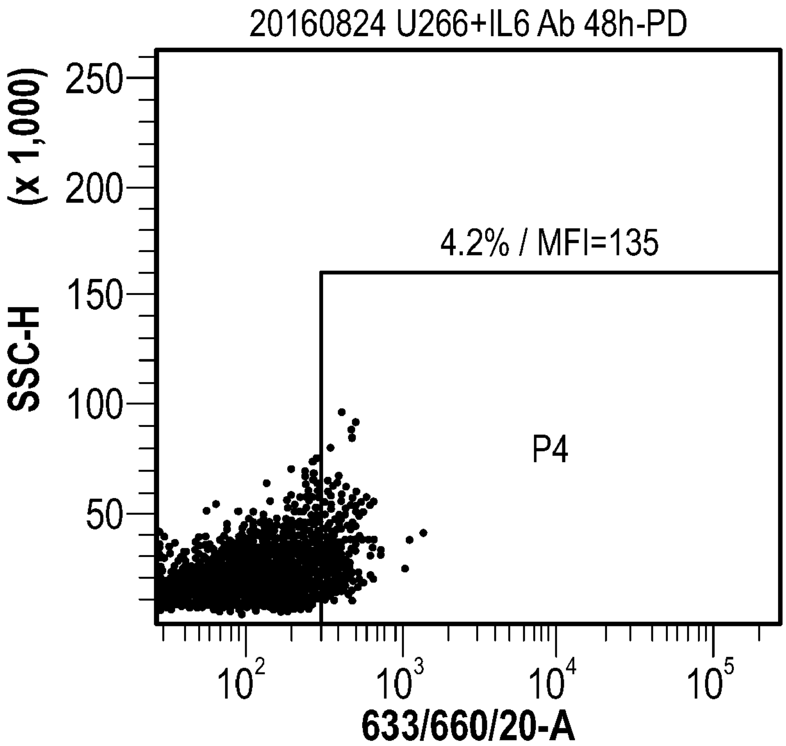


+Isotype control

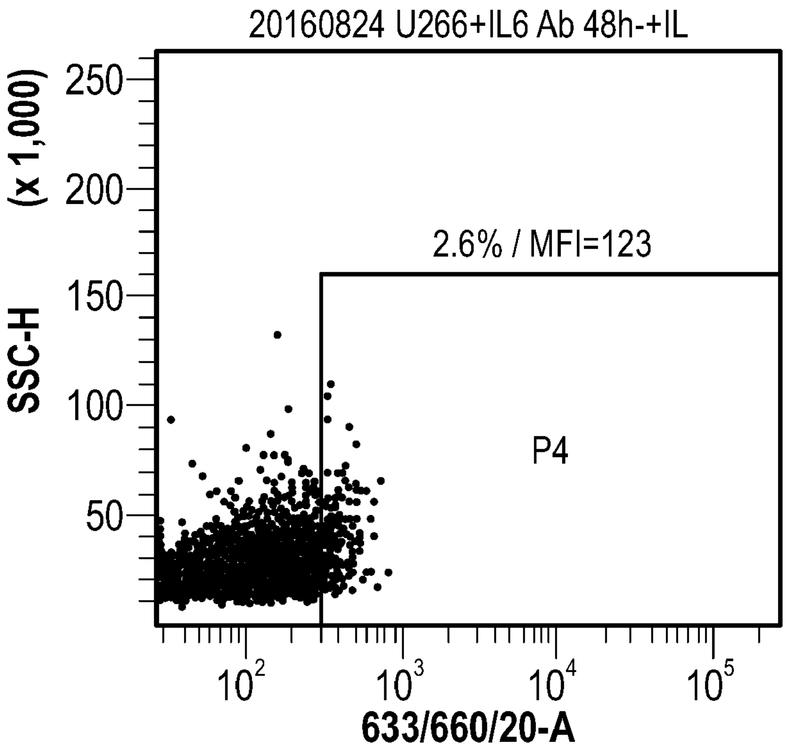


+IL-6 blocking Ab (10ug/mL, 48hrs)

FIG. 57

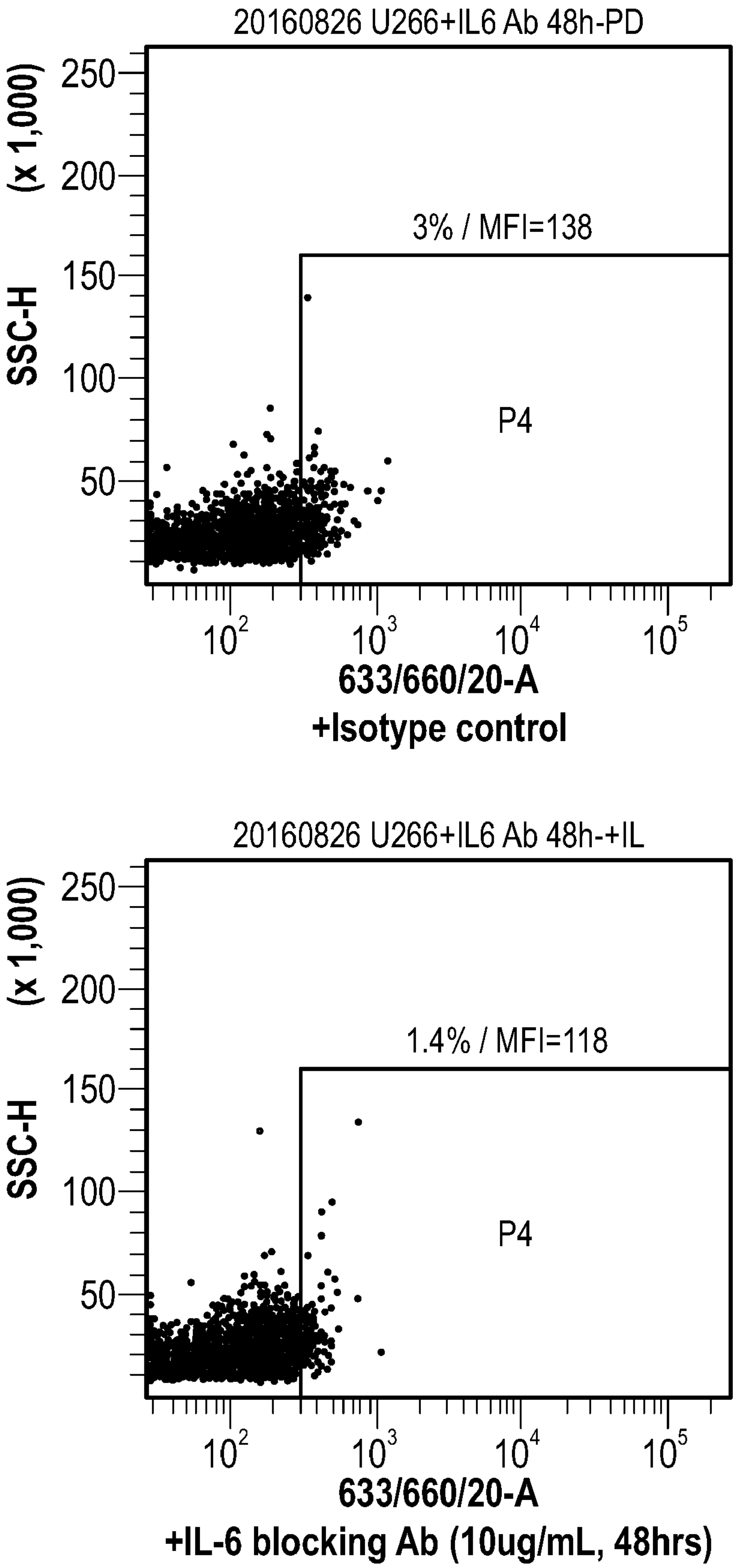
20160826 U266+IL6 Ab 48h-PD
SSC-H (x 1,000)
250
200
150
100
50
3% / MFI=138
P4
$10^2$
$10^3$
$10^4$
$10^5$
633/660/20-A
+Isotype control
20160826 U266+IL6 Ab 48h-+IL
SSC-H (x 1,000)
250
200
150
100
50
1.4% / MFI=118
P4
$10^2$
$10^3$
$10^4$
$10^5$
633/660/20-A
+IL-6 blocking Ab (10ug/mL, 48hrs)

FIG. 58

| | Sample Name | Median, 633_660_20-A |
|---|---|---|
| | 20160824 U266+IL6 Ab 48h_+IL6 Ab PDL1_027.fcs | 110 |
| | 20160824 U266+IL6 Ab 48h_PDL1_024.fcs | 119 |
| | 20160824 U266+IL6 Ab 48h_+IL6 Ab BLANK_026.fcs | 75.3 |

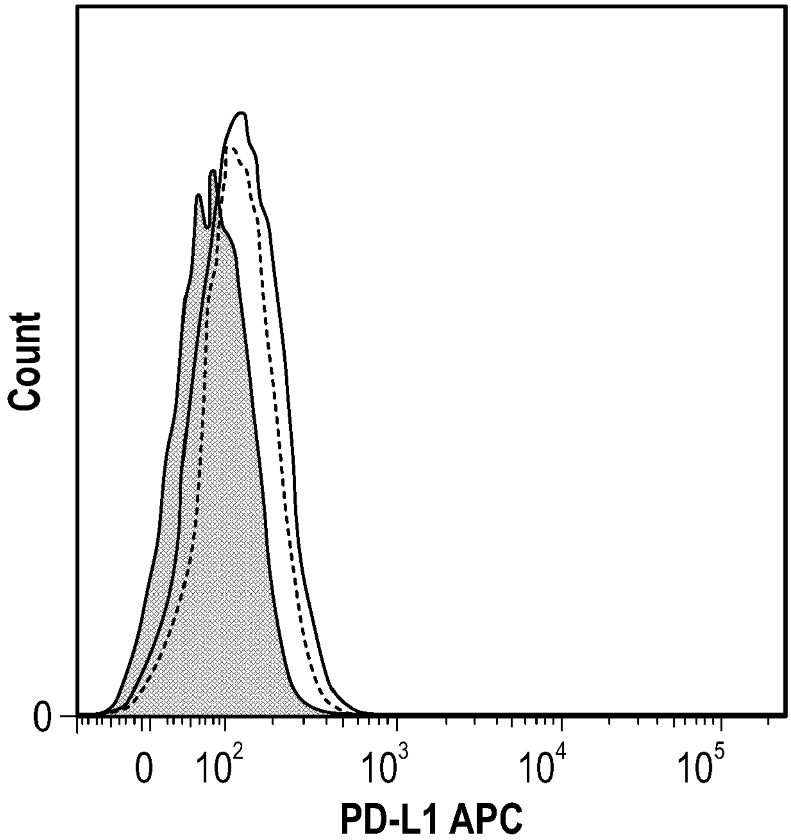


FIG. 59

| | Sample Name | Median, 633_660_20-A |
|---|---|---|
| - - - - - - - - - - | 20160826 U266+IL6 Ab 48h_+IL6 Ab PDL1_015.fcs | 108 |
| ——— | 20160826 U266+IL6 Ab 48h_PDL1 iso_012.fcs | 128 |
| (shaded) | 20160826 U266+IL6 Ab 48h_+IL6 Ab BLANK_014.fcs | 82.9 |

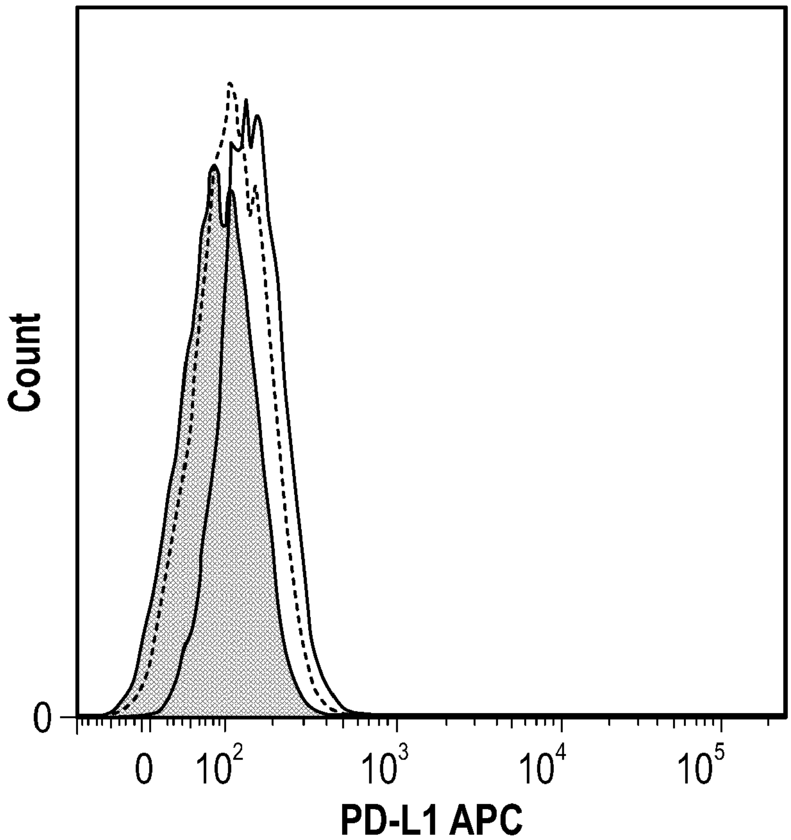


FIG. 60

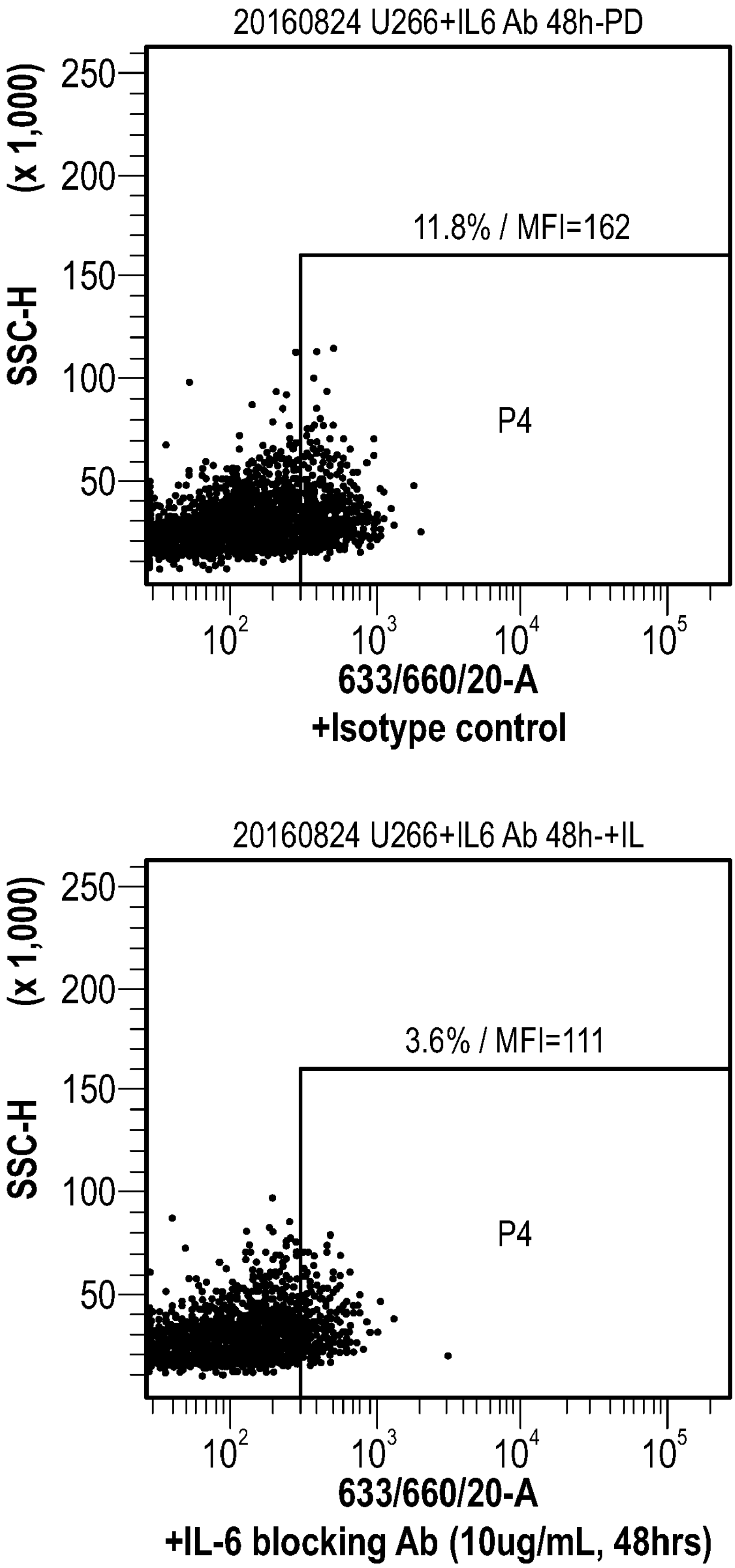
20160824 U266+IL6 Ab 48h-PD
SSC-H (x 1,000)
250
200
150
100
50
11.8% / MFI=162
P4
$10^2$
$10^3$
$10^4$
$10^5$
633/660/20-A
+Isotype control
20160824 U266+IL6 Ab 48h-+IL
SSC-H (x 1,000)
250
200
150
100
50
3.6% / MFI=111
P4
$10^2$
$10^3$
$10^4$
$10^5$
633/660/20-A
+IL-6 blocking Ab (10ug/mL, 48hrs)

**FIG. 61**

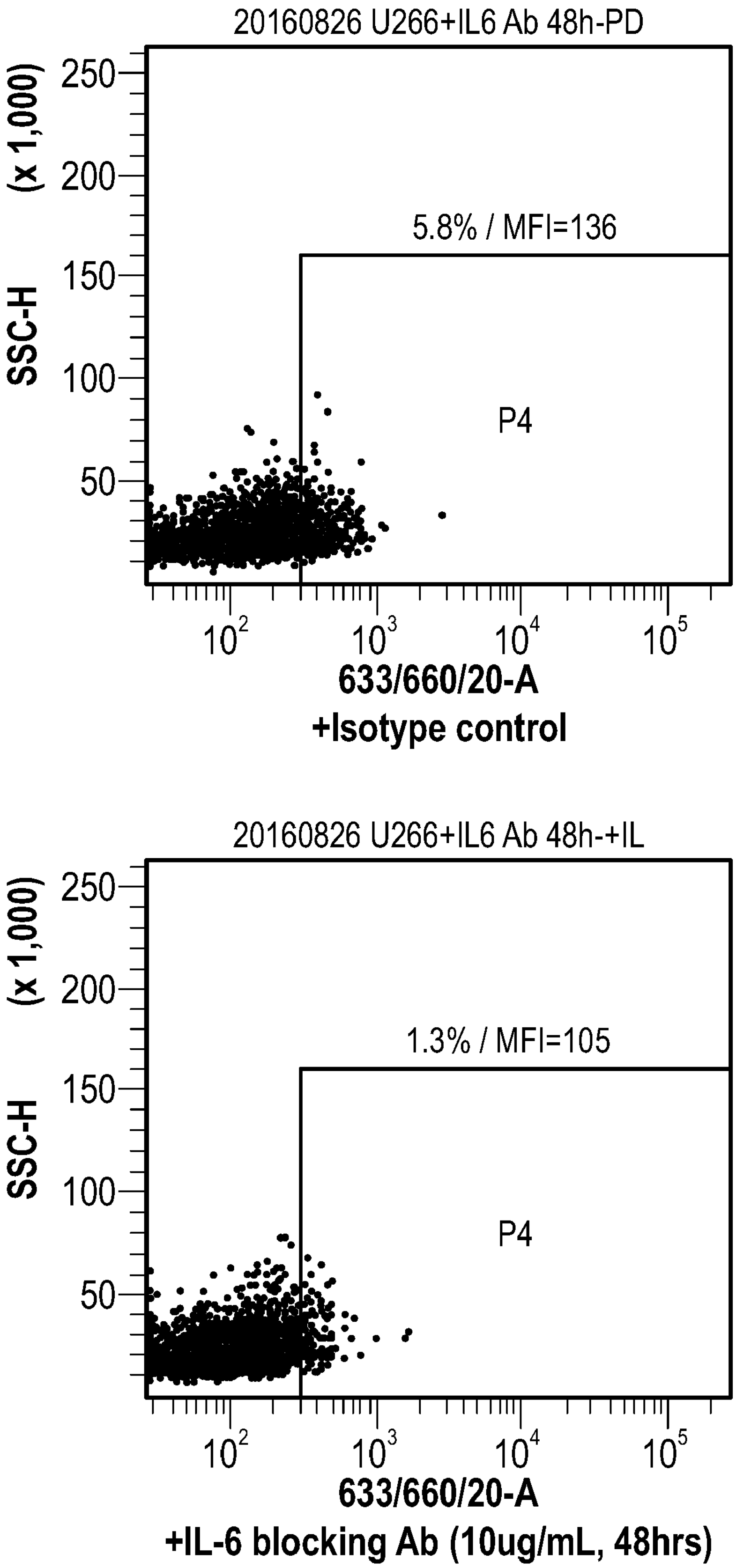
20160826 U266+IL6 Ab 48h-PD
SSC-H (x 1,000)
250
200
150
100
50
5.8% / MFI=136
P4
$10^2$
$10^3$
$10^4$
$10^5$
633/660/20-A
+Isotype control
20160826 U266+IL6 Ab 48h-+IL
SSC-H (x 1,000)
250
200
150
100
50
1.3% / MFI=105
P4
$10^2$
$10^3$
$10^4$
$10^5$
633/660/20-A
+IL-6 blocking Ab (10ug/mL, 48hrs)

FIG. 62

| | Sample Name | Median, 633_660_20-A |
|---|---|---|
| - - - - - - - - - - | 20160824 U266+IL6 Ab 48h_+IL6 Ab PDL2_028.fcs | 90.7 |
| ———— | 20160824 U266+IL6 Ab 48h_PDL2_025.fcs | 123 |
| (shaded) | 20160824 U266+IL6 Ab 48h_BLANK_023.fcs | 84.9 |

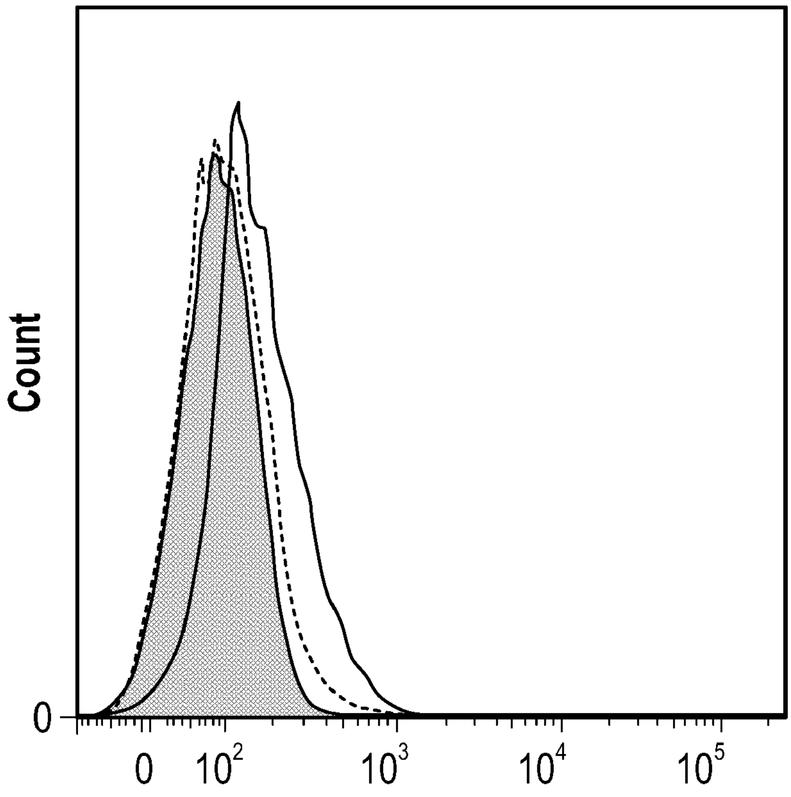


FIG. 63

| | Sample Name | Median, 633_660_20-A |
|---|---|---|
| | 20160826 U266+IL6 Ab 48h_+IL6 Ab PDL2_016.fcs | 92.9 |
| | 20160826 U266+IL6 Ab 48h_PDL2_013.fcs | 116 |
| | 20160826 U266+IL6 Ab 48h_BLANK iso_011.fcs | 93.8 |

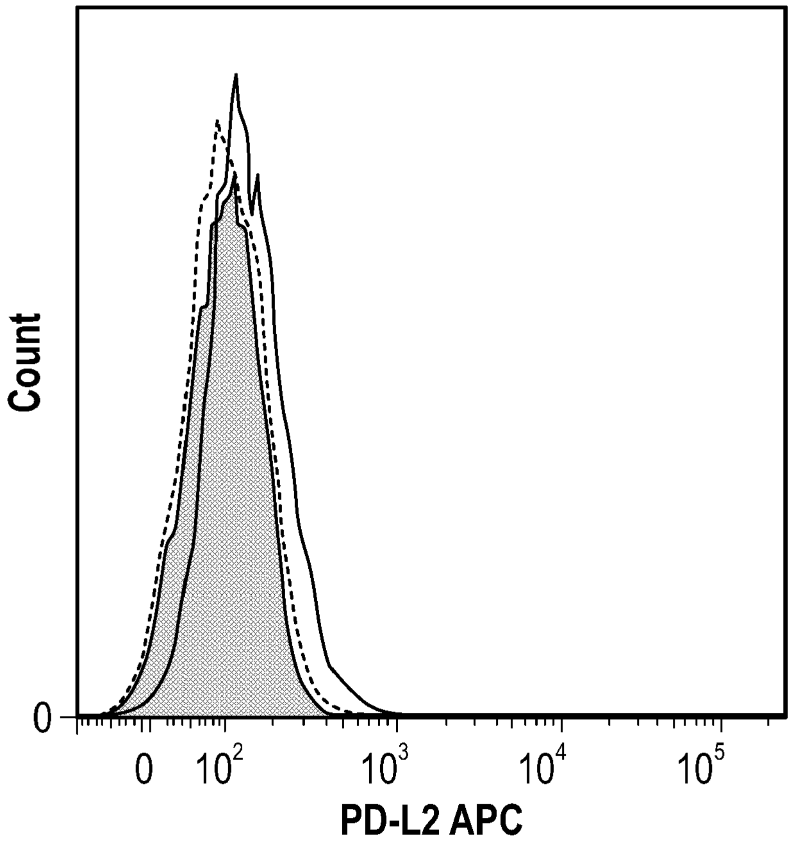


FIG. 64

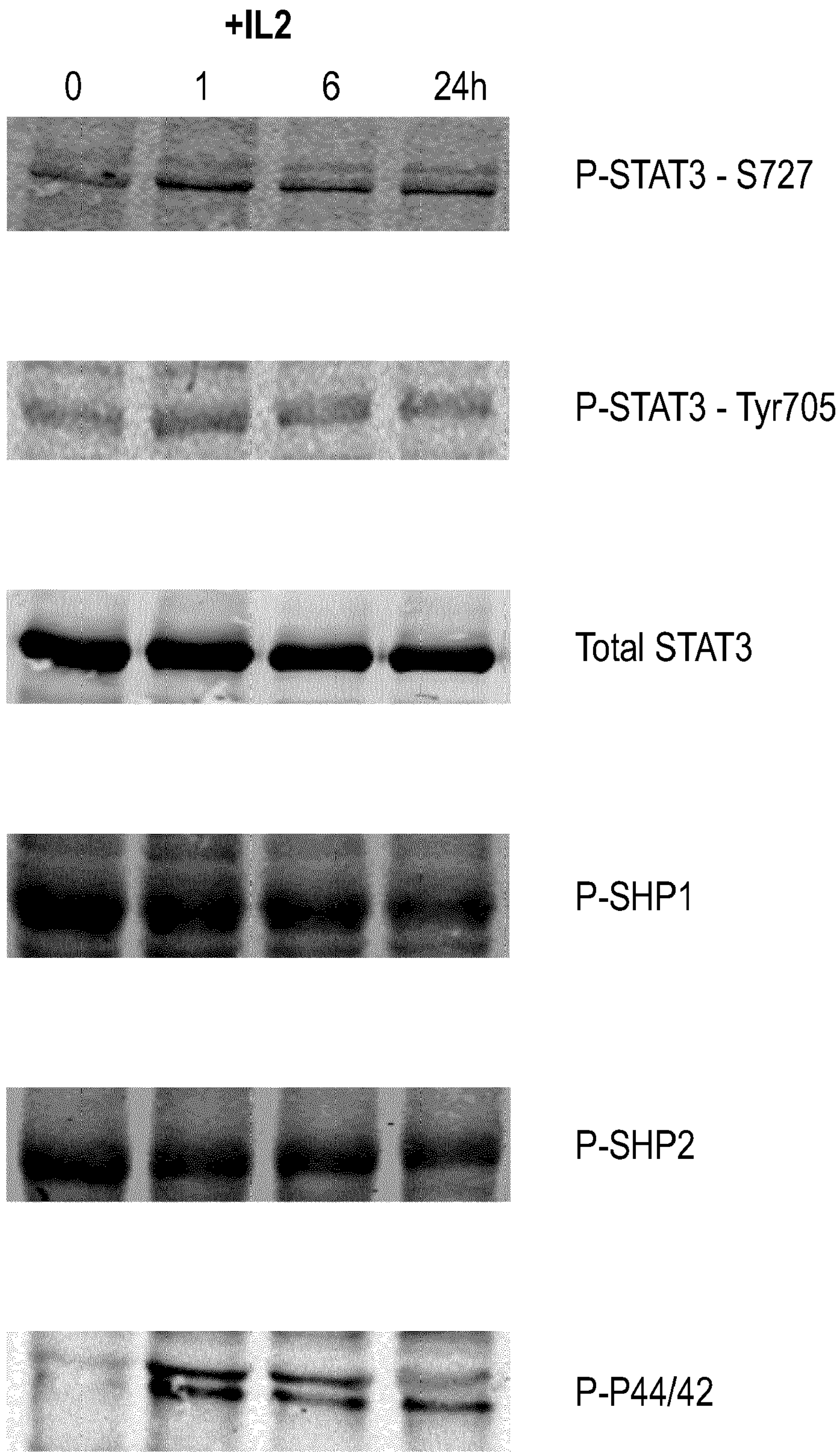
+IL2
0
1
6
24h
P-STAT3 - S727
P-STAT3 - Tyr705
Total STAT3
P-SHP1
P-SHP2
P-P44/42

FIG. 65

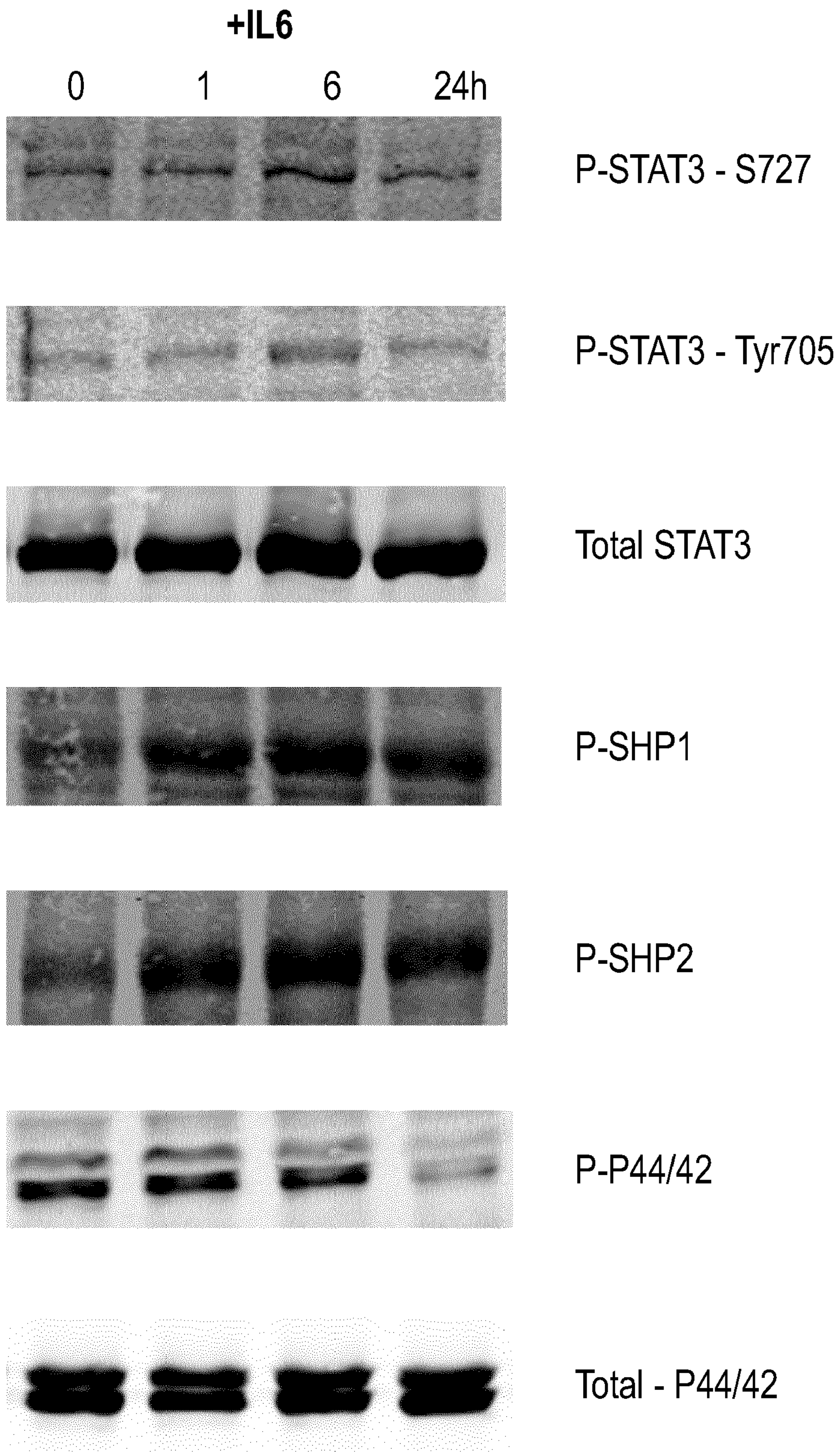
+IL6
0
1
6
24h
P-STAT3 - S727
P-STAT3 - Tyr705
Total STAT3
P-SHP1
P-SHP2
P-P44/42
Total - P44/42

FIG. 66

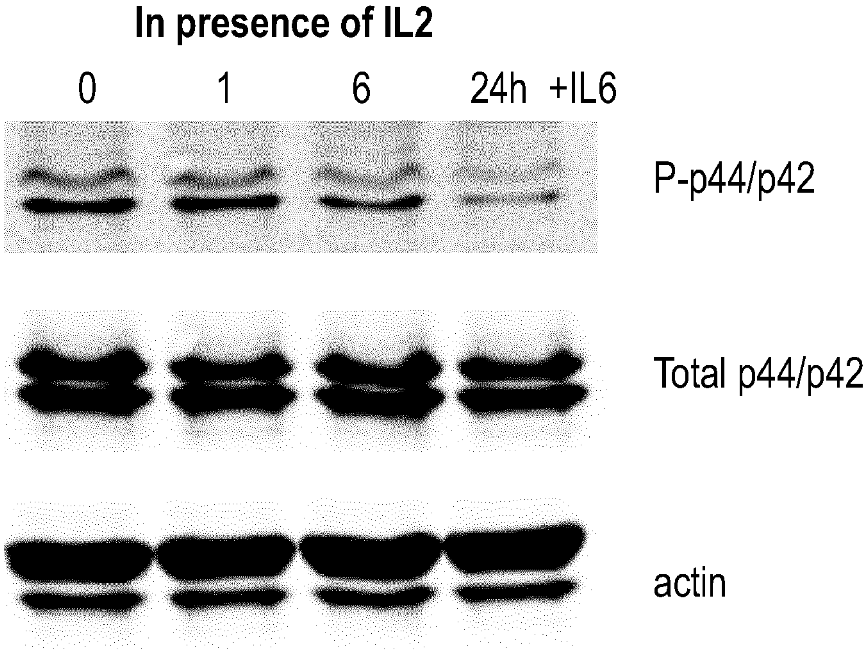


FIG. 67

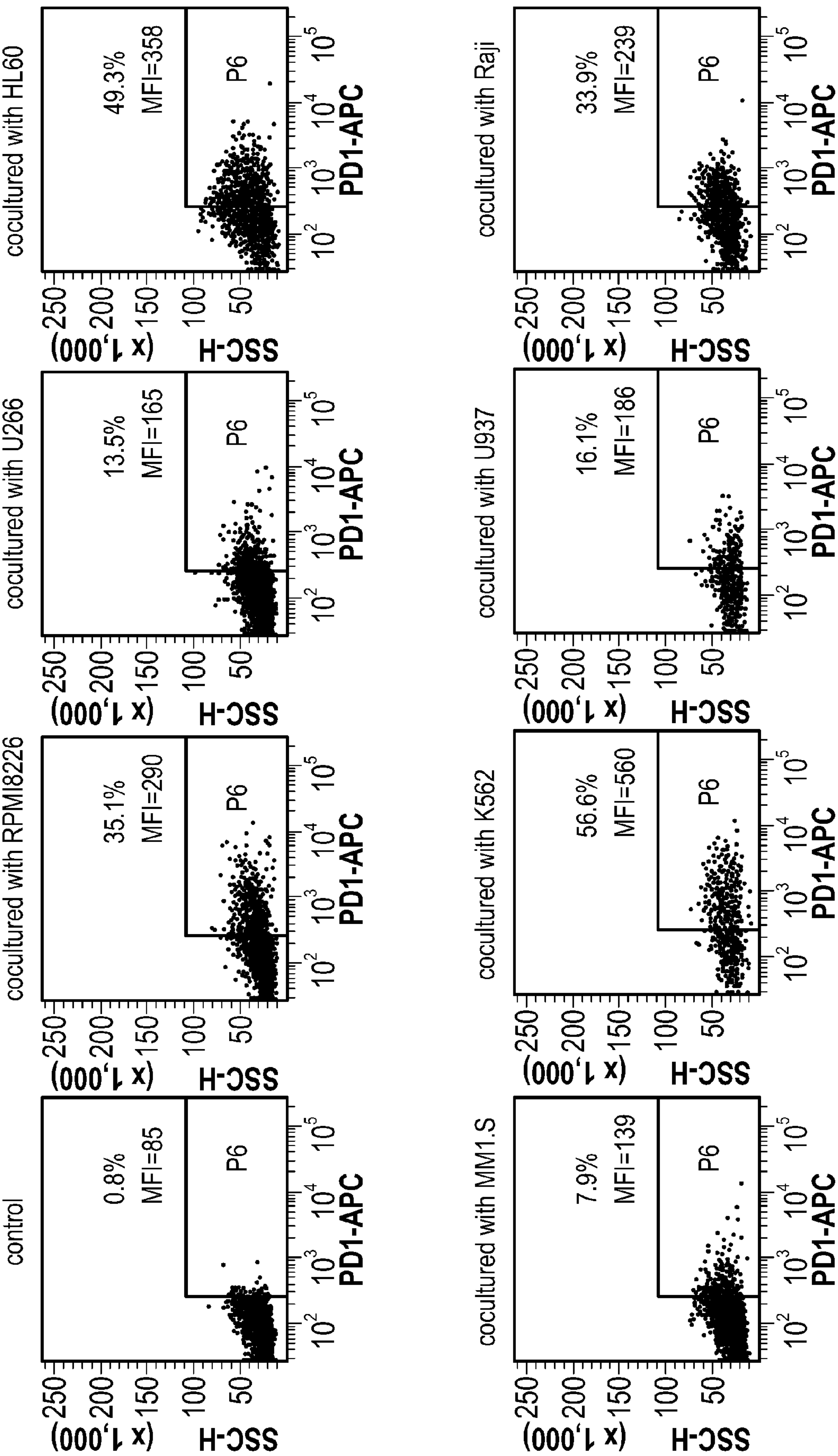
control
0.8%
MFI=85
cocultured with RPMI8226
35.1%
MFI=290
cocultured with U266
13.5%
MFI=165
cocultured with HL60
49.3%
MFI=358
cocultured with MM1.S
7.9%
MFI=139
cocultured with K562
56.6%
MFI=560
cocultured with U937
16.1%
MFI=186
cocultured with Raji
33.9%
MFI=239
P6
SSC-H (x 1,000)
PD1-APC

FIG. 68

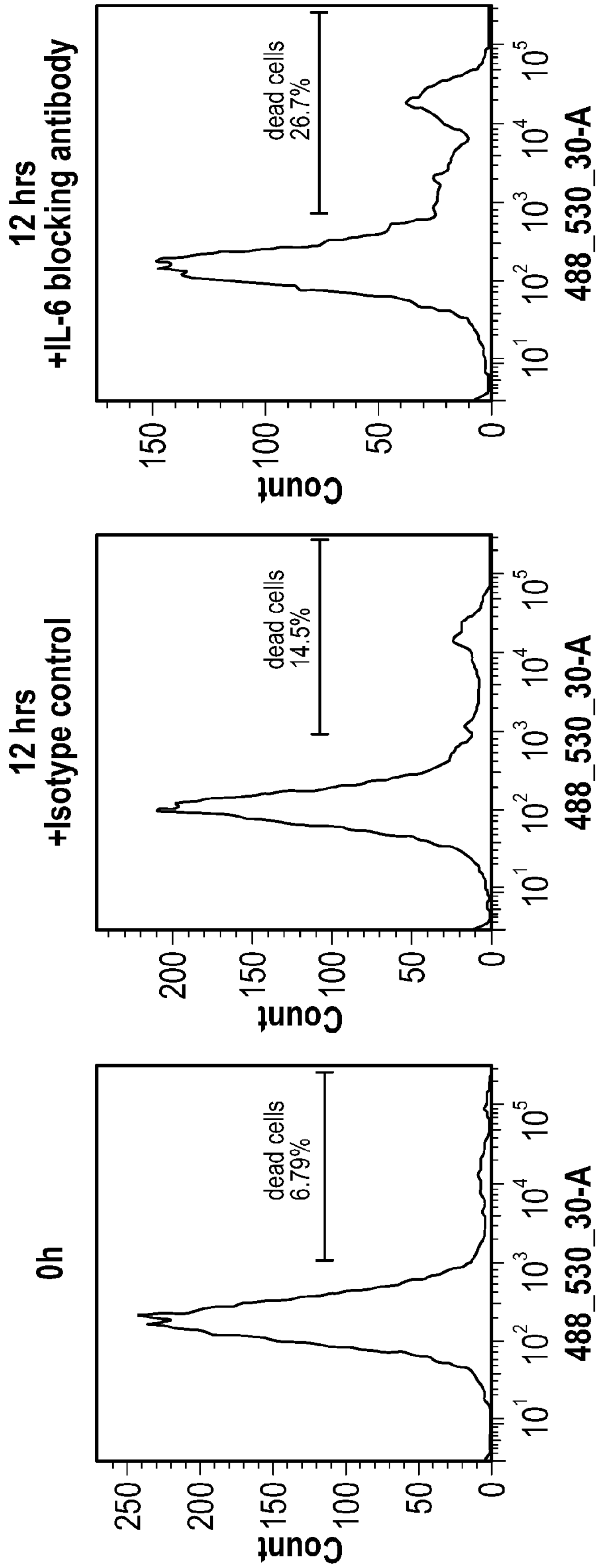
12 hrs
+IL-6 blocking antibody
dead cells
26.7%
Count
488_530_30-A
12 hrs
+Isotype control
dead cells
14.5%
Count
488_530_30-A
0h
dead cells
6.79%
Count
488_530_30-A

FIG. 69

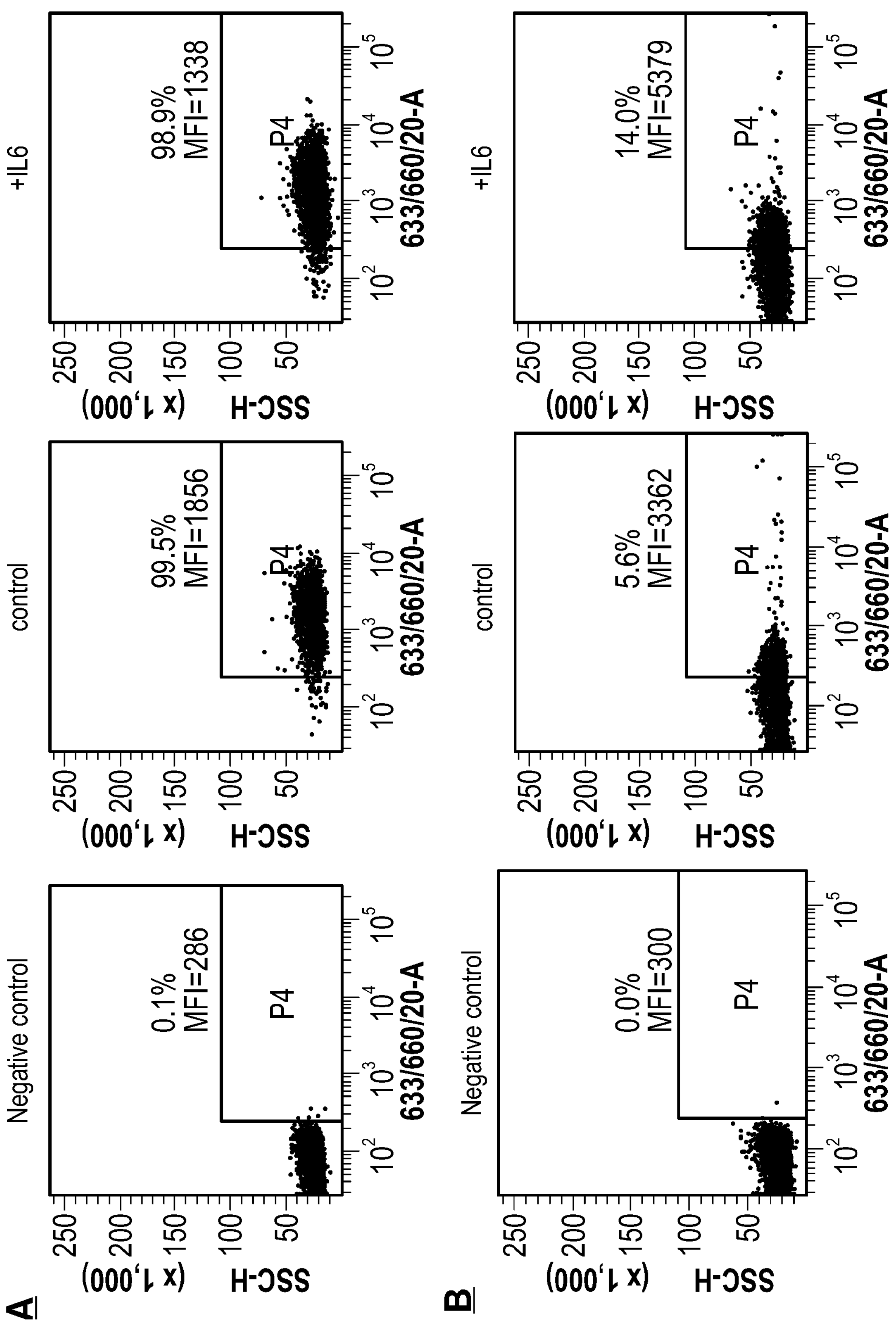
A
Negative control
0.1%
MFI=286
P4
control
99.5%
MFI=1856
P4
+IL6
98.9%
MFI=1338
P4
B
Negative control
0.0%
MFI=300
P4
control
5.6%
MFI=3362
P4
+IL6
14.0%
MFI=5379
P4
SSC-H (x 1,000)
250
200
150
100
50
633/660/20-A
$10^2$
$10^3$
$10^4$
$10^5$

FIG. 70

Europäisches Patentamt
European Patent Office
Office européen des brevets

# EUROPEAN SEARCH REPORT

Application Number
EP 22 15 6380

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAE-HWE HEO ET AL: "Potential therapeutic implications of IL-6/IL-6R/gp130-targeting agents in breast cancer", ONCOTARGET, vol. 7, no. 13, 29 March 2016 (2016-03-29), XP055456966, DOI: 10.18632/oncotarget.7102 * table 1 * ----- | 1-15 | INV. A61P35/00 C07K16/24 A61K31/00 A61K35/17 C07K14/00 C12N15/113 A61K39/395 |
| A,D | CIFALDI ET AL.: ARTHRITIS RHEUMATOL, vol. 67, no. 11, November 2015 (2015-11), pages 3037-46, XP002806191, * Results * ----- | 1-15 | |
| | | | TECHNICAL FIELDS SEARCHED (IPC) |
| | | | C07K A61K C12N A61P |

The present search report has been drawn up for all claims

1

| Place of search | Date of completion of the search | Examiner |
|---|---|---|
| The Hague | 21 April 2022 | Vadot, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009077857 A


### Non-patent literature cited in the description


- **CIFALDI et al.** *Arthritis Rheumatol,* November 2015, vol. 67 (11), 3037-46
- **KANG et al.** *Hum Reprod,* 10 October 2014, vol. 29 (10), 2176-89
- **WANG et al.** *PLoS One,* 07 October 2013, vol. 8 (10), e75788
- **TAMURA et al.** *Leukemia,* February 2013, vol. 27 (2), 464-72
- **IDORN et al.** *Cancer Immunol Immunother,* May 2017, vol. 66 (5), 667-671